# EUROPEAN PATENT APPLICATION

(11) **EP 4 763 997 A1**
(43) Date of publication of application: **24.06.2026**
(21) Application number: 26156156.7
(22) Date of filing: 10.06.2020
(51) Int. Cl.: C12Q 1/6883, G01N 33/68

(54) **BIOMARKER FOR DIAGNOSIS OF CEREBRAL NERVOUS SYSTEM DISEASES**

(30) Priority: 10.06.2019 KR 20190067945
(62) Divisional of application: 20821679.6
(71) Applicant: Industry-Academic Cooperation Foundation, Yonsei University, Seoul 03722 (KR); University - Industry Cooperation Group of Kyung Hee University, Yongin-si, Gyeonggi-do 17104 (KR)
(72) Inventor: KIM, Yong Bae, 06006 Seoul (KR); HONG, Chang Ki, 06273 Seoul (KR); LEE, Hyung Keun, 06279 Seoul (KR); JI, Yong Woo, 06523 Seoul (KR); KIM, Kwang Pyo, 01750 Seoul (KR); LEE, Hyeong Min, 16705 Suwon-si Gyeonggi-do (KR); HAAM, Seung Joo, 04427 Seoul (KR)
(74) Representative: Thomann, William John

(57) **Abstract**

The present invention relates to biomarkers capable of diagnosing various brain and nervous system diseases, and a method of providing information for diagnosing brain and nervous system diseases using the same. According to the present invention, it is possible to diagnose, at an early stage, the onset of a brain and nervous system disease or the likelihood of developing the disease or diagnose the progress or prognosis of the disease or the therapeutic effect against the disease, by measuring the expression level of the biomarker protein of the present invention or a gene encoding the same in the aqueous humor of the eye.

## Description

### Technical Field

The present invention relates to biomarkers capable of diagnosing various brain and nervous system diseases and a method of providing information for diagnosing brain and nervous system diseases using the same.

### Background Art

The brain and nervous system refers to a body control system composed of the brain, spinal cord, cerebral nerves, spinal nerves, autonomic nervous system, and the like. Brain and nervous system diseases are diverse including cerebral palsy, brain injury, traumatic brain injury, ischemic brain injury, concussion, brain contusion, cerebrovascular attack, cerebral infarction, cerebral hemorrhage, Parkinson's disease, Alzheimer's disease, Huntington's disease, paralysis, dementia, Lou Gehrig's disease, Huntington's disease, Pick disease, Creutzfeldt-Jakob disease, amyotrophic lateral sclerosis, primary lateral sclerosis, degenerative ataxia, multiple sclerosis, nervous system dysfunction, memory loss, epilepsy, encephalitis, prion disease, and neuropathy. Brain injury refers to a state in which an abnormality occurs in brain nerve tissues from various causes, including internal or external causes, resulting in an abnormality in behavior or function. Brain injury may be caused by cerebrovascular diseases such as open head injury, obstructive head injury, deceleration injury, exposure to toxic substances, oxygen deprivation, tumor, infection, and cerebrovascular attack.

Meanwhile, neurodegenerative diseases among the brain and nervous system diseases refer to a gradual structural and functional loss of nerve cells (neurons), and the signs of onset thereof appear gradually, and these diseases often develop with age. Once these diseases develop, these continue to progress over several years or decades until death, and it is known that these diseases are significantly influenced by genetic factors due to family history. Neurodegenerative diseases mainly invade a specific part of the nervous system and are accompanied by symptoms such as dementia, extrapyramidal abnormalities, cerebellar abnormalities, sensory disturbances, and movement disorders, and may also invade several parts, causing complex symptoms. Diagnosis of these diseases is made according to the clinical findings of the patients, and in this case, it is difficult to diagnose because the symptoms are diverse and different diseases often show common clinical symptoms.

The important cause of Alzheimer's disease, one of the representative neurodegenerative diseases, is known to be β-amyloid accumulation in the brain and the resulting neurotoxicity. In particular, it is known that Alzheimer's disease develops as protein β-amyloid builds plaques and tangles accumulate in the brain. When Alzheimer's disease occurs, histopathological features appear such as general atrophy of the brain, expansion of the ventricles, multiple lesions of nerve fibers (neurofibrillary tangle), and neurites (senile plaques), and decline in intellectual functions such as memory, judgment and language ability, and behavioral pattern disorder appear, and in severe cases, psychiatric symptoms such as depression are also accompanied. In addition, as the above-described symptoms progress gradually, the patient may lead to death after about 6 to 8 years after the onset of the disease.

However, since many methods for slowing the progression of brain and nervous system diseases have recently been developed, early diagnosis of the brain and nervous system diseases is of paramount importance. However, there is currently no reliable early diagnostic test, and a brain biopsy method is generally performed after patient's death. Therefore, there is a need to develop a composition or diagnostic method capable of accurately diagnosing various brain and nervous system diseases, including Alzheimer's disease and Parkinson's disease, at early stages.

### DISCLOSURE

### Technical Problem

An object of the present invention is to provide a composition or kit capable of diagnosing a brain and nervous system disease.

Another object of the present invention is to provide a method for providing information for diagnosing a brain and nervous system disease.

Another object of the present invention is to provide a method for screening a substance that induces a brain nervous system disease.

However, the objects to be achieved by the present invention are not limited to the above-mentioned objects, and other objects not mentioned herein will be clearly understood by those skilled in the art from the following description.

### Technical Solution

As used herein, the term "diagnosis" or "diagnosing" means identifying the presence or characteristics of a pathological condition. For the purposes of the present invention, the diagnosis means determining either whether a brain and nervous system disease has developed or the likelihood of developing the disease, thereby diagnosing the onset of various brain and nervous system diseases in early stages.

In the present specification, the brain and nervous system disease may be a disease selected from the group consisting of dementia, Alzheimer's disease, cerebrovascular attack, Parkinson's disease, Huntington's disease, Pick's disease, amyotrophic lateral sclerosis (ALS), Creutzfeldt-Jakob disease (CJD), progressive supranuclear palsy, multiple system atrophy, olivopontocerebellar atrophy (OPCA), Shy-Drager syndrome, essential tremor, cortico-basal ganlionic degeneration, diffuse Lewy body disease, striatonigral degeneration, and brain tumor.

In the present specification, the brain tumor may be a disease selected from the group consisting of glioblastoma, medulloblastoma, astrocytoma, primitive neuroectodermal tumor, and brainstem glioma, but is not limited thereto, and may include any tumor growing in the brain.

The genes described as biomarkers in the present specification are human (Homo sapiens) genes, and information about the genes can be easily found at https://www.uniprot.org/uniprot/P52566.

One embodiment of the present invention is directed to a biomarker for diagnosing a brain and nervous system disease comprising: at least one gene selected from the group shown in Table 1 below; or a protein encoded thereby:

**[Table 1]**

| Accession number | Gene name |
|---|---|
| Q06830 | PRDX1 |
| P23515 | OMG |
| P02458 | COL2A1 |
| 014594 | NCAN |
| P13500 | CCL2 |
| Q01469 | FABP5 |
| Q9UFP1 | FAM198A |
| Q63HQ2 | EGFLAM |
| P22303 | ACHE |
| A0A087WWD4 | NCAM1 |
| Q15904 | ATP6AP1 |
| P15328 | FOLR1 |
| P48058 | GRIA4 |
| P50395 | GDI2 |
| O95897 | OLFM2 |
| P00441 | SOD1 |
| O75973 | C1QL1 |
| Q99519 | NEU1 |
| P23471 | PTPRZ1 |
| P03973 | SLPI |
| Q53EL9 | SEZ6 |
| P43251 | BTD |
| A0A087WZM2 | RNASET2 |
| 014818 | PSMA7 |
| Q8WZA1 | POMGNT1 |
| P04083 | ANXA1 |
| Q99574 | SERPINI1 |
| P58546 | MTPN |
| Q14019 | COTL1 |
| P23468 | PTPRD |
| P10745 | RBP3 |
| P00533 | EGFR |
| P27797 | CALR |
| Q9P2S2 | NRXN2 |
| P68104 | EEF1A1 |
| P56159 | GFRA1 |
| A0A1B0GV53 | CLEC19A |
| 094919 | ENDOD1 |
| P60709 | ACTB |
| P07711 | CTSL |
| P11021 | HSPA5 |
| P18669 | PGAM1 |
| H7BY58 | PCMT1 |
| Q9NS 15 | LTBP3 |
| B5MCX6 | VSTM2A |
| Q9UHC6 | CNTNAP2 |
| P11117 | ACP2 |
| P78324 | SIRPA |
| 095841 | ANGPTL1 |
| Q15818 | NPTX1 |
| P08123 | COL1A2 |
| Q92876 | KLK6 |
| P16278 | GLB1 |
| P18206 | VCL |
| P13639 | EEF2 |
| P23141 | CES1 |
| Q92563 | SPOCK2 |
| P22352 | GPX3 |
| Q86UN2 | RTN4RL1 |
| O00264 | PGRMC 1 |
| P10643 | C7 |
| A0A096LPE2 | SAA2-SAA4 |
| P02647 | APOA1 |
| P49788 | RARRES 1 |
| 000451 | GFRA2 |
| P02748 | C9 |
| P02760 | AMBP |
| P06727 | APOA4 |
| Q9BTY2 | FUCA2 |
| P0DJI8 | SAA1 |
| P05546 | SERPIND1 |
| P07358 | C8B |
| Q06828 | FMOD |
| P00450 | CP |
| P22692 | IGFBP4 |
| O95497 | VNN1 |
| P07315 | CRYGC |
| Q96PD5 | PGLYRP2 |
| Q 14847 | LASP1 |
| P10451 | SPP1 |
| J3KQ66 | RELN |
| P39059 | COL15A1 |
| Q8IWV2 | CNTN4 |
| P31946 | YWHAB |
| P06276 | BCHE |
| Q5VU97 | CACHD1 |
| Q9HC56 | PCDH9 |
| P54826 | GAS1 |
| K7ES00 | H3F3B |
| Q495W5 | FUT11 |
| Q99941 | ATF6B |
| P02743 | APCS |
| Q14982 | OPCML |
| Q9HBL6 | LRTM1 |
| P04745 | AMY1A |
| P34059 | GALNS |
| Q9NZK5 | ADA2 |
| Q9H4F8 | SMOC1 |
| Q12797 | ASPH |
| Q9HC57 | WFDC1 |
| Q6FHJ7 | SFRP4 |
| Q9HCQ7 | NPVF |
| P14151 | SELL |
| P06703 | S100A6 |
| P09382 | LGALS 1 |
| P00390 | GSR |
| Q8TDF5 | NETO1 |
| P29279 | CTGF |
| P62937 | PPIA |
| Q96LR4 | FAM19A4 |
| P13646 | KRT13 |
| P61604 | HSPE1 |
| O43827 | ANGPTL7 |
| P08670 | VIM |
| A0A0A0MRJ7 | F5 |
| Q53RD9 | FBLN7 |
| P15121 | AKR1B1 |
| A6NC48 | BST1 |
| O60242 | ADGRB3 |
| P40925 | MDH1 |
| E9PDN6 | CNTNAP4 |
| Q96KP4 | CNDP2 |
| O95965 | ITGBL1 |
| P15144 | ANPEP |
| Q9NZ08 | ERAP1 |
| P55287 | CDH11 |
| P05546 | SERPIND1 |
| P60174 | TPI1 |
| O43278 | SPINT 1 |
| Q14520 | HABP2 |
| P14384 | CPM |
| P00742 | F10 |
| O95497 | VNN1 |
| P04155 | TFF1 |
| Q13201 | MMRN1 |
| Q9HCQ7 | NPVF |
| Q92954 | PRG4 |
| O94910 | ADGRL1 |
| E9PLM6 | MDK |
| P02818 | BGLAP |
| Q13510 | ASAH1 |
| P19957 | PI3 |
| P01833 | PIGR |
| P48745 | NOV |
| P31431 | SDC4 |
| Q9HAT2 | SIAE |
| P49908 | SELENOP |
| Q14508 | WFDC2 |
| P02753 | RBP4 |
| E9PR17 | CD59 |
| P20933 | AGA |
| P21246 | PTN |
| AOA087WWT2 | NRN1 |
| Q9ULB1 | NRXN1 |
| Q9NP84 | TNFRSF12A |
| Q9Y287 | ITM2B |
| O95206 | PCDH8 |
| P11684 | SCGB1A1 |
| P33151 | CDH5 |
| P35318 | ADM |
| 000115 | DNASE2 |
| 043291 | SPINT2 |
| Q8NHP8 | PLBD2 |
| A8MV23 | SERPINE3 |
| Q13308 | PTK7 |
| P61626 | LYZ |
| A0A1B0GV53 | CLEC19A |
| Q9P121 | NTM |
| Q12841 | FSTL1 |
| P02671 | FGA |
| P07333 | CSF1R |
| P06727 | APOA4 |
| P02741 | CRP |
| Q9GZX9 | TWSG1 |
| Q9P0K1 | ADAM22 |
| O00468 | AGRN |
| P11047 | LAMC1 |
| Q9UBX7 | KLK11 |
| P98160 | HSPG2 |
| Q9GZP0 | PDGFD |
| P20774 | OGN |
| P16930 | FAH |
| Q92563 | SPOCK2 |
| Q16270 | IGFBP7 |
| 014672 | ADAM10 |
| Q969El | LEAP2 |
| Q5VZE7 | SPINK4 |
| Q8NBJ4 | GOLM1 |
| Q02985 | CFHR3 |
| P0DJI8 | SAA1 |
| P30043 | BLVRB |
| O95274 | LYPD3 |
| P49788 | RARRES1 |
| P02750 | LRG1 |
| P23142 | FBLN1 |
| P48745 | NOV |
| Q9NPY3 | CD93 |
| 015240 | VGF |
| Q08174 | PCDH1 |
| P07225 | PROS1 |
| Q14847 | LASP1 |
| Q99983 | OMD |
| P55289 | CDH12 |
| P27918 | CFP |
| P31431 | SDC4 |
| P24043 | LAMA2 |
| P12111 | COL6A3 |
| Q14515 | SPARCL1 |
| Q14766 | LTBP1 |
| P08185 | SERPINA6 |
| Q13231 | CHIT1 |
| 014773 | TPP1 |
| P00492 | HPRT1 |
| Q96DR8 | MUCL1 |
| P15121 | AKR1B1 |
| Q99969 | RARRES2 |
| P04075 | ALDOA |
| P06396 | GSN |
| Q16568 | CARTPT |
| P26447 | S100A4 |
| P14625 | HSP90B1 |
| P08670 | VIM |
| Q86SF2 | GALNT7 |
| Q9UJJ9 | GNPTG |
| Q8NFY4 | SEMA6D |
| Q7Z7H5 | TMED4 |
| Q9Y646 | CPQ |
| Q9Y2I2 | NTNG1 |
| P40967 | PMEL |
| P07451 | CA3 |
| J3KNP4 | SEMA4B |
| O95336 | PGLS |
| P00441 | SOD1 |
| P10586 | PTPRF |
| Q86UD 1 | OAF |
| P41222 | PTGDS |
| A6NGN9 | IGLON5 |
| P42857 | NSG1 |
| F5GWQ8 | CLUL1 |
| Q8N436 | CPXM2 |
| Q96FE5 | LINGO1 |
| Q495W5 | FUT11 |
| Q658N2 | WSCD1 |
| Q5JS37 | NHLRC3 |
| Q99784 | OLFM1 |
| Q8NFP4 | MDGA1 |
| Q96JP9 | CDHR1 |
| P08758 | ANXA5 |
| Q92484 | SMPDL3A |
| Q16849 | PTPRN |
| Q8WXD2 | SCG3 |
| O75326 | SEMA7A |
| Q86VZ4 | LRP11 |
| P02649 | APOE |
| Q 17R60 | IMPG1 |
| Q9UNW1 | MINPP 1 |
| P08294 | SOD3 |
| P15848 | ARSB |

In the present invention, as the biomarker, one present in the aqueous humor of the eye may increase the accuracy in diagnosing the brain and nervous system disease.

In the present invention, the "aqueous humor" is a transparent liquid like water, provides nutrition to the lens and cornea, structurally supports the eye, and fills the anterior chamber and the posterior chamber.

### Biomarkers for Diagnosing Alzheimer's Disease

One embodiment of the present invention is directed to a biomarker for diagnosing Alzheimer's disease among brain and nervous system diseases comprising: at least one gene selected from the group shown in Table 2 below; or a protein encoded thereby:

**[Table 2]**

| Accession number | Gene name |
|---|---|
| Q06830 | PRDX1 |
| P23515 | OMG |
| P02458 | COL2A1 |
| 014594 | NCAN |
| P13500 | CCL2 |
| Q01469 | FABP5 |
| Q9UFP1 | FAM198A |
| Q63HQ2 | EGFLAM |
| P22303 | ACHE |
| A0A087WWD4 | NCAM1 |
| Q15904 | ATP6AP1 |
| P15328 | FOLR1 |
| P48058 | GRIA4 |
| P50395 | GDI2 |
| O95897 | OLFM2 |
| P00441 | SOD1 |
| O75973 | C1QL1 |
| Q99519 | NEU1 |
| P23471 | PTPRZ1 |
| P03973 | SLPI |
| Q53EL9 | SEZ6 |
| P43251 | BTD |
| A0A087WZM2 | RNASET2 |
| 014818 | PSMA7 |
| Q8WZA1 | POMGNT1 |
| P04083 | ANXA1 |
| Q99574 | SERPINI1 |
| P58546 | MTPN |
| Q14019 | COTL1 |
| P23468 | PTPRD |
| P10745 | RBP3 |
| P00533 | EGFR |
| P27797 | CALR |
| Q9P2S2 | NRXN2 |
| P68104 | EEF1A1 |
| P56159 | GFRA1 |
| A0A1B0GV53 | CLEC19A |
| 094919 | ENDOD1 |
| P60709 | ACTB |
| P07711 | CTSL |
| P11021 | HSPA5 |
| P18669 | PGAM1 |
| H7BY58 | PCMT1 |
| Q9NS15 | LTBP3 |
| B5MCX6 | VSTM2A |
| Q9UHC6 | CNTNAP2 |
| P11117 | ACP2 |
| P78324 | SIRPA |
| 095841 | ANGPTL1 |
| Q15818 | NPTX1 |
| P08123 | COL1A2 |
| Q92876 | KLK6 |
| P16278 | GLB1 |
| P18206 | VCL |
| P13639 | EEF2 |
| P23141 | CES1 |
| Q92563 | SPOCK2 |
| P22352 | GPX3 |
| Q86UN2 | RTN4RL1 |
| O00264 | PGRMC1 |
| P10643 | C7 |
| A0A096LPE2 | SAA2-SAA4 |
| P02647 | APOA1 |
| P49788 | RARRES1 |
| 000451 | GFRA2 |
| P02748 | C9 |
| P02760 | AMBP |
| P06727 | APOA4 |
| Q9BTY2 | FUCA2 |
| P0DJI8 | SAA1 |
| P05546 | SERPIND 1 |
| P07358 | C8B |
| Q06828 | FMOD |
| P00450 | CP |
| P22692 | IGFBP4 |
| O95497 | VNN1 |
| P07315 | CRYGC |
| Q96PD5 | PGLYRP2 |
| Q14847 | LASP1 |
| P10451 | SPP1 |
| J3KQ66 | RELN |
| P39059 | COL15A1 |
| Q8IWV2 | CNTN4 |
| P31946 | YWHAB |
| P06276 | BCHE |

The biomarker of the present invention may preferably be: at least one gene selected from the group consisting of ACHE, SPP1, EEF2, PRDX1, CES1, YWHAB, CNTN4 and BCHE; or a protein encoded thereby.

Among the biomarkers of the present invention, at least one gene selected from the group shown in Table 3 below, or a protein encoded thereby may have a higher expression level than that in a normal control group:

**[Table 3]**

| Accession number | Gene name |
|---|---|
| Q06830 | PRDX1 |
| P23515 | OMG |
| P02458 | COL2A1 |
| 014594 | NCAN |
| P13500 | CCL2 |
| Q01469 | FABP5 |
| Q9UFP1 | FAM198A |
| Q63HQ2 | EGFLAM |
| P22303 | ACHE |
| A0A087WWD4 | NCAM1 |
| Q15904 | ATP6AP1 |
| P15328 | FOLR1 |
| P48058 | GRIA4 |
| P50395 | GDI2 |
| O95897 | OLFM2 |
| P00441 | SOD1 |
| O75973 | C1QL1 |
| Q99519 | NEU1 |
| P23471 | PTPRZ1 |
| P03973 | SLPI |
| Q53EL9 | SEZ6 |
| P43251 | BTD |
| A0A087WZM2 | RNASET2 |
| 014818 | PSMA7 |
| Q8WZA1 | POMGNT1 |
| P04083 | ANXA1 |
| Q99574 | SERPINI1 |
| P58546 | MTPN |
| Q14019 | COTL1 |
| P23468 | PTPRD |
| P10745 | RBP3 |
| P00533 | EGFR |
| P27797 | CALR |
| Q9P2S2 | NRXN2 |
| P68104 | EEF1A1 |
| P56159 | GFRA1 |
| A0A1B0GV53 | CLEC19A |
| 094919 | ENDOD1 |
| P60709 | ACTB |
| P07711 | CTSL |
| P11021 | HSPA5 |
| P18669 | PGAM1 |
| H7BY58 | PCMT1 |
| Q9NS15 | LTBP3 |
| B5MCX6 | VSTM2A |
| Q9UHC6 | CNTNAP2 |
| P11117 | ACP2 |
| P78324 | SIRPA |
| 095841 | ANGPTL1 |
| Q15818 | NPTX1 |
| P08123 | COL1A2 |
| Q92876 | KLK6 |
| P16278 | GLB1 |
| P18206 | VCL |
| P13639 | EEF2 |
| P23141 | CES1 |

Among the biomarkers of the present invention, preferably, at least one gene selected from the group consisting of ACHE, SPP1, EEF2, PRDX1 and CES1, or a protein encoded thereby may have a higher expression level than that in a normal control group.

Among the biomarkers of the present invention, at least one gene selected from the group shown in Table 4 below, or a protein encoded thereby may have a lower expression level than that in a normal control group:

**[Table 4]**

| Accession number | Gene name |
|---|---|
| Q92563 | SPOCK2 |
| P22352 | GPX3 |
| Q86UN2 | RTN4RL1 |
| O00264 | PGRMC1 |
| P10643 | C7 |
| A0A096LPE2 | SAA2-SAA4 |
| P02647 | APOA1 |
| P49788 | RARRES1 |
| 000451 | GFRA2 |
| P02748 | C9 |
| P02760 | AMBP |
| P06727 | APOA4 |
| Q9BTY2 | FUCA2 |
| P0DJI8 | SAA1 |
| P05546 | SERPIND 1 |
| P07358 | C8B |
| Q06828 | FMOD |
| P00450 | CP |
| P22692 | IGFBP4 |
| O95497 | VNN1 |
| P07315 | CRYGC |
| Q96PD5 | PGLYRP2 |
| Q14847 | LASP1 |
| P10451 | SPP1 |
| J3KQ66 | RELN |
| P39059 | COL15A1 |
| Q8IWV2 | CNTN4 |
| P31946 | YWHAB |
| P06276 | BCHE |

Among the biomarkers of the present invention, preferably, at least one gene selected from the group consisting of YWHAB, CNTN4 and BCHE, or a protein encoded thereby may have a lower expression level than that in a normal control group.

### Biomarkers for Diagnosing Parkinson's Disease

Another embodiment of the present invention is directed to a biomarker for diagnosing Parkinson's disease among brain and nervous system diseases comprising: at least one gene selected from the group shown in Table 5 below; a protein encoded thereby:

**[Table 5]**

| Accession number | Gene name |
|---|---|
| Q5VU97 | CACHD1 |
| Q9HC56 | PCDH9 |
| P54826 | GAS 1 |
| K7ES00 | H3F3B |
| Q495W5 | FUT 11 |
| Q99941 | ATF6B |
| P02743 | APCS |
| Q14982 | OPCML |
| Q9HBL6 | LRTM1 |
| P04745 | AMY1A |
| P34059 | GALNS |
| Q9NZK5 | ADA2 |
| Q9H4F8 | SMOC1 |
| Q12797 | ASPH |
| Q9HC57 | WFDC 1 |
| Q6FHJ7 | SFRP4 |
| Q9HCQ7 | NPVF |
| P14151 | SELL |
| P06703 | S100A6 |
| P09382 | LGALS1 |
| P00390 | GSR |
| Q8TDF5 | NETO1 |
| P29279 | CTGF |

The biomarker of the present invention may preferably be: at least one gene selected from the group consisting of CACHD1, PCDH9, GAS1, H3F3B, FUT11, ATF6B, SELL, S100A6, LGALS1, GSR, NETO1 and CTGF; or a protein encoded thereby.

Among the biomarkers of the present invention, at least one gene selected from the group shown in Table 6 below, or a protein encoded thereby may have a higher expression level than that in a normal control group:

**[Table 6]**

| Accession number | Gene name |
|---|---|
| Q5VU97 | CACHD1 |
| Q9HC56 | PCDH9 |
| P54826 | GAS1 |
| K7ES00 | H3F3B |
| Q495W5 | FUT 11 |
| Q99941 | ATF6B |
| P02743 | APCS |
| Q14982 | OPCML |
| Q9HBL6 | LRTM1 |
| P04745 | AMY1A |
| P34059 | GALNS |
| Q9NZK5 | ADA2 |
| Q9H4F8 | SMOC1 |
| Q12797 | ASPH |
| Q9HC57 | WFDC 1 |
| Q6FHJ7 | SFRP4 |
| Q9HCQ7 | NPVF |

Among the biomarkers of the present invention, preferably, at least one gene selected from the group consisting of CACHD1, PCDH9, GAS1, H3F3B, FUT11 and ATF6B, or a protein encoded thereby may have a higher expression level than that in a normal control group.

Among the biomarker of the present invention, at least one gene selected from the group shown in Table 7 below, or a protein encoded thereby may have a lower expression level than that in a normal control group:

**[Table 7]**

| Accession number | Gene name |
|---|---|
| P14151 | SELL |
| P06703 | S100A6 |
| P09382 | LGALS 1 |
| P00390 | GSR |
| Q8TDF5 | NETO1 |
| P29279 | CTGF |

### Biomarkers for Diagnosing Cerebrovascular attack

Still another embodiment of the present invention is directed to a biomarker for diagnosing cerebrovascular attack among brain and nervous system diseases comprising: at least one gene selected from the group shown in Table 8 below; or a protein encoded thereby:

**[Table 8]**

| Accession number | Gene name |
|---|---|
| P62937 | PPIA |
| Q96LR4 | FAM19A4 |
| P13646 | KRT13 |
| P61604 | HSPE1 |
| O43827 | ANGPTL7 |
| P08670 | VIM |
| P10451 | SPP1 |
| A0A0A0MRJ7 | F5 |
| Q53RD9 | FBLN7 |
| P15121 | AKR1B1 |
| A6NC48 | BST1 |
| O60242 | ADGRB3 |
| P40925 | MDH1 |
| E9PDN6 | CNTNAP4 |
| Q96KP4 | CNDP2 |
| O95965 | ITGBL1 |
| P15144 | ANPEP |
| Q9NZ08 | ERAP1 |
| P55287 | CDH11 |
| P05546 | SERPIND1 |
| P60174 | TPI1 |
| O43278 | SPINT1 |
| Q14520 | HABP2 |
| P14384 | CPM |
| P00742 | F10 |
| O95497 | VNN1 |
| P04155 | TFF1 |
| Q13201 | MMRN1 |
| Q9HCQ7 | NPVF |
| Q92954 | PRG4 |
| 094910 | ADGRL1 |
| E9PLM6 | MDK |
| P02818 | BGLAP |
| Q13510 | ASAH1 |
| P19957 | PI3 |
| P01833 | PIGR |
| P48745 | NOV |
| P31431 | SDC4 |
| Q9HAT2 | SIAE |
| P49908 | SELENOP |
| Q14508 | WFDC2 |
| P02753 | RBP4 |
| E9PR17 | CD59 |
| P20933 | AGA |
| P21246 | PTN |
| AOA087WWT2 | NRN1 |
| Q9ULB1 | NRXN1 |
| Q9NP84 | TNFRSF 12A |
| Q9Y287 | ITM2B |
| O95206 | PCDH8 |
| P11684 | SCGB1A1 |
| P33151 | CDH5 |
| P35318 | ADM |
| 000115 | DNASE2 |
| 043291 | SPINT2 |
| Q8NHP8 | PLBD2 |
| A8MV23 | SERPINE3 |
| Q13308 | PTK7 |
| P61626 | LYZ |
| A0A1B0GV53 | CLEC19A |

The biomarker of the present invention may preferably be: at least one gene selected from the group consisting of PPIA, FAM19A4, KRT13, HSPE1, ANGPTL7, VIM, TFF1, MMRN1, NPVF, PRG4, ADGRL1 and MDK; or a protein encoded thereby.

Among the biomarkers of the present invention, at least one gene selected from the group shown in Table 9 below, or a protein encoded thereby may have a higher expression level than that in a normal control group:

**[Table 9]**

| Accession number | Gene name |
|---|---|
| P62937 | PPIA |
| Q96LR4 | FAM19A4 |
| P13646 | KRT13 |
| P61604 | HSPE1 |
| O43827 | ANGPTL7 |
| P08670 | VIM |
| P10451 | SPP1 |
| A0A0A0MRJ7 | F5 |
| Q53RD9 | FBLN7 |
| P15121 | AKR1B1 |
| A6NC48 | BST1 |
| O60242 | ADGRB3 |
| P40925 | MDH1 |
| E9PDN6 | CNTNAP4 |
| Q96KP4 | CNDP2 |
| O95965 | ITGBL1 |
| P15144 | ANPEP |
| Q9NZ08 | ERAP1 |
| P55287 | CDH11 |
| P05546 | SERPIND 1 |
| P60174 | TPI1 |
| O43278 | SPINT1 |
| Q14520 | HABP2 |
| P14384 | CPM |
| P00742 | F10 |
| O95497 | VNN1 |

Among the biomarkers of the present invention, preferably, at least one gene selected from the group consisting of PPIA, FAM19A4, KRT13, HSPE1, ANGPTL7 and VIM, or a protein encoded thereby may have a higher expression level than that in a normal control group.

Among the biomarkers of the present invention, at least one gene selected from the group shown in Table 10 below, or a protein encoded thereby may have a lower expression level than that in a normal control group:

**[Table 10]**

| Accession number | Gene name |
|---|---|
| P04155 | TFF1 |
| Q13201 | MMRN1 |
| Q9HCQ7 | NPVF |
| Q92954 | PRG4 |
| 094910 | ADGRL1 |
| E9PLM6 | MDK |
| P02818 | BGLAP |
| Q13510 | ASAH1 |
| P19957 | PI3 |
| P01833 | PIGR |
| P48745 | NOV |
| P31431 | SDC4 |
| Q9HAT2 | SIAE |
| P49908 | SELENOP |
| Q14508 | WFDC2 |
| P02753 | RBP4 |
| E9PR17 | CD59 |
| P20933 | AGA |
| P21246 | PTN |
| AOA087WWT2 | NRN1 |
| Q9ULB1 | NRXN1 |
| Q9NP84 | TNFRSF12A |
| Q9Y287 | ITM2B |
| O95206 | PCDH8 |
| P11684 | SCGB1A1 |
| P33151 | CDH5 |
| P35318 | ADM |
| 000115 | DNASE2 |
| 043291 | SPINT2 |
| Q8NHP8 | PLBD2 |
| A8MV23 | SERPINE3 |
| Q13308 | PTK7 |
| P61626 | LYZ |
| A0A1B0GV53 | CLEC19A |

Among the biomarkers of the present invention, preferably, at least one gene selected from the group consisting of TFF1, MMRN1, NPVF, PRG4, ADGRL1 and MDK, or a protein encoded thereby may have a lower expression level than that in a normal control group.

### Biomarkers for Diagnosing Brain Tumor

Still another embodiment of the present invention is directed to a biomarker for diagnosing brain tumor among brain and nervous system diseases comprising: at least one gene selected from the group shown in Table 11 below; or a protein encoded thereby:

**[Table 11]**

| Accession number | Gene name |
|---|---|
| Q9P121 | NTM |
| Q12841 | FSTL1 |
| P02671 | FGA |
| P07333 | CSF1R |
| P06727 | APOA4 |
| P02741 | CRP |
| Q9GZX9 | TWSG1 |
| Q9P0K1 | ADAM22 |
| O00468 | AGRN |
| P11047 | LAMC1 |
| Q9UBX7 | KLK11 |
| P98160 | HSPG2 |
| Q9GZP0 | PDGFD |
| P20774 | OGN |
| P16930 | FAH |
| Q92563 | SPOCK2 |
| Q16270 | IGFBP7 |
| 014672 | ADAM10 |
| Q969E1 | LEAP2 |
| Q5VZE7 | SPINK4 |
| Q8NBJ4 | GOLM1 |
| Q02985 | CFHR3 |
| P0DJI8 | SAA1 |
| P30043 | BLVRB |
| O95274 | LYPD3 |
| P49788 | RARRES1 |
| P02750 | LRG1 |
| P23142 | FBLN1 |
| P48745 | NOV |
| Q9NPY3 | CD93 |
| 015240 | VGF |
| Q08174 | PCDH1 |
| P07225 | PROS 1 |
| Q14847 | LASP1 |
| Q99983 | OMD |
| P55289 | CDH12 |
| P27918 | CFP |
| P31431 | SDC4 |
| P24043 | LAMA2 |
| P12111 | COL6A3 |
| Q14515 | SPARCL1 |
| Q14766 | LTBP1 |
| P08185 | SERPINA6 |
| Q13231 | CHIT1 |
| 014773 | TPP1 |
| P00492 | HPRT1 |
| Q96DR8 | MUCL1 |
| P15121 | AKR1B1 |
| Q99969 | RARRES2 |
| P04075 | ALDOA |
| P06396 | GSN |
| Q16568 | CARTPT |
| P26447 | S100A4 |
| P14625 | HSP90B1 |
| P08670 | VIM |
| Q86SF2 | GALNT7 |
| Q9UJJ9 | GNPTG |
| Q8NFY4 | SEMA6D |
| Q7Z7H5 | TMED4 |
| Q9Y646 | CPQ |
| Q9Y2I2 | NTNG1 |
| P40967 | PMEL |
| P07451 | CA3 |
| J3KNP4 | SEMA4B |
| O95336 | PGLS |
| P00441 | SOD1 |
| P10586 | PTPRF |
| Q86UD 1 | OAF |
| P41222 | PTGDS |
| A6NGN9 | IGLON5 |
| P42857 | NSG1 |
| F5GWQ8 | CLUL1 |
| Q8N436 | CPXM2 |
| Q96FE5 | LINGO1 |
| Q495W5 | FUT11 |
| Q658N2 | WSCD1 |
| Q5JS37 | NHLRC3 |
| Q99784 | OLFM1 |
| Q8NFP4 | MDGA1 |
| Q96JP9 | CDHR1 |
| P08758 | ANXA5 |
| Q92484 | SMPDL3A |
| Q16849 | PTPRN |
| Q8WXD2 | SCG3 |
| O75326 | SEMA7A |
| Q86VZ4 | LRP11 |
| P02649 | APOE |
| Q 17R60 | IMPG1 |
| Q9UNW1 | MINPP1 |
| P08294 | SOD3 |
| P15848 | ARSB |

The biomarker of the present invention may preferably be: at least one gene selected from the group consisting of NTM, FSTL1, FGA, CSF1R, APOA4, CRP, HPRT1, MUCL1, AKR1B1, RARRES2, ALDOA and GSN; or a protein encoded thereby.

In the biomarkers of the present invention, at least one gene selected from the group shown in Table 12 below, or a protein encoded thereby may have a higher expression level than that in a normal control group:

**[Table 12]**

| Accession number | Gene name |
|---|---|
| Q9P121 | NTM |
| Q12841 | FSTL1 |
| P02671 | FGA |
| P07333 | CSF1R |
| P06727 | APOA4 |
| P02741 | CRP |
| Q9GZX9 | TWSG1 |
| Q9P0K1 | ADAM22 |
| O00468 | AGRN |
| P11047 | LAMC1 |
| Q9UBX7 | KLK11 |
| P98160 | HSPG2 |
| Q9GZP0 | PDGFD |
| P20774 | OGN |
| P16930 | FAH |
| Q92563 | SPOCK2 |
| Q16270 | IGFBP7 |
| 014672 | ADAM10 |
| Q969E1 | LEAP2 |
| Q5VZE7 | SPINK4 |
| Q8NBJ4 | GOLM1 |
| Q02985 | CFHR3 |
| P0DJI8 | SAA1 |
| P30043 | BLVRB |
| O95274 | LYPD3 |
| P49788 | RARRES1 |
| P02750 | LRG1 |
| P23142 | FBLN1 |
| P48745 | NOV |
| Q9NPY3 | CD93 |
| 015240 | VGF |
| Q08174 | PCDH1 |
| P07225 | PROS1 |
| Q14847 | LASP1 |
| Q99983 | OMD |
| P55289 | CDH12 |
| P27918 | CFP |
| P31431 | SDC4 |
| P24043 | LAMA2 |
| P12111 | COL6A3 |
| Q14515 | SPARCL1 |
| Q14766 | LTBP1 |
| P08185 | SERPINA6 |
| Q13231 | CHIT1 |
| 014773 | TPP1 |

Among the biomarkers of the present invention, preferably, at least one gene selected from the group consisting of NTM, FSTL1, FGA, CSF1R, APOA4 and CRP, or a protein encoded thereby may have a higher expression level than that in a normal control group.

Among the biomarkers of the present invention, at least one gene selected from the group shown in Table 13 below, or a protein encoded thereby may have a lower expression level than that in a normal control group:

**[Table 13]**

| Accession number | Gene name |
|---|---|
| P00492 | HPRT1 |
| Q96DR8 | MUCL1 |
| P15121 | AKR1B1 |
| Q99969 | RARRES2 |
| P04075 | ALDOA |
| P06396 | GSN |
| Q16568 | CARTPT |
| P26447 | S100A4 |
| P14625 | HSP90B1 |
| P08670 | VIM |
| Q86SF2 | GALNT7 |
| Q9UJJ9 | GNPTG |
| Q8NFY4 | SEMA6D |
| Q7Z7H5 | TMED4 |
| Q9Y646 | CPQ |
| Q9Y2I2 | NTNG1 |
| P40967 | PMEL |
| P07451 | CA3 |
| J3KNP4 | SEMA4B |
| O95336 | PGLS |
| P00441 | SOD1 |
| P10586 | PTPRF |
| Q86UD1 | OAF |
| P41222 | PTGDS |
| A6NGN9 | IGLON5 |
| P42857 | NSG1 |
| F5GWQ8 | CLUL1 |
| Q8N436 | CPXM2 |
| Q96FE5 | LINGO1 |
| Q495W5 | FUT11 |
| Q658N2 | WSCD 1 |
| Q5JS37 | NHLRC3 |
| Q99784 | OLFM1 |
| Q8NFP4 | MDGA1 |
| Q96JP9 | CDHR1 |
| P08758 | ANXA5 |
| Q92484 | SMPDL3A |
| Q16849 | PTPRN |
| Q8WXD2 | SCG3 |
| O75326 | SEMA7A |
| Q86VZ4 | LRP11 |
| P02649 | APOE |
| Q 17R60 | IMPG1 |
| Q9UNW1 | MINPP1 |
| P08294 | SOD3 |
| P15848 | ARSB |

Among the biomarkers of the present invention, preferably, at least one gene selected from the group consisting of HPRT1, MUCL1, AKR1B1, RARRES2, ALDOA and GSN, or a protein encoded thereby may have a lower expression level than that in a normal control group.

Another embodiment of the present invention is directed to a composition for diagnosing brain and nervous system diseases containing an agent capable of measuring the expression level of either at least one gene selected from the group shown in Table 1 above, or a protein encoded thereby.

In the present invention, the agent for measuring the expression level of the biomarker protein is not particularly limited, but may comprise, for example, at least one selected from the group consisting of antibodies, oligopeptides, ligands, peptide nucleic acids (PNAs) and aptamers, which bind specifically to the protein.

In the present invention, the "antibody" refers to a substance that specifically binds to an antigen and causes an antigen-antibody reaction. For the purposes of the present invention, the antibody refers to an antibody that specifically binds to the biomarker protein. Examples of the antibody of the present invention include all of polyclonal antibodies, monoclonal antibodies, and recombinant antibodies. The antibody may be readily produced using techniques well known in the art. For example, the polyclonal antibody may be produced by a method well known in the art, which includes the process of obtaining a serum containing the antibody by injecting the antigen of the biomarker protein into an animal and collecting blood from the animal. This polyclonal antibody may be produced from any animal such as goat, rabbit, sheep, monkey, horse, pig, cow, dog, or the like. In addition, the monoclonal antibody may be produced using the hybridoma method well known in the art (see Kohler and Milstein (1976) European Journal of Immunology 6:511-519), or the phage antibody library technology (see Clackson et al, Nature, 352:624-628, 1991; Marks et al, J. Mol. Biol., 222:58, 1-597, 1991). The antibody produced by the above method may be isolated and purified using a method such as gel electrophoresis, dialysis, salt precipitation, ion exchange chromatography, or affinity chromatography. In addition, examples of the antibody of the present disclosure include not only a complete form having two full-length light chains and two full-length heavy chains, but also functional fragments of an antibody molecule. "Functional fragment of an antibody molecule" refers to a fragment having at least an antigen-binding function, and examples thereof include Fab, F(ab'), F(ab')2 and Fv.

In the present invention, "PNA (Peptide Nucleic Acid)" refers to an artificially synthesized DNA or RNA-like polymer, which was first introduced by the Professors Nielsen, Egholm, Berg and Buchardt at University of Copenhagen, Denmark in 1991. DNA has a phosphate-ribose sugar backbone, but PNA has repeated N-(2-aminoethyl)-glycine backbones linked via peptide bonds, and thus has a significantly increased binding affinity for DNA or RNA and significantly increased stability. Thus, PNA is used for molecular biology, diagnostic assays and antisense therapies. The PNA is disclosed in detail in the literature [Nielsen PE, Egholm M, Berg RH, Buchardt O (December 1991). "Sequence-selective recognition of DNA by strand displacement with a thyminesubstituted polyamide". Science 254(5037): 1497-1500].

In the present invention, the "aptamer" refers to an oligonucleotide or a peptide molecule, and the general contents of the aptamer are disclosed in detail in the literature [Bock LC et al., Nature 355(6360):5646(1992); Hoppe-Seyler F, Butz K "Peptide aptamers: powerful new tools for molecular medicine". J Mol Med. 78(8):42630(2000); Cohen BA, Colas P, Brent R. "An artificial cell-cycle inhibitor isolated from a combinatorial library". Proc Natl Acad Sci USA. 95(24): 142727(1998)].

In the present invention, the agent for measuring the expression level of the gene encoding the biomarker protein may comprise at least one selected from the group consisting of primers, probes, and antisense nucleotides, which bind specifically to the gene encoding the biomarker protein.

In the present invention, the term "primer" refers to a fragment that recognizes a target gene sequence, and comprises a pair of forward and reverse primers, but is preferably a pair of primers providing analysis results with specificity and sensitivity. When the nucleic acid sequence of the primer is a sequence inconsistent with the non-target sequence present in the sample, and thus is a primer that amplifies only the target gene sequence containing the complementary primer binding site without inducing nonspecific amplification, high specificity may be imparted.

In the present invention, the term "probe" refers to a substance capable of binding specifically to a target substance to be detected in the sample, and refers to a substance capable of specifically detecting the presence of the target substance in the sample through the binding. The type of probe is not particularly limited so long as it is commonly used in the art. Preferably, the probe may be PNA (peptide nucleic acid), LNA (locked nucleic acid), a peptide, a polypeptide, a protein, an RNA or a DNA, and most preferably PNA. More specifically, the probe is a biomolecule derived from an organism or an analogue thereof, or is produced *in vitro.* Examples of the probe include an enzyme, a protein, an antibody, a microorganism, an animal and/or plant cell and organ, a neuron, DNA and RNA. Examples of the DNA include cDNA, genomic DNA, and an oligonucleotide, examples of the RNA include genomic RNA, mRNA and an oligonucleotide, and examples of the protein include antibodies, antigens, enzymes, peptides, and the like.

In the present invention, the term "LNA (locked nucleic acid)" refers to a nucleic acid analogue containing a 2'-O or 4'-C methylene bridge [J Weiler, J Hunziker and J Hall Gene Therapy (2006) 13, 496.502]. LNA nucleosides comprise the common bases of DNA and RNA, and can form base pairs according to the Watson-Crick base-pair rule. However, LNA fails to form an ideal shape in the Watson-Crick bond due to "locking" of the molecule attributable to the methylene bridge. When LNA is incorporated in a DNA or RNA oligonucleotide, it can more rapidly pair with a complementary nucleotide chain, thus increasing the stability of the double strand.

In the present invention, the term "antisense" means an oligomer that has a nucleotide sequence and a backbone between subunits, wherein an antisense oligomer is hybridized with the target sequence in the RNA by Watson-Crick base pairing to typically allow the formation of the mRNA and RNA:oligomer heterodimers in the target sequence. The oligomer may have an accurate or approximate sequence complementarity to the target sequence.

Information on the biomarker protein or the gene encoding the protein according to the present invention is known. Thus, based on this information, any person skilled in the art will be able to easily design a primer, probe or antisense nucleotide that binds specifically to the gene encoding the protein.

In the diagnostic composition of the present invention, as the biomarker gene or the protein encoded thereby, one present in the aqueous humor of the eye is preferred because it may increase the accuracy in diagnosing the onset of the brain and nervous system disease or in diagnosing the likelihood of developing the disease.

### Composition for Diagnosing Alzheimer's Disease

One embodiment of the present invention is directed to a composition for diagnosing Alzheimer's disease among brain and nervous system diseases containing: an agent for measuring the expression level of either at least one gene selected from the group shown in Table 2 above, or a protein encoded thereby.

The diagnostic composition of the present invention may preferably contain an agent capable of measuring the expression level of either at least one gene selected from the group consisting of ACHE, SPP1, EEF2, PRDX1, CES1, YWHAB, CNTN4 and BCHE, or a protein encoded thereby.

In the diagnostic composition of the present invention, when the expression level of either at least one gene selected from the group shown in Table 3 above, or a protein encoded thereby is higher than that in a normal control group, it can be diagnosed that the likelihood of developing brain and nervous system diseases, preferably Alzheimer's disease, is high.

In the diagnostic composition of the present invention, preferably, when the expression level of either at least one gene selected from the group consisting of ACHE, SPP1, EEF2, PRDX1 and CES1, or a protein encoded thereby is higher than that in a normal control group, it can be diagnosed that the likelihood of developing brain and nervous system diseases, preferably Alzheimer's disease, is high.

In the diagnostic composition of the present invention, when the expression level of either at least one gene selected from the group shown in Table 4 above, or a protein encoded thereby is lower than that in a normal control group, it can be diagnosed that the likelihood of developing brain and nervous system diseases, preferably Alzheimer's disease, is high.

In the diagnostic composition of the present invention, preferably, when the expression level of either at least one gene selected from the group consisting of YWHAB, CNTN4 and BCHE, or a protein encoded thereby is lower than that in a normal control group, it can be diagnosed that the likelihood of developing a brain and nervous system disease, preferably Alzheimer's disease, is high.

### Composition for Diagnosing Parkinson's Disease

Another embodiment of the present invention is directed to a composition for diagnosing Parkinson's disease among brain and nervous system diseases containing: an agent for measuring the expression level of either at least one gene selected from the group shown in Table 5 above, or a protein encoded thereby.

The diagnostic composition of the present invention may preferably contain an agent capable of measuring the expression level of either at least one gene selected from the group consisting of CACHD1, PCDH9, GAS1, H3F3B, FUT11, ATF6B, SELL, S100A6, LGALS1, GSR, NETO1 and CTGF, or a protein encoded thereby.

In the diagnostic composition of the present invention, when the expression level of either at least one gene selected from the group shown in Table 6 above, or a protein encoded thereby is higher than that in a normal control group, it can be diagnosed that the likelihood of developing brain and nervous system diseases, preferably Parkinson's disease, is high.

In the diagnostic composition of the present invention, preferably, when the expression level of either at least one gene selected from the group consisting of CACHD1, PCDH9, GAS1, H3F3B, FUT11 and ATF6B, or a protein encoded thereby is higher than that in a normal control group, it can be diagnosed that the likelihood of developing a brain and nervous system disease, preferably Parkinson's disease, is high.

In the diagnostic composition of the present invention, when the expression level of either at least one gene selected from the group shown in Table 7 above, or a protein encoded thereby is lower than that in a normal control group, it can be diagnosed that the likelihood of developing a brain and nervous system disease, preferably Parkinson's disease, is high.

### Composition for Diagnosing Cerebrovascular attack

Another embodiment of the present invention is directed to a composition for diagnosing cerebrovascular attack among brain and nervous system diseases containing: an agent for measuring the expression level of either at least one gene selected from the group shown in Table 8 above, or a protein encoded thereby.

The diagnostic composition of the present invention may preferably contain an agent capable of measuring the expression level of either at least one gene selected from the group consisting of PPIA, FAM19A4, KRT13, HSPE1, ANGPTL7, VIM, TFF1, MMRN1, NPVF, PRG4, ADGRL1 and MDK, or a protein encoded thereby.

In the diagnostic composition of the present invention, when the expression level of either at least one gene selected from the group shown in Table 9 above, or a protein encoded thereby is higher than that in a normal control group, it can be diagnosed that the likelihood of developing a brain and nervous system disease, preferably cerebrovascular attack, is high.

In the diagnostic composition of the present invention, preferably, when the expression level of either at least one gene selected from the group consisting of PPIA, FAM19A4, KRT13, HSPE1, ANGPTL7 and VIM, or a protein encoded thereby is higher than that in a normal control group, it can be diagnosed that the likelihood of developing a brain and nervous system disease, preferably cerebrovascular attack, is high.

In the diagnostic composition of the present invention, when the expression level of either at least one gene selected from the group shown in Table 10 above, or a protein encoded thereby is lower than that in a normal control group, it can be diagnosed that the likelihood of developing a brain and nervous system disease, preferably cerebrovascular attack, is high.

In the diagnostic composition of the present invention, preferably, when the expression level of either at least one gene selected from the group consisting of TFF1, MMRN1, NPVF, PRG4, ADGRL1 and MDK, or a protein encoded thereby is lower than that in a normal control group, it can be diagnosed that the likelihood of developing a brain and nervous system disease, preferably cerebrovascular attack, is high.

### Composition for Diagnosing Brain Tumor

Another embodiment of the present invention is directed to a composition for diagnosing brain tumor among brain and nervous system diseases containing: an agent for measuring the expression level of either at least one gene selected from the group shown in Table 11 above, or a protein encoded thereby.

The diagnostic composition of the present invention may preferably contain an agent capable of measuring the expression level of either at least one gene selected from the group consisting of NTM, FSTL1, FGA, CSF1R, APOA4, CRP, HPRT1, MUCL1, AKR1B1, RARRES2, ALDOA and GSN, or a protein encoded thereby.

In the diagnostic composition of the present invention, when the expression level of either at least one gene selected from the group shown in Table 12 above, or a protein encoded thereby is higher than that in a normal control group, it can be diagnosed that the likelihood of developing a brain and nervous system disease, preferably brain tumor, is high.

In the diagnostic composition of the present invention, preferably, when the expression level of either at least one gene selected from the group consisting of NTM, FSTL1, FGA, CSF1R, APOA4 and CRP, or a protein encoded thereby is higher than that in a normal control group, it can be diagnosed that the likelihood of developing a brain and nervous system disease, preferably brain tumor, is high.

In the diagnostic composition of the present invention, when the expression level of either at least one gene selected from the group shown in Table 13 above, or a protein encoded thereby is lower than that in a normal control group, it can be diagnosed that the likelihood of developing a brain and nervous system disease, preferably brain tumor, is high.

In the diagnostic composition of the present invention, preferably, when the expression level of either at least one gene selected from the group consisting of HPRT1, MUCL1, AKR1B1, RARRES2, ALDOA and GSN, or a protein encoded thereby is lower than that in a normal control group, it can be diagnosed that the likelihood of developing a brain and nervous system disease, preferably brain tumor, is high.

Another embodiment of the present invention is directed to a kit for diagnosing a brain and nervous system disease comprising the composition for diagnosing a brain and nervous system disease according to the present invention.

According to the present invention, it is possible to diagnose the onset of a brain and nervous system disease or the likelihood of developing the disease, at early stages by using the diagnostic kit.

Details regarding to the diagnostic compositions of the present invention and brain and nervous system diseases overlap with those described in the diagnostic compositions of the present invention, and thus detailed description thereof will be omitted to avoid excessive complexity of the specification.

In the present invention, the kit may be an RT-PCR kit, a DNA chip kit, an ELISA kit, a protein chip kit, a rapid kit, or a multiple-reaction monitoring (MRM) kit, but is not limited thereto.

The diagnostic kit of the present disclosure may further comprise one or more other component compositions, solutions or devices suitable for the analysis method.

For example, the diagnostic kit according to the present invention may further comprise essential elements required for performing a reverse transcription polymerase chain reaction. The reverse transcription polymerase chain reaction kit comprises a primer pair specific for the gene encoding the marker protein. The primer is an oligonucleotide having a sequence specific for the nucleic acid sequence of the gene, and may have a length of about 7 bp to 50 bp, more preferably about 10 bp to 30 bp. The kit may also comprise a primer specific for the nucleic acid sequence of the control gene. In addition, the reverse transcription polymerase chain reaction kit may comprise test tubes or other appropriate containers, reaction buffers (at various pHs and magnesium concentrations), deoxynucleotides (dNTPs), enzymes such as Taq-polymerase and reverse transcriptase, DNase and/or RNase inhibitors, DEPC water, sterile water, and the like.

In addition, the diagnostic kit of the present invention may comprise essential elements required for performing DNA chip assay. The DNA chip kit may comprise a substrate to which a cDNA or oligonucleotide corresponding to a gene or a fragment thereof is attached, and a reagent, an agent, an enzyme, and the like for producing a fluorescent-labeled probe. The substrate may also comprise a cDNA or oligonucleotide corresponding to a control gene or a fragment thereof.

In addition, the diagnostic kit of the present invention may comprise essential elements required for performing ELISA. The ELISA kit comprises an antibody specific for the protein. The antibody has high specificity and affinity for the marker protein and little cross-reactivity with other proteins, and is a monoclonal antibody, a polyclonal antibody or a recombinant antibody. The ELISA kit may also comprise an antibody specific for the control protein. In addition, the ELISA kit may comprise reagents capable of detecting the bound antibody, such as a labeled secondary antibody, chromophores, an enzyme (e.g., conjugated to an antibody) and substrates thereof, or other substances that may bind to the antibody.

Another embodiment of the present invention is directed to a method for providing information for diagnosing a brain and nervous system disease, the method comprising a step of measuring the expression level of either at least one gene selected from the group shown in Table 1 above, or a protein encoded thereby, in a biological sample isolated from a subject of interest.

In the present invention, the term "subject of interest" refers to a subject whose onset of a brain and nervous system disease is uncertain and who is likely to develop the disease.

In the present invention, the "biological sample" refers to any material, biological fluid, tissue or cells obtained from or derived from the subject. Preferably, the biological sample is the aqueous humor of the eye because it can increase the accuracy in diagnosing the brain and nervous system disease.

The method of the present invention may comprise a step of measuring the expression levels of the biomarker proteins or genes encoding the same, listed in Table 1 above, in the biological sample isolated as described above.

In the present disclosure, the agent for measuring the expression level of the protein encoded by the biomarker protein is not particularly limited, but may preferably comprise at least one selected from the group consisting of antibodies, oligopeptides, ligands, PNAs (peptide nucleic acids) and aptamers, which bind specifically to the protein encoded by the biomarker gene.

In the present disclosure, methods for measuring or comparatively analyzing the expression level of the protein encoded by the biomarker gene include, but are not limited to, protein chip analysis, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) assay, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, and enzyme-linked immunosorbent assay (ELISA).

In the present invention, the agent for measuring the expression level of the biomarker gene may comprise at least one selected from the group consisting of primers, probes and antisense oligonucleotides, which bind specifically to the biomarker gene.

In the present invention, analysis methods of measuring the expression level of the biomarker gene to confirm the presence or expression level of the gene include, but are not limited to, reverse transcription polymerase chain reaction (RT-PCR), competitive RT-PCR, real-time RT-PCR, RNase protection assay (RPA), Northern blotting, or DNA chip assay.

The method of the present invention may comprise a step of predicting that the likelihood of developing the brain and nervous system disease is high, when the expression level of either the biomarker gene or the protein encoded thereby, measured in the biological sample from the subject, is higher or lower than the expression level in the normal control group.

### Method for Providing Information for Diagnosing Alzheimer's Disease

The step of measuring the expression level may comprise a step of measuring the expression level of either at least one gene selected from the group shown in Table 2 above, or a protein encoded thereby.

In the method for providing information according to the present invention, the step of measuring the expression level may preferably be performed by measuring the expression level of either at least one gene selected from the group consisting of ACHE, SPP1, EEF2, PRDX1, CES1, YWHAB, CNTN4 and BCHE, or a protein encoded thereby.

In the method for providing information according to the present invention, when the expression level of either at least one gene selected from the group shown in Table 3 above, or a protein encoded thereby is higher than that in a normal control group, it may be predicted that the likelihood of developing brain and nervous system diseases, preferably Alzheimer's disease, is high.

In the method for providing information according to the present invention, preferably, when the expression level of either at least one gene selected from the group consisting of ACHE, SPP1, EEF2, PRDX1 and CES1, or a protein encoded thereby is higher than that in a normal control group, it may be predicted that the likelihood of developing brain and nervous system diseases, preferably Alzheimer's disease, is high.

In the method for providing information according to the present invention, when the expression level of either at least one gene selected from the group shown in Table 4 above, or a protein encoded thereby is lower than that in a normal control group, it may be predicted that the likelihood of developing brain and nervous system diseases, preferably Alzheimer's disease, is high.

In the method for providing information according to the present invention, preferably, when the expression level of either at least one gene selected from the group consisting of YWHAB, CNTN4 and BCHE, or a protein encoded thereby is lower than that in a normal control group, it may be predicted that the likelihood of developing brain and nervous system diseases, preferably Alzheimer's disease, is high.

Furthermore, the method of the present invention may further comprise a step of subjecting the subject of interest to appropriate treatment such as administration of a drug for the disease, when it is predicted or diagnosed that the likelihood of developing brain and nervous system diseases, preferably Alzheimer's disease, is high as described above.

When the method of the present invention is used, it is possible to diagnose the onset of a brain and nervous system disease or the likelihood of developing the disease, and to track the progress or prognosis of the disease or the therapeutic effect against the disease.

### Method for Providing Information for Diagnosing Parkinson's Disease

The step of measuring the expression level may comprise a step of measuring the expression level of either at least one gene selected from the group shown in Table 5 above, or a protein encoded thereby.

In the method for providing information according to the present invention, the step of measuring the expression level may preferably be performed by measuring the expression level of either at least one gene selected from the group consisting of CACHD1, PCDH9, GAS1, H3F3B, FUT11, ATF6B, SELL, S100A6, LGALS1, GSR, NETO1 and CTGF, or a protein encoded thereby.

In the method for providing information according to the present invention, when the expression level of either at least one gene selected from the group shown in Table 6 above, or a protein encoded thereby is higher than that in a normal control group, it may be predicted that the likelihood of developing brain and nervous system diseases, preferably Parkinson's disease, is high.

In the method for providing information according to the present invention, preferably. when the expression level of either at least one gene selected from the group consisting of CACHD1, PCDH9, GAS1, H3F3B, FUT11 and ATF6B, or a protein encoded thereby is higher than that in a normal control group, it may be predicted that the likelihood of developing brain and nervous system diseases, preferably Parkinson's disease, is high.

In the method for providing information according to the present invention, when the expression level of either at least one gene selected from the group shown in Table 7 above, or a protein encoded thereby is lower than that in a normal control group, it may be predicted that the likelihood of developing brain and nervous system diseases, preferably Parkinson's disease, is high.

Furthermore, the method of the present invention may further comprise a step of subjecting the subject of interest to appropriate treatment such as administration of a drug for the disease, when it is predicted or diagnosed that the likelihood of developing brain and nervous system diseases, particularly, Parkinson's disease, is high as described above.

When the method of the present invention is used, it is possible to diagnose the onset of the brain and nervous system disease or the likelihood of developing the disease, and to track the progress or prognosis of the disease or the therapeutic effect against the disease.

### Method for Providing Information for Diagnosing Cerebrovascular attack

In the method for providing information according to the present invention, the step of measuring the expression level may comprise a step of measuring the expression level of either at least one gene selected from the group shown in Table 8 above, or a protein encoded thereby.

In the method for providing information according to the present invention, the step of measuring the expression level may preferably be performed by measuring the expression level of either at least one gene selected from the group consisting of PPIA, FAM19A4, KRT13, HSPE1, ANGPTL7, VIM, TFF1, MMRN1, NPVF, PRG4, ADGRL1 and MDK, or a protein encoded thereby.

In the method for providing information according to the present invention, when the expression level of either at least one gene selected from the group shown in Table 9 above, or a protein encoded thereby is higher than that in a normal control group, it may be predicted that the likelihood of developing brain and nervous system diseases, preferably cerebrovascular attack, is high.

In the method for providing information according to the present invention, preferably, when the expression level of either at least one gene selected from the group consisting of PPIA, FAM19A4, KRT13, HSPE1, ANGPTL7 and VIM, or a protein encoded thereby is higher than that in a normal control group, it may be predicted that the likelihood of developing brain and nervous system diseases, preferably cerebrovascular attack, is high.

In the method for providing information according to the present invention, when the expression level of either at least one gene selected from the group shown in Table 10 above, or a protein encoded thereby is lower than that in a normal control group, it may be predicted that the likelihood of developing brain and nervous system diseases, preferably cerebrovascular attack, is high.

In the method for providing information according to the present invention, preferably, when the expression level of either at least one gene selected from the group consisting of TFF1, MMRN1, NPVF, PRG4, ADGRL1 and MDK, or a protein encoded thereby is lower than that in a normal control group, it may be predicted that the likelihood of developing brain and nervous system diseases, preferably cerebrovascular attack, is high.

Furthermore, the method of the present invention may further comprise a step of subjecting the subject of interest to appropriate treatment such as administration of a drug for the disease, when it is predicted or diagnosed that the likelihood of developing brain and nervous system diseases, particularly cerebrovascular attack, is high as described above.

When the method of the present invention is used, it is possible to diagnose the onset of a brain and nervous system disease or the likelihood of developing the disease, and to track the progress or prognosis of the disease or the therapeutic effect against the disease.

### Method for Providing Information for Diagnosing Brain Tumor

In the method for providing information according to the present invention, the step of measuring the expression level may comprise a step of measuring the expression level of either at least one gene selected from the group shown in Table 11 above, or a protein encoded thereby.

In the method for providing information according to the present invention, the step of measuring the expression level may preferably be performed by measuring the expression level of either at least one gene selected from the group consisting of NTM, FSTL1, FGA, CSF1R, APOA4, CRP, HPRT1, MUCL1, AKR1B1, RARRES2, ALDOA and GSN, or a protein encoded thereby.

In the method for providing information according to the present invention, when the expression level of either at least one gene selected from the group shown in Table 12 above, or a protein encoded thereby is higher than that in a normal control group, it may be predicted that the likelihood of developing brain and nervous system diseases, preferably brain tumor, is high.

In the method for providing information according to the present invention, preferably, when the expression level of either at least one gene selected from the group consisting of NTM, FSTL1, FGA, CSF1R, APOA4 and CRP, or a protein encoded thereby is higher than that in a normal control group, it may be predicted that the likelihood of developing brain and nervous system diseases, preferably brain tumor, is high.

In the method for providing information according to the present invention, when the expression level of either at least one gene selected from the group shown in Table 13 above, or a protein encoded thereby is lower than that in a normal control group, it may be predicted that the likelihood of developing brain and nervous system diseases, preferably brain tumor, is high.

In the method for providing information according to the present invention, preferably, when the expression level of either at least one gene selected from the group consisting of HPRT1, MUCL1, AKR1B1, RARRES2, ALDOA and GSN, or a protein encoded thereby is lower than that in a normal control group, it may be predicted that the likelihood of developing brain and nervous system diseases, preferably brain tumor, is high.

Furthermore, the method of the present invention may further comprise a step of subjecting the subject of interest to appropriate treatment such as administration of a drug for the disease, when it is predicted or diagnosed that the likelihood of developing brain and nervous system diseases, particularly brain tumor, is high as described above.

When the method of the present invention is used, it is possible to diagnose the onset of a brain and nervous system disease or the likelihood of developing the disease, and to track the progress or prognosis of the disease or the therapeutic effect against the disease.

Another embodiment of the present invention is directed to a method for screening a drug that induces a brain and nervous system disease, the method comprising steps of: treating an isolated biological sample with a candidate substance expected to induce the brain and nervous system disease; and

measuring the expression level of either at least one gene selected from the group shown in Table 1 above, or a protein encoded thereby, in the biological sample treated with the candidate substance.

In the present invention, the isolated biological sample may be a biological sample isolated from a subject with or without a brain and nervous system disease. Specifically, the biological sample is preferably the aqueous humor of the eye.

In addition, in the present invention, the candidate substance comprises any substance, molecule, element, compound, entity, or a combination thereof. Examples of the candidate substance include, but are not limited to, proteins, polypeptides, small organic molecules, polysaccharides, polynucleotides, and the like. In addition, the candidate substance may also be a natural product, a synthetic compound, or a combination of two or more substances.

In the present invention, the method may comprise a step of determining that the candidate substance is an inducer of a brain and nervous system disease, when the expression level of either the biomarker gene or a protein encoded thereby in the biological sample after treatment with the candidate substance is higher or lower than that before treatment with the candidate substance.

### Method for Screening Inducer of Alzheimer's Disease

In the screening method of the present invention, the step of measuring the expression level may be performed by measuring the expression level of either at least one gene selected from the group shown in Table 2 above, or a protein encoded thereby.

In the screening method of the present invention, the step of measuring the expression level may be performed by measuring the expression level of either at least one gene selected from the group consisting of ACHE, SPP1, EEF2, PRDX1 and CES1, or a protein encoded thereby.

In the screening method of the present invention, when the expression level of either at least one selected from the group shown in Table 3 above, or a protein encoded thereby is higher than that before treatment with the candidate substance, it may be determined that the candidate substance is an inducer of a brain and nervous system disease, preferably an inducer of Alzheimer's disease.

In the screening method of the present invention, preferably, when the expression level of either at least one gene selected from the group consisting of ACHE, SPP1, EEF2, PRDX1 and CES1, or a protein encoded thereby is higher than that before treatment with the candidate substance, it may be determined that the candidate substance is an inducer of a brain and nervous system disease, preferably an inducer of Alzheimer's disease.

In the screening method of the present invention, when the expression level of either at least one gene selected from the group shown in Table 4 above, or a protein encoded thereby is lower than that before treatment with the candidate substance, it may be determined that the candidate substance is an inducer of a brain and nervous system disease, preferably an inducer of Alzheimer's disease.

In the screening method of the present invention, preferably, when the expression level of either at least one gene selected from the group consisting of YWHAB, CNTN4 and BCHE, or a protein encoded thereby is lower than that before treatment with the candidate substance, it may be determined that the candidate substance is an inducer of a brain and nervous system disease, preferably an inducer of Alzheimer's disease.

### Method for Screening Inducer of Parkinson's Disease

In the screening method of the present invention, the step of measuring the expression level may be performed by measuring the expression level of either at least one gene selected from the group shown in Table 5 above, or a protein encoded thereby.

In the screening method of the present invention, the step of measuring the expression level may be performed by measuring the expression level of either at least one gene selected from the group consisting of CACHD1, PCDH9, GAS1, H3F3B, FUT11, ATF6B, SELL, S100A6, LGALS1, GSR, NETO1 and CTGF, or a protein encoded thereby.

In the screening method of the present invention, when the expression level of either at least one gene selected from the group shown in Table 6 above, or a protein encoded thereby is higher than that before treatment with the candidate substance, it may be determined that the candidate substance is an inducer of a brain and nervous system disease, preferably an inducer of Parkinson's disease.

In the screening method of the present invention, preferably, when the expression level of either at least one gene selected from the group consisting of CACHD1, PCDH9, GAS1, H3F3B, FUT11 and ATF6B, or a protein encoded thereby is higher than that before treatment with the candidate substance, it may be determined that the candidate substance is an inducer of a brain and nervous system disease, preferably an inducer of Parkinson's disease.

In the screening method of the present invention, when the expression level of either at least one gene selected from the group shown in Table 7 above, or a protein encoded thereby is lower than that before treatment with the candidate substance, it may be determined that the candidate substance is an inducer of a brain and nervous system disease, preferably an inducer of Parkinson's disease.

### Method for Screening Inducer of Cerebrovascular attack

In the screening method of the present invention, the step of measuring the expression level may be performed by measuring the expression level of either at least one gene selected from the group shown in Table 8 above, or a protein encoded thereby.

In the screening method of the present invention, the step of measuring the expression level may be performed by measuring the expression level of either at least one gene selected from the group consisting of PPIA, FAM19A4, KRT13, HSPE1, ANGPTL7, VIM, TFF1, MMRN1, NPVF, PRG4, ADGRL1 and MDK, or a protein encoded thereby.

In the screening method of the present invention, when the expression level of either at least one gene selected from the group shown in Table 9 above, or a protein encoded thereby is higher than that before treatment with the candidate substance, it may be determined that the candidate substance is an inducer of a brain and nervous system disease, preferably an inducer of cerebrovascular attack.

In the screening method of the present invention, preferably, when the expression level of either at least one gene selected from the group consisting of PPIA, FAM19A4, KRT13, HSPE1, ANGPTL7 and VIM, or a protein encoded thereby is higher than that before treatment with the candidate substance, it may be determined that the candidate substance is an inducer of a brain and nervous system disease, preferably an inducer of cerebrovascular attack.

In the screening method of the present invention, when the expression level of either at least one gene selected from the group shown in Table 10 above, or a protein encoded thereby is lower than that before treatment with the candidate substance, it may be determined that the candidate substance is an inducer of a brain and nervous system disease, preferably an inducer of cerebrovascular attack.

In the screening method of the present invention, preferably, when the expression level of either at least one gene selected from the group consisting of TFF1, MMRN1, NPVF, PRG4, ADGRL1 and MDK, or a protein encoded thereby is lower than that before treatment with the candidate substance, it may be determined that the candidate substance is an inducer of a brain and nervous system disease, preferably an inducer of cerebrovascular attack.

### Method for Screening Inducer of Brain Tumor

In the screening method of the present invention, the step of measuring the expression level may be performed by measuring the expression level of either at least one gene selected from the group shown in Table 11 above, or a protein encoded thereby.

In the screening method of the present invention, the step of measuring the expression level may be performed by measuring the expression level of either at least one gene selected from the group consisting of NTM, FSTL1, FGA, CSF1R, APOA4, CRP, HPRT1, MUCL1, AKR1B1, RARRES2, ALDOA and GSN, or a protein encoded thereby.

In the screening method of the present invention, when the expression level of either at least one gene selected from the group shown in Table 12 above, or a protein encoded thereby is higher than that before treatment with the candidate substance, it may be determined that the candidate substance is an inducer of a brain and nervous system disease, preferably an inducer of brain tumor.

In the screening method of the present invention, preferably, when the expression level of either at least one gene selected from the group consisting of NTM, FSTL1, FGA, CSF1R, APOA4 and CRP, or a protein encoded thereby is higher than that before treatment with the candidate substance, it may be determined that the candidate substance is an inducer of a brain and nervous system disease, preferably an inducer of brain tumor.

In the screening method of the present invention, when the expression level of either at least one gene selected from the group shown in Table 13 above, or a protein encoded thereby is lower than that before treatment with the candidate substance, it may be determined that the candidate substance is an inducer of a brain and nervous system disease, preferably an inducer of brain tumor.

In the screening method of the present invention, preferably, when the expression level of either at least one gene selected from the group consisting of HPRT1, MUCL1, AKR1B1, RARRES2, ALDOA and GSN, or a protein encoded thereby is lower than that before treatment with the candidate substance, it may be determined that the candidate substance is an inducer of a brain and nervous system disease, preferably an inducer of brain tumor.

In the screening method of the present invention, details regarding the agent for measuring the expression level and the method for measuring the expression level overlap with those described in the method for providing information for diagnosis according to the present invention, and thus description thereof will be herein omitted to avoid excessive complexity of the specification.

### Advantageous Effects

According to the present invention, it is possible to diagnose, at an early stage, the onset of a brain and nervous system disease or the likelihood of developing the disease or diagnose the progress or prognosis of the disease or the therapeutic effect against the disease, by measuring the expression level of the biomarker protein of the present invention or a gene encoding the same in the aqueous humor of the eye.

### Brief Description of Drawings

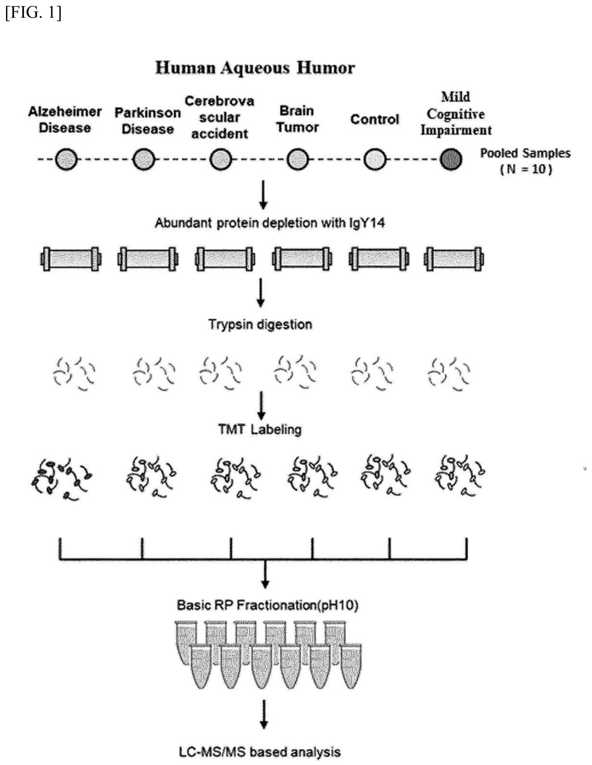

FIG. 1 shows an experimental design of Example 1 of the present invention.

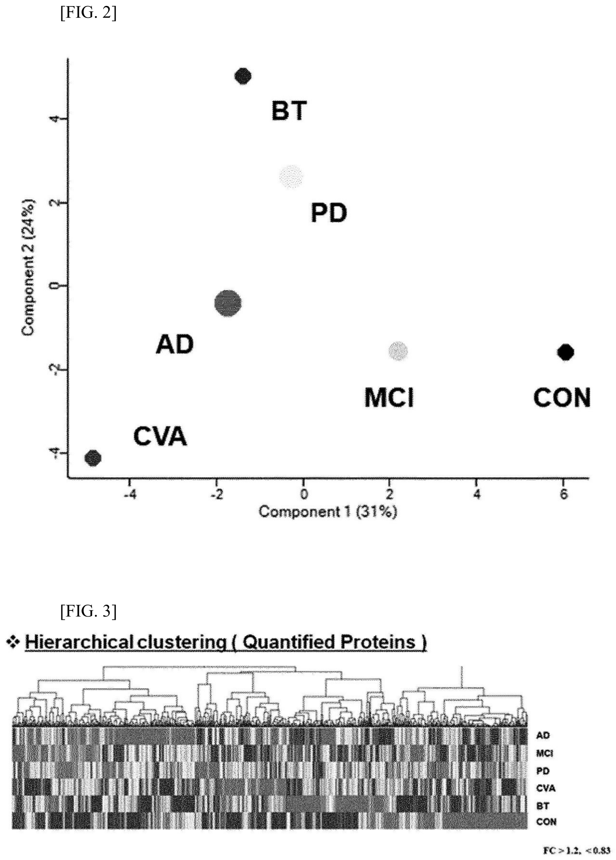

FIG. 2 shows the results of performing principal component analysis (PCA) of biomarker proteins whose expression levels changed specifically in aqueous humor from Alzheimer's disease (AD), Parkinson's disease (PD), cerebrovascular attack (CVA) and brain tumor (BT) patients, in Example 1 of the present invention.
FIG. 3 shows the results of performing hierarchical clustering analysis of biomarker proteins whose expression levels changed specifically in aqueous humor from Alzheimer's disease (AD), Parkinson's disease (PD), cerebrovascular attack (CVA) and brain tumor (BT) patients, in Example 1 of the present invention.

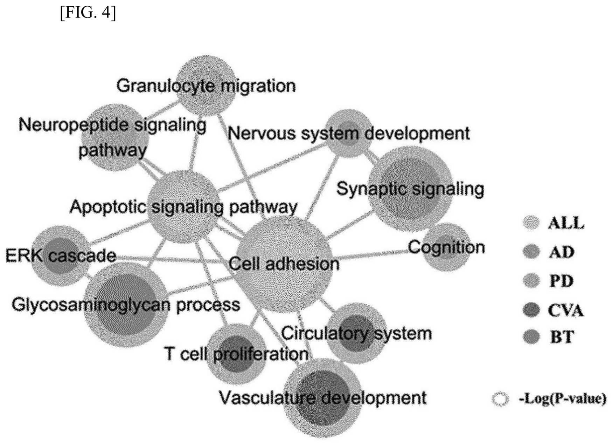

FIG. 4 shows the results of performing KEGG and GOBP analysis of biomarker proteins whose expression levels changed specifically in aqueous humor from Alzheimer's disease (AD), Parkinson's disease (PD), cerebrovascular attack (CVA) and brain tumor (BT) patients, in Example 1 of the present invention.

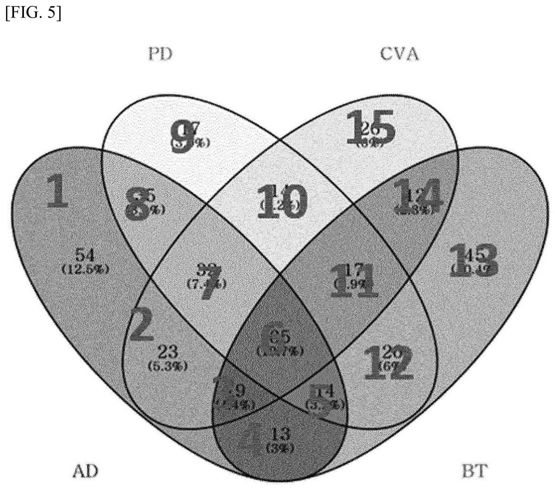

FIG. 5 is a diagram showing the results of examining whether biomarker proteins whose expression levels were upregulated specifically in aqueous humor from Alzheimer's disease (AD), Parkinson's disease (PD), cerebrovascular attack (CVA) and brain tumor (BT) patients overlap between the groups, in Example 1 of the present invention.

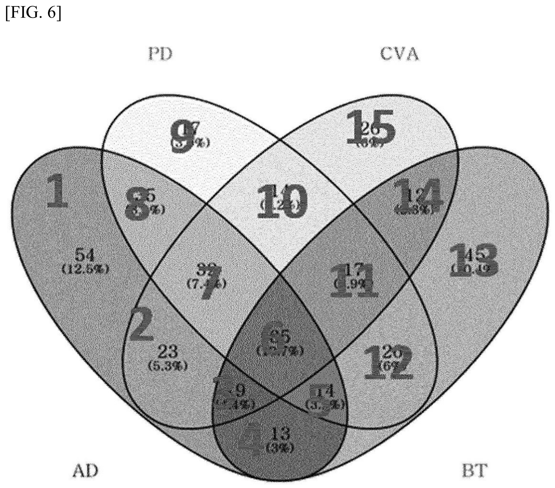

FIG. 6 is a diagram showing the results of examining whether biomarker proteins whose expression levels were downregulated specifically in aqueous humor from Alzheimer's disease (AD), Parkinson's disease (PD), cerebrovascular attack (CVA) and brain tumor (BT) patients overlap between the groups, in Example 1 of the present invention.

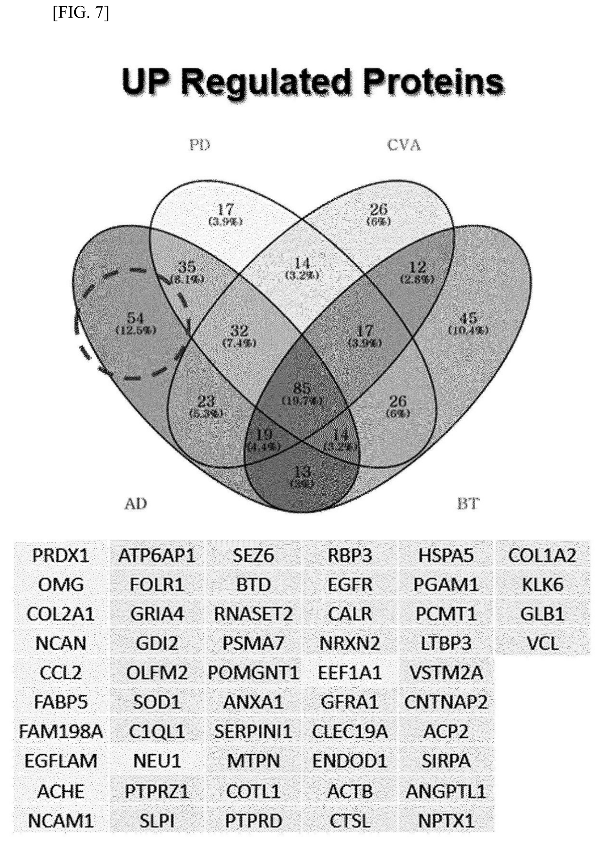

FIG. 7 is a diagram showing the results of examining whether biomarker proteins whose expression levels were upregulated specifically in aqueous humor from Alzheimer's disease (AD) patients overlap between the groups, in Example 1 of the present invention.

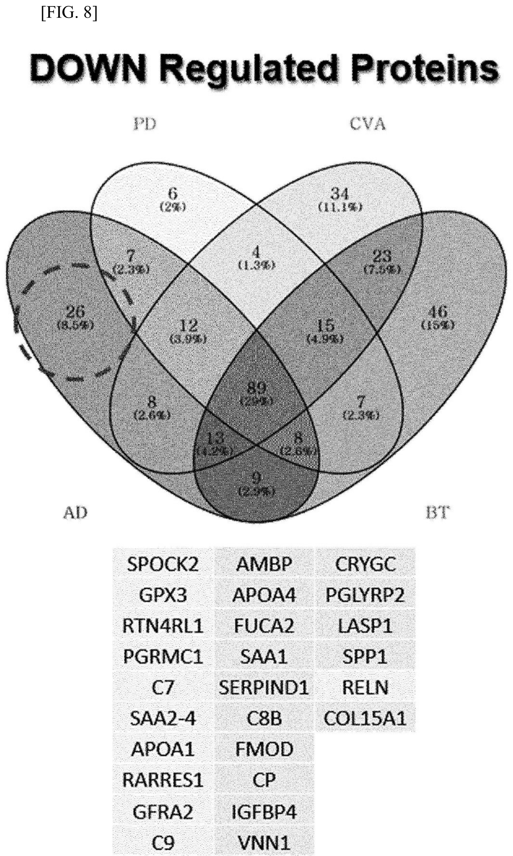

FIG. 8 is a diagram showing the results of examining whether biomarker proteins whose expression levels were downregulated specifically in aqueous humor from Alzheimer's disease (AD) patients overlap between the groups, in Example 1 of the present invention.

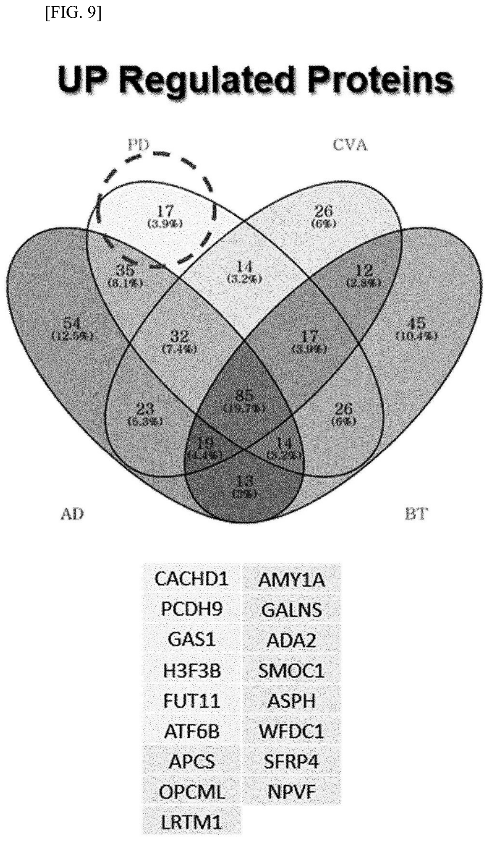

FIG. 9 is a diagram showing the results of examining whether biomarker proteins whose expression levels were upregulated specifically in aqueous humor from Parkinson's disease (PD) patients overlap between the groups, in Example 1 of the present invention.

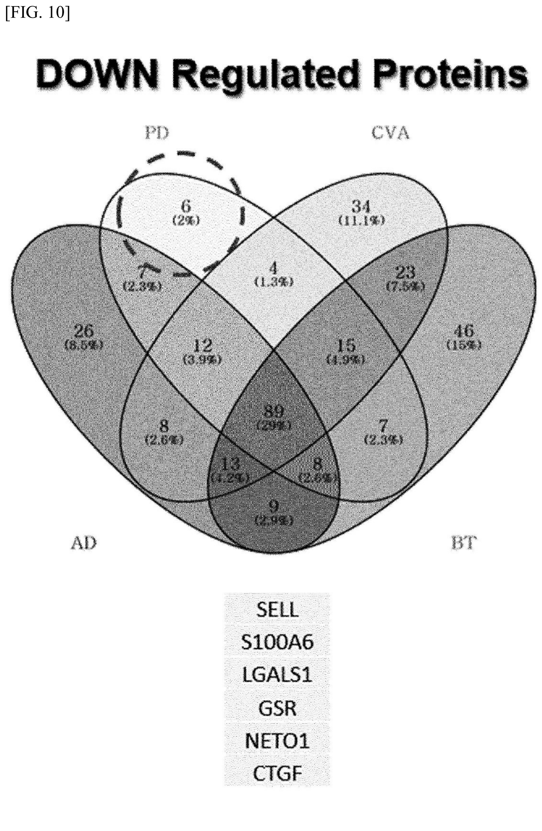

FIG. 10 is a diagram showing the results of examining whether biomarker proteins whose expression levels were downregulated specifically in aqueous humor from Parkinson's disease (PD) patients overlap between the groups, in Example 1 of the present invention.

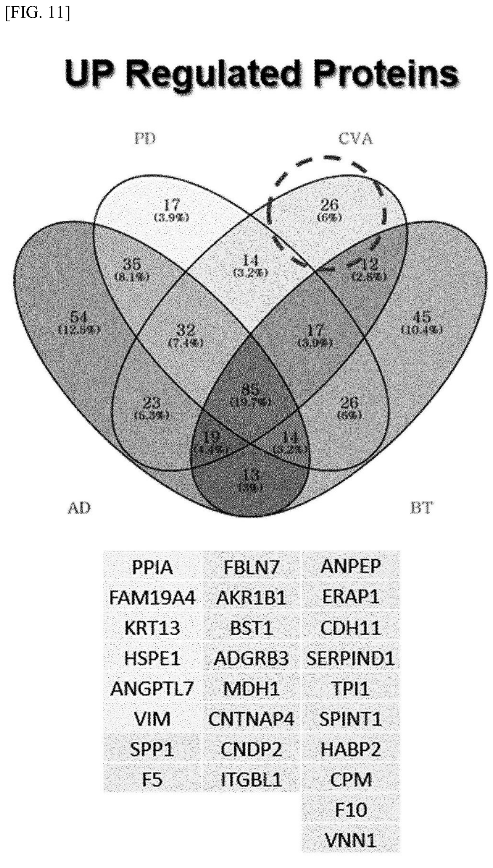

FIG. 11 is a diagram showing the results of examining whether biomarker proteins whose expression levels were upregulated specifically in aqueous humor from cerebrovascular attack (CVA) patients overlap between the groups, in Example 1 of the present invention.

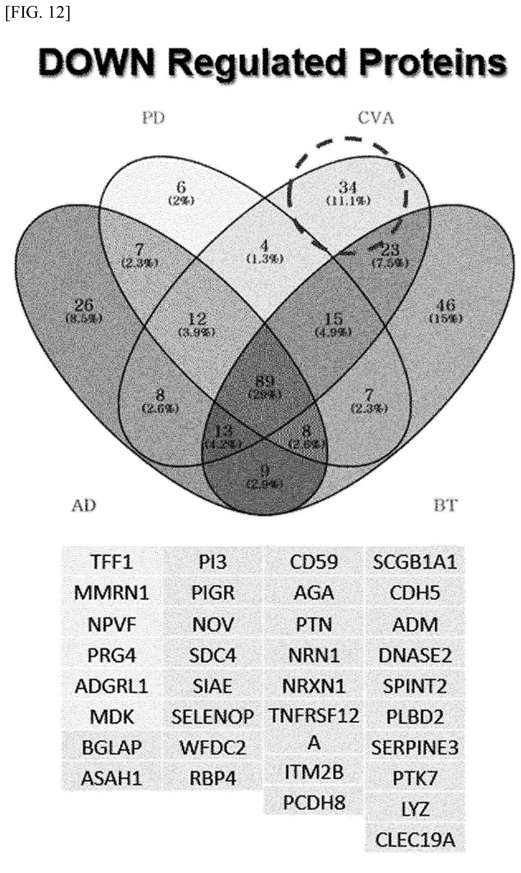

FIG. 12 is a diagram showing the results of examining whether biomarker proteins whose expression levels were downregulated specifically in aqueous humor from cerebrovascular attack (CVA) patients overlap between the groups, in Example 1 of the present invention.

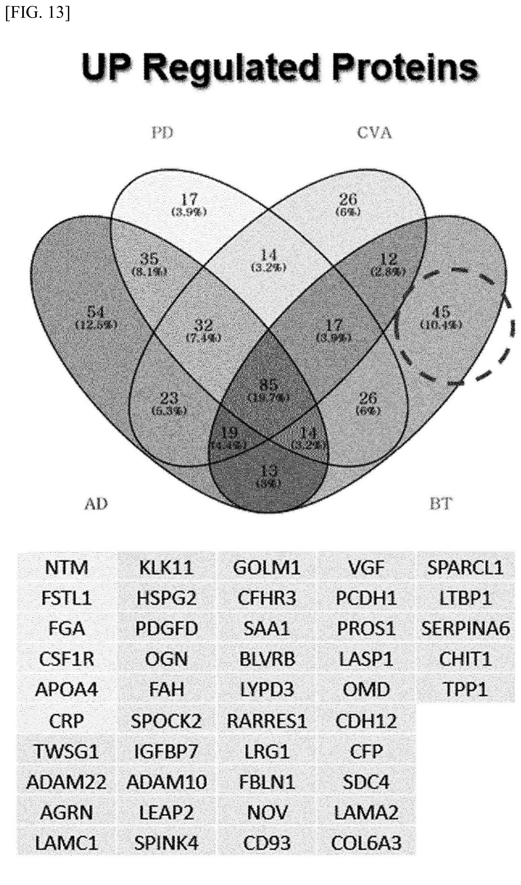

FIG. 13 is a diagram showing the results of examining whether biomarker proteins whose expression levels were upregulated specifically in aqueous humor from brain tumor (BT) patients overlap between the groups, in Example 1 of the present invention.

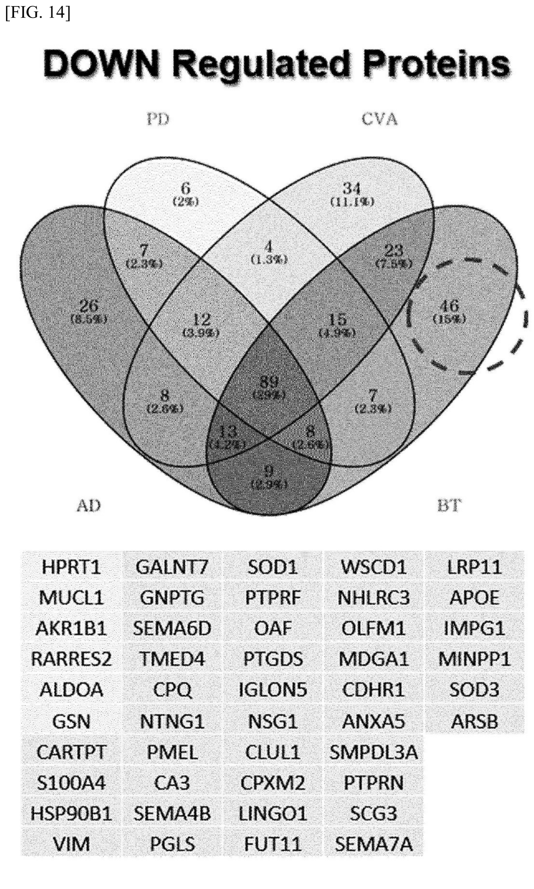

FIG. 14 is a diagram showing the results of examining whether biomarker proteins whose expression levels were downregulated specifically in aqueous humor from brain tumor (BT) patients overlap between the groups, in Example 1 of the present invention.

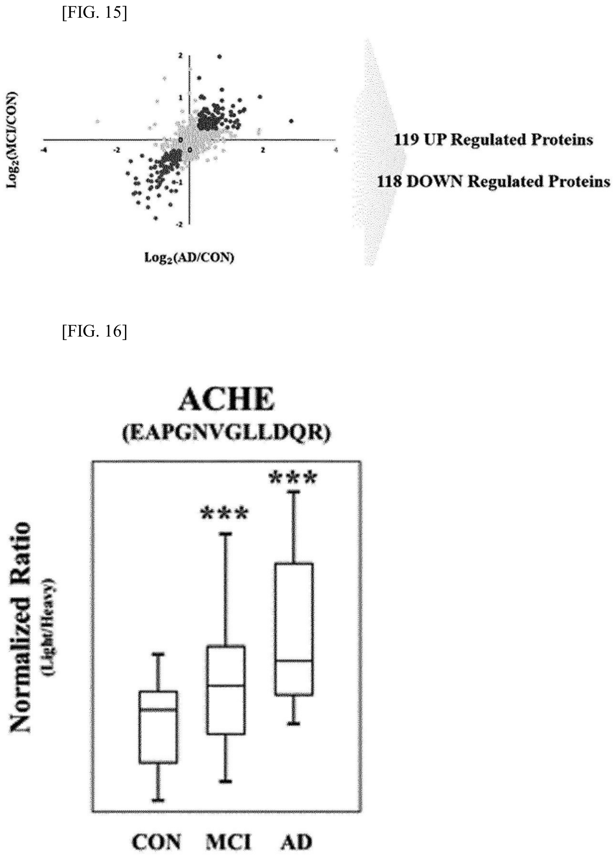

FIG. 15 shows the results of analyzing the distribution of biomarker proteins whose expression levels were upregulated or downregulated commonly in aqueous humor from Alzheimer's disease (AD) patients and patients with mild cognitive impairment (MCI) which is a pre-stage of Alzheimer's disease, in Example 2 of the present invention.
FIG. 16 shows the results of analyzing changes in the expression levels of ACHE protein in aqueous humor from a normal control group (CON), patients with mild cognitive impairment (MCI) which is a pre-stage of Alzheimer's disease, and Alzheimer's disease (AD) patients, in Example 2 of the present invention.

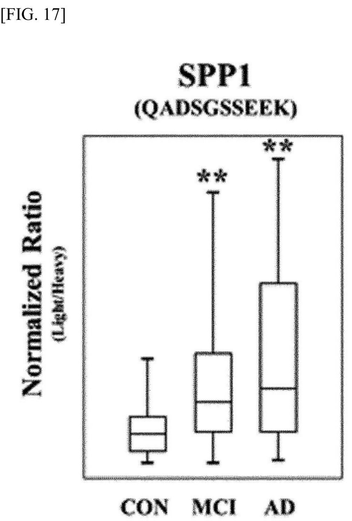

FIG. 17 shows the results of analyzing changes in the expression levels of SPP1 protein in aqueous humor from a normal control group (CON), patients with mild cognitive impairment (MCI) which is a pre-stage of Alzheimer's disease, and Alzheimer's disease (AD) patients, in Example 2 of the present invention.

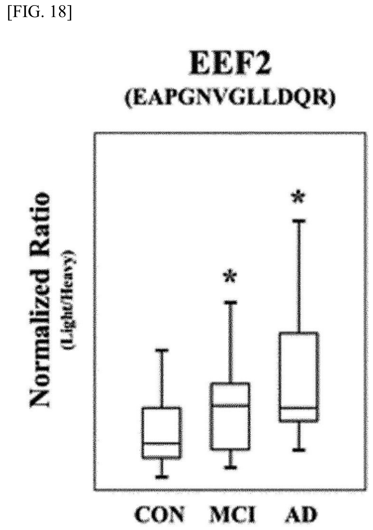

FIG. 18 shows the results of analyzing changes in the expression levels of EEF2 protein in aqueous humor from a normal control group (CON), patients with mild cognitive impairment (MCI) which is a pre-stage of Alzheimer's disease, and Alzheimer's disease (AD) patients, in Example 2 of the present invention.

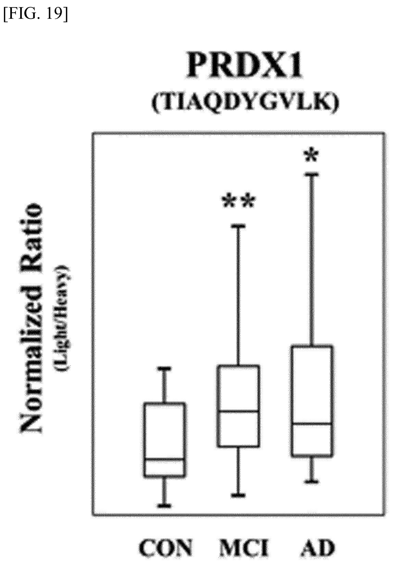

FIG. 19 shows the results of analyzing changes in the expression levels of PRDX1 protein in aqueous humor from a normal control group (CON), patients with mild cognitive impairment (MCI) which is a pre-stage of Alzheimer's disease, and Alzheimer's disease (AD) patients, in Example 2 of the present invention.

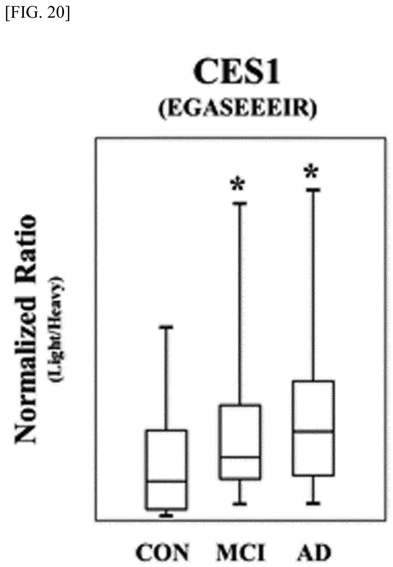

FIG. 20 shows the results of analyzing changes in the expression levels of CES1 protein in aqueous humor from a normal control group (CON), patients with mild cognitive impairment (MCI) which is a pre-stage of Alzheimer's disease, and Alzheimer's disease (AD) patients, in Example 2 of the present invention.

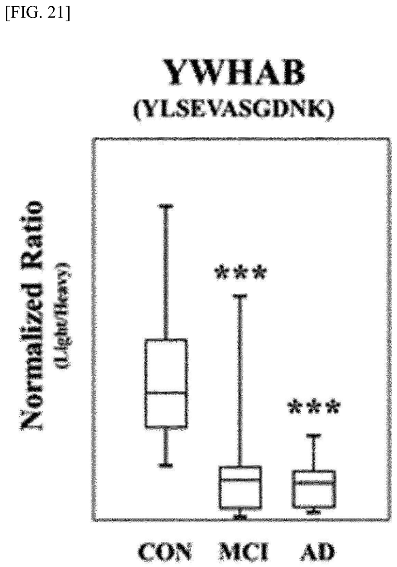

FIG. 21 shows the results of analyzing changes in the expression levels of YWHAB protein in aqueous humor from a normal control group (CON), patients with mild cognitive impairment (MCI) which is a pre-stage of Alzheimer's disease, and Alzheimer's disease (AD) patients, in Example 2 of the present invention.

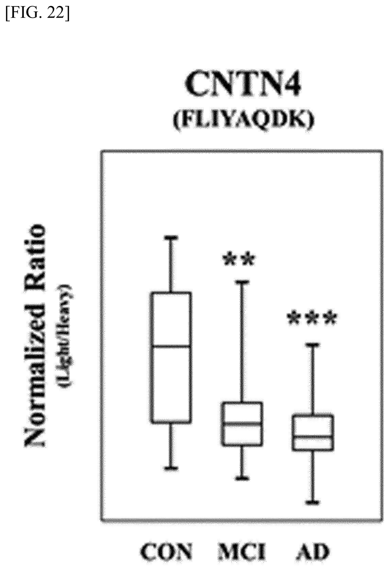

FIG. 22 shows the results of analyzing changes in the expression levels of CNTN4 protein in aqueous humor from a normal control group (CON), patients with mild cognitive impairment (MCI) which is a pre-stage of Alzheimer's disease, and Alzheimer's disease (AD) patients, in Example 2 of the present invention.

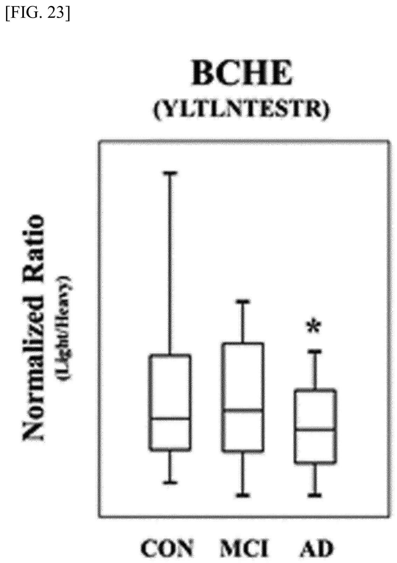

FIG. 23 shows the results of analyzing changes in the expression levels of BCHE protein in aqueous humor from a normal control group (CON), patients with mild cognitive impairment (MCI) which is a pre-stage of Alzheimer's disease, and Alzheimer's disease (AD) patients, in Example 2 of the present invention.
In FIGS. 16 to 23, * means P-value <0.05, ** means P-value <0.01, and *** means P-value <0.001.
FIG. 24 shows the results of evaluating the relative protein abundance of SPP1, CNTN4, YWHAB and ACHE in aqueous humor from Alzheimer's disease (AD) patients in Example 2 of the present invention.
FIG. 25 shows ROC curves for ACHE and SPP1 expressed in aqueous humor from Alzheimer's disease (AD) patients versus a normal control group (CON), for diagnosing Alzheimer's disease, in Example 2 of the present invention.
FIG. 26 shows ROC curves for ACHE and SPP1 expressed in aqueous humor from mild cognitive impairment (MCI) patients versus a normal control group (CON), for diagnosing Alzheimer's disease, in Example 2 of the present invention.
FIG 27 shows the areas under the curve for ROC curves for ACHE and SPP1 expressed either in aqueous humor from Alzheimer's disease (AD) patients versus a normal control group (CON), or in aqueous humor from mild cognitive impairment (MCI) patients versus a normal control group (CON), for diagnosing Alzheimer's disease, in Example 2 of the present invention.

### Best Mode

One embodiment of the present invention is directed to a biomarker for diagnosing a brain and nervous system disease comprising: at least one gene selected from the group shown in Table 1 above; and a protein encoded thereby.

Another embodiment of the present invention is directed to a biomarker for diagnosing Alzheimer's disease comprising: at least one gene selected from the group shown in Table 2 above; and a protein encoded thereby.

Still another embodiment of the present invention is directed to a biomarker for diagnosing Parkinson's disease comprising: at least one gene selected from the group shown in Table 5 above; and a protein encoded thereby.

Yet another embodiment of the present invention is directed to a biomarker for diagnosing cerebrovascular attack comprising: at least one gene selected from the group shown in Table 8 above; and a protein encoded thereby.

Still yet another embodiment of the present invention is directed to a biomarker for diagnosing brain tumor comprising: at least one gene selected from the group shown in Table 11 above; and a protein encoded thereby.

As the biomarker in the present invention, one present in the aqueous humor of the eye may increase the accuracy in diagnosing a brain and nervous system disease.

### Mode for Invention

Hereinafter, the present invention will be described in more detail with reference to examples. However, the following examples serve merely to illustrate the present disclosure, and the scope of the present invention is not limited by these examples.

### Examples

### [Example 1] Screening of Aqueous Humor Biomarkers for Diagnosing Brain and Nervous System Diseases

In order to screen aqueous humor biomarkers for diagnosing brain and nervous system diseases, the following experiment was performed according to the experimental design shown in FIG. 1.

### 1. Patient Recruitment

This study was reviewed and approved as a prospective study by the Institutional Review Board and ethics Committee of Gangnam Severance Hospital, and all patients involved in the study consented to the study after hearing sufficient explanations about the study. For global proteomic profiling in aqueous humor (AH) from patients with brain and nervous system diseases, experimental groups, each including 10 patients diagnosed with each of Alzheimer's disease (AD) and its prestage mild cognitive impairment (MCI), Parkinson's disease (PD), cerebrovascular attack (CVA) and brain tumor (BT, glioblastoma), were first recruited. A normal control group (Control, CON) included 10 people without underlying disease, who underwent general senile cataract surgery.

For the marker candidates derived in the profiling step, proteins common between the diseases or aqueous humor (AH) proteins specific to each disease were analyzed, and then the analysis of an aqueous humor sample from an individual patient was performed to screen AH protein markers capable of specifically diagnosing AD and MCI. To this end, 20 MCI patients, 47 AD patients and 52 control people were additionally recruited and included in the study.

The AD patients were selected from among patients diagnosed with AD as a result of amyloid positron emission tomography in the neurology department of the hospital, and patients of the PD, CVA and BT groups were selected from among patients diagnosed with the corresponding diseases through specialist's medical examination and brain imaging in the neurology department and neurosurgery department of the hospital. In addition, the MCI patients were selected from people having a mini-mental state examination (MMSE) of 18 to 23 without brain and nervous system diseases, among patients who saw specialists in the neurology department of the hospital due to memory loss. Among them, aqueous humor was collected from cases of cataract surgery in the ophthalmology department of the hospital.

All the subjects did not have specific past medical history including hypertension, diabetes and immune diseases, and also did not have past ocular history such as ocular trauma and uveitis. All the patients were recruited so that there was no discrimination between the left and right eyes and there was no difference in gender ratio, age, etc. between the groups.

Statistical analysis of clinical data was performed using SPSS v.21.0 (IBM Corp., Armonk, NY, USA). After the normality of the data was confirmed using the Kolmogorov-Smirnov test, the Mann-Whitney U test or Wilcoxon signed rank test was used for non-normally distributed data. P values of less than 0.05 in all statistical tests were considered statistically significant.

### 2. Aqueous Humor Collection

For cataract surgery, complete disinfection for surgery was performed, and then a side-port incision of 0.5 mm in size was made in the periphery of the cornea of the eye in the same manner as the general surgical procedure. At this time, the aqueous humor filling the eye flowed out of the eye. The aqueous humor was collected from the front of the eye at the primary side port incision using a 26-gauge syringe, and then the originally planned conventional eye surgery was performed. In this case, the amount of aqueous humor collected was about 0.05 to 0.15 cc, and the collected aqueous humor was placed in a 1.5-ml Eppendorf tube and stored in a cryogenic freezer at -80°C until analysis was performed.

### 3. Proteomic Analysis of Aqueous Humor

First, for profiling, the proteins contained in the aqueous humor samples of each group were degraded into peptides in the following manner. 8 M urea in 100 mM ammonium bicarbonate (Sigma, St. Louis, MO, USA) was mixed with each sample at a ratio of 1:3 (sample: urea) to a final concentration of 6 M or more, and incubated at room temperature for 20 minutes. Then, the proteins were denatured using 10 mM dithiothreitol (DTT, Sigma) for reduction and 30 mM iodoacetamide (IAA, Sigma) for alkylation. Trypsin was added to each sample (1:50 = trypsin: sample) and incubated at 37°C overnight. The activated trypsin reaction was quenched with 0.4% TFA, and the peptides were desalted with a C18 Harvard macro spin column. The resulting peptides were dried and stored at -80°C. The peptides were resuspended in 0.1% formic acid and analyzed using a Q Exactive TM orbitrap hybrid mass spectrometer coupled with Nanoacquity UPLC (Waters, Manchester, UK). For protein identification, the 'razor plus unique peptides' setting in MaxQuant was used. Proteins were quantified using the XIC-based label-free quantification (LFQ) algorithm in MaxQuant. As the data of each group, 'LFQ intensities' from MaxQuant were obtained, and all LFQ intensities were transformed to log₂ values. Proteins that did not display all values in three measurements were filtered out.

] Among the detected total proteins, proteins showing a 1.2-fold change (FC; increase or decrease) and a P value of less than 0.05 from t-test statistical analysis in LFQ intensity were classified to differentially expressed proteins (DEPs). In individual analysis of the verification step for screening of MCI and AD-specific markers, 2 µg of the peptide sample was subjected to the same pre-treatment procedure as in the profiling experiment, and then mass spectrometry was performed with Q-Exactive plus interfaced with an EASY-nLC 1000 UHPLC System. After quantification of the screened DEPs, target analysis was performed in Spectronaut Pulsar using the XXX spectral library. Perseus software (v.1.5.0.31) was used for statistical analysis.

### 4. Proteomic Bioinformatics Analysis

The gene ontology biological process (GOBP) terms associated with the identified proteins were analyzed using the Bioinformatics Database for Annotation, Visualization and Integrated Discovery. Functional annotation clustering and Kyoto Encyclopedia of Genes and Genomes (KEGG)) pathway mapping was also performed. Bioinformatics-based functional classification was performed with P <0.05. To construct a network model, interaction information was collected from the STRING 9.1 public database. The network model was built using Cytoscape software.

### 5. Experimental Results

The present inventors attempted to observe changes in the brain through proteomic changes of AH in the eye. There are not many studies on the analysis of AH at home and abroad, and there is nothing particularly about brain neurological diseases other than ophthalmic diseases. Therefore, the present inventors made it possible to show superior efficiency compared to existing studies from the proteomic analysis of AH. That is, AH may be collected from a patient in a very small amount (about 0.1 cc), and the removal of albumin and the like from AH was essential because the ratio of albumin and the like in AH is high. The present inventors established a flow-through enrichment method using freeze-drying, thus completing a pretreatment method capable of peptization without protein loss. The present inventors removed non-specific proteins through the established method and then performed verification through electrophoresis and mass spectrometry analysis.

As a result, the present inventors made it possible to improve the protein identification rate in AH by more than three times, and could identify proteins more than twice as much as in the previous study by detecting a total of 1911 proteins in AH.

As a result of performing principal component analysis (PCA), the AH proteins expressed in each brain and nervous system disease showed different expression patterns between the groups. Although AH is a fluid of the eye that is part of the cerebral nerve organ, but not an organ in direct contact with the brain, it was clearly grouped in PCA. It could be confirmed that MCI, a mild memory decline, was similar to that in the normal control group (CON), and then similar to that in the AD group, suggesting that the proteomic change in AH depending on the brain and nervous system disease was reliable (FIG. 2).

Hierarchical clustering analysis of DEPs among the expressed proteins was performed (FIG. 3). As a result, it could be confirmed again that the expression patterns of AH proteins were very different between the brain and nervous system diseases, like the results of PCA. The numbers of significantly up- or down-regulated proteins in the AD, PD, CVA and BT groups are shown in Tables 14 to 17 below, respectively.

**[Table 14] Number of significantly up- or down-regulated proteins in AD group**

| DEP (Differentially Expressed Proteins) | |
|---|---|
| UP | DOWN |
| 275 | 172 |

**[Table 15] Number of significantly up- or down-regulated proteins in PD group**

| DEP (Differentially Expressed Proteins) | |
|---|---|
| UP | DOWN |
| 240 | 148 |

**[Table 16] Number of significantly up- or down-regulated proteins in CVA group**

| DEP (Differentially Expressed Proteins) | |
|---|---|
| UP | DOWN |
| 228 | 198 |

**[Table 17] Number of significantly up- or down-regulated proteins in BT group**

| DEP (Differentially Expressed Proteins) | |
|---|---|
| UP | DOWN |
| 231 | 210 |

KEGG and GOBP analysis was performed in order to determine what kind of protein-to-protein interaction between DEPs and all DEPs expressed in AH in the presence of each brain and nervous system disease (FIG. 4). As a result, it was confirmed that all the brain diseases showed processes of cell adhesion and apoptosis signaling pathway, and in the AD group, AH proteins were involved in pathways related to nervous system development, synaptic signaling and cognition. In addition, the AH proteins of the PD group were involved in pathways such as granulocyte migration and neuropeptide signaling pathway, the AH proteins of the CVA groups were involved in pathways such as circulatory system, T cell proliferation and vasculature development, and the AH proteins of the BT group were involved in extracellular-signal-regulated kinase (ERK) cascade, glycosaminoglycan process, and the like. In view of the general pathophysiology of each brain and nervous system disease, it could be confirmed that the biological processes in which AH proteins were involved were well established. All of the AH DEPs expressed in each brain and nervous system disease were analyzed. The AH DEPs whose expressions were upregulated in each of AD, PD, CVA and BT compared to the normal control group are shown in Tables 18 to 21 below, and the AH DEPs whose expression levels were downregulated are shown in Tables 22 to 25 below.

**[Table 18] Types and expression fold changes of proteins whose expression levels were significantly upregulated in AD group**

| Accession number | Gene name | Fold change |
|---|---|---|
| P02511 | CRYAB | 13.47 |
| P04792 | HSPB1 | 6.89 |
| P31947 | SFN | 3.79 |
| P 19013 | KRT4 | 3.75 |
| P02745 | C1QA | 3.73 |
| P01824 | IGHV4-39 | 2.84 |
| P51858 | HDGF | 2.79 |
| O60883 | GPR37L1 | 2.60 |
| P05813 | CRYBA1 | 2.58 |
| P03950 | ANG | 2.54 |
| P15559 | NQO1 | 2.49 |
| Q08257 | CRYZ | 2.48 |
| Q86YZ3 | HRNR | 2.37 |
| P13645 | KRT10 | 2.36 |
| H7C2N1 | PTMA | 2.32 |
| P02538 | KRT6A | 2.30 |
| Q9BX67 | JAM3 | 2.28 |
| P23490 | LOR | 2.20 |
| P02489 | CRYAA | 2.20 |
| P53673 | CRYBA4 | 2.18 |
| Q7Z794 | KRT77 | 2.18 |
| P53674 | CRYBB1 | 2.16 |
| P12109 | COL6A1 | 2.05 |
| P29401 | TKT | 2.05 |
| P99999 | CYCS | 2.04 |
| O75095 | MEGF6 | 2.02 |
| P13647 | KRT5 | 1.99 |
| P02461 | COL3A1 | 1.95 |
| Q5T749 | KPRP | 1.95 |
| Q06830 | PRDX1 | 1.93 |
| P22735 | TGM1 | 1.92 |
| P23515 | OMG | 1.90 |
| Q86UN3 | RTN4RL2 | 1.89 |
| P20962 | PTMS | 1.88 |
| Q02747 | GUCA2A | 1.88 |
| P45877 | PPIC | 1.87 |
| P12645 | BMP3 | 1.85 |
| Q9BWQ8 | FAIM2 | 1.85 |
| P30838 | ALDH3A1 | 1.85 |
| Q5T750 | XP32 | 1.84 |
| P09493 | TPM1 | 1.77 |
| P02458 | COL2A1 | 1.77 |
| P06733 | ENO1 | 1.77 |
| P62258 | YWHAE | 1.76 |
| O75368 | SH3BGRL | 1.75 |
| Q08188 | TGM3 | 1.75 |
| Q5D862 | FLG2 | 1.73 |
| Q15847 | ADIRF | 1.73 |
| P19438 | TNFRSF1A | 1.72 |
| P48163 | ME1 | 1.71 |
| P05362 | ICAM1 | 1.71 |
| O75223 | GGCT | 1.70 |
| P02533 | KRT14 | 1.70 |
| P10124 | SRGN | 1.69 |
| P14618 | PKM | 1.68 |
| P63104 | YWHAZ | 1.68 |
| P47929 | LGALS7 | 1.67 |
| Q9ULI3 | HEG1 | 1.67 |
| P40394 | ADH7 | 1.65 |
| P02814 | SMR3B | 1.65 |
| P62979 | RPS27A | 1.64 |
| P04406 | GAPDH | 1.64 |
| O14594 | NCAN | 1.64 |
| Q8N1N4 | KRT78 | 1.64 |
| P04000 | OPN1LW | 1.63 |
| P35908 | KRT2 | 1.63 |
| P35527 | KRT9 | 1.62 |
| P43320 | CRYBB2 | 1.62 |
| A0A0G2JIW1 | HSPA1B | 1.62 |
| A0A0U1RRH7 | H2A | 1.61 |
| P09211 | GSTP1 | 1.61 |
| P12273 | PIP | 1.60 |
| Q96PQ0 | SORCS2 | 1.59 |
| Q14697 | GANAB | 1.59 |
| P52566 | ARHGDIB | 1.59 |
| P00352 | ALDH1A1 | 1.57 |
| Q6UXI7 | VIT | 1.57 |
| P20810 | CAST | 1.56 |
| P00558 | PGK1 | 1.56 |
| O75874 | IDH1 | 1.55 |
| P30041 | PRDX6 | 1.55 |
| Q04695 | KRT17 | 1.54 |
| Q9NZ53 | PODXL2 | 1.54 |
| P23435 | CBLN1 | 1.53 |
| P13639 | EEF2 | 1.53 |
| P62328 | TMSB4X | 1.52 |
| Q9Y5Z4 | HEBP2 | 1.51 |
| A6NFX8 | NUDT5 | 1.50 |
| P08779 | KRT16 | 1.50 |
| P05089 | ARG1 | 1.49 |
| P20930 | FLG | 1.49 |
| Q9BXJ4 | C1QTNF3 | 1.49 |
| Q9UBG0 | MRC2 | 1.49 |
| P42357 | HAL | 1.49 |
| P13500 | CCL2 | 1.48 |
| P28074 | PSMB5 | 1.48 |
| X6R8A1 | CTSA | 1.48 |
| P35754 | GLRX | 1.47 |
| P07195 | LDHB | 1.46 |
| O15118 | NPC1 | 1.46 |
| P02794 | FTH1 | 1.46 |
| Q01469 | FABP5 | 1.46 |
| Q13835 | PKP1 | 1.46 |
| Q8IVA1 | PCP2 | 1.45 |
| E9PK25 | CFL1 | 1.45 |
| A0A0A0MQU6 | SEMA6A | 1.45 |
| P08253 | MMP2 | 1.45 |
| Q9NSB2 | KRT84 | 1.45 |
| Q14393 | GAS6 | 1.44 |
| Q9UFP1 | FAM198A | 1.44 |
| P04264 | KRT1 | 1.44 |
| P55058 | PLTP | 1.44 |
| P24592 | IGFBP6 | 1.43 |
| Q8TCZ2 | CD99L2 | 1.43 |
| P13521 | SCG2 | 1.43 |
| P04075 | ALDOA | 1.43 |
| P0C6S8 | LINGO3 | 1.43 |
| O00754 | MAN2B1 | 1.43 |
| Q63HQ2 | EGFLAM | 1.42 |
| P21246 | PTN | 1.42 |
| P10523 | SAG | 1.41 |
| Q9Y6X5 | ENPP4 | 1.41 |
| P30086 | PEBP1 | 1.41 |
| P02788 | LTF | 1.40 |
| Q9GZZ8 | LACRT | 1.40 |
| Q92743 | HTRA1 | 1.40 |
| Q92752 | TNR | 1.40 |
| O43493 | TGOLN2 | 1.40 |
| O00292 | LEFTY2 | 1.40 |
| P29966 | MARCKS | 1.40 |
| P07900 | HSP90AA1 | 1.39 |
| P22303 | ACHE | 1.39 |
| P07737 | PFN1 | 1.39 |
| A0A087WWD4 | NCAM1 | 1.39 |
| P21333 | FLNA | 1.38 |
| Q32Q12 | NME1-NME2 | 1.38 |
| P02792 | FTL | 1.38 |
| Q9NZT1 | CALML5 | 1.38 |
| Q6UY11 | DLK2 | 1.38 |
| B4DPQ0 | C1R | 1.38 |
| P14174 | MIF | 1.37 |
| Q7Z5L0 | VMO1 | 1.37 |
| Q9H8J5 | MANSC1 | 1.37 |
| P02818 | BGLAP | 1.37 |
| Q15904 | ATP6AP1 | 1.37 |
| Q14050 | COL9A3 | 1.37 |
| P15924 | DSP | 1.37 |
| P32004 | L1CAM | 1.37 |
| P15328 | FOLR1 | 1.37 |
| Q92765 | FRZB | 1.36 |
| P48058 | GRIA4 | 1.36 |
| P10646 | TFPI | 1.35 |
| Q9NZH8 | IL36G | 1.35 |
| A0A0G2JLB3 | GBA | 1.35 |
| Q9NX62 | IMPAD1 | 1.35 |
| P04156 | PRNP | 1.35 |
| J3KRP0 | CNDP1 | 1.35 |
| Q6EMK4 | VASN | 1.34 |
| P50395 | GDI2 | 1.34 |
| A0A0A0MS64 | MEGF11 | 1.34 |
| P28838 | LAP3 | 1.33 |
| P22792 | CPN2 | 1.33 |
| O95897 | OLFM2 | 1.33 |
| P00441 | SOD1 | 1.33 |
| Q9Y617 | PSAT1 | 1.33 |
| P22223 | CDH3 | 1.33 |
| Q96KG7 | MEGF10 | 1.33 |
| O95967 | EFEMP2 | 1.33 |
| P35579 | MYH9 | 1.32 |
| Q9NRN5 | OLFML3 | 1.32 |
| P16152 | CBR1 | 1.32 |
| P31025 | LCN1 | 1.32 |
| Q04760 | GLO1 | 1.32 |
| P40926 | MDH2 | 1.32 |
| O75973 | C1QL1 | 1.31 |
| H3BSR6 | CX3CL1 | 1.31 |
| Q14314 | FGL2 | 1.31 |
| P00338 | LDHA | 1.31 |
| P07320 | CRYGD | 1.31 |
| Q99519 | NEU1 | 1.30 |
| P23471 | PTPRZ1 | 1.30 |
| P22897 | MRC1 | 1.30 |
| P15090 | FABP4 | 1.30 |
| P03973 | SLPI | 1.30 |
| Q53EL9 | SEZ6 | 1.30 |
| A0A1W2PRB8 | MED17 | 1.30 |
| Q16531 | DDB1 | 1.29 |
| P43251 | BTD | 1.29 |
| Q04721 | NOTCH2 | 1.29 |
| P22314 | UBA1 | 1.29 |
| Q99523 | SORT1 | 1.29 |
| P06702 | S100A9 | 1.29 |
| O15335 | CHAD | 1.29 |
| Q9Y6R7 | FCGBP | 1.29 |
| P01036 | CST4 | 1.29 |
| Q6P9A2 | GALNT18 | 1.28 |
| Q9BY79 | MFRP | 1.28 |
| A0A087WZM2 | RNASET2 | 1.28 |
| G3V5Z7 | PSMA6 | 1.28 |
| P14923 | JUP | 1.28 |
| O14818 | PSMA7 | 1.28 |
| Q8WZA1 | POMGNT1 | 1.28 |
| O95206 | PCDH8 | 1.28 |
| P04083 | ANXA1 | 1.28 |
| Q5JRA6 | MIA3 | 1.28 |
| P05109 | S100A8 | 1.28 |
| Q99574 | SERPINI1 | 1.28 |
| Q96HF1 | SFRP2 | 1.27 |
| Q9H3G5 | CPVL | 1.27 |
| P09871 | C1S | 1.27 |
| P58546 | MTPN | 1.27 |
| Q14019 | COTL1 | 1.27 |
| P31944 | CASP14 | 1.27 |
| Q99715 | COL12A1 | 1.27 |
| P08581 | MET | 1.26 |
| P23468 | PTPRD | 1.26 |
| P04278 | SHBG | 1.26 |
| P07451 | CA3 | 1.26 |
| A1L4H1 | SSC5D | 1.26 |
| Q9NT99 | LRRC4B | 1.26 |
| O00462 | MANBA | 1.26 |
| P10745 | RBP3 | 1.26 |
| P00533 | EGFR | 1.26 |
| P01040 | CSTA | 1.26 |
| P27797 | CALR | 1.26 |
| Q9P2S2 | NRXN2 | 1.25 |
| Q96S96 | PEBP4 | 1.25 |
| P68104 | EEF1A1 | 1.25 |
| P36955 | SERPINF1 | 1.25 |
| P02766 | TTR | 1.25 |
| Q9BRK5 | SDF4 | 1.25 |
| P56159 | GFRA1 | 1.25 |
| O43707 | ACTN4 | 1.25 |
| Q9Y5Y7 | LYVE1 | 1.24 |
| A0A1B0GV53 | CLEC19A | 1.24 |
| Q5T1H1 | EYS | 1.24 |
| O94919 | ENDOD1 | 1.24 |
| A0A0B4J1R4 | HPD | 1.24 |
| Q9ULX7 | CA14 | 1.24 |
| P60709 | ACTB | 1.24 |
| P07711 | CTSL | 1.24 |
| P11021 | HSPA5 | 1.24 |
| P18669 | PGAM1 | 1.23 |
| H7BY58 | PCMT1 | 1.23 |
| Q9NS15 | LTBP3 | 1.23 |
| P16109 | SELP | 1.23 |
| Q92729 | PTPRU | 1.23 |
| B5MCX6 | VSTM2A | 1.23 |
| Q9UHC6 | CNTNAP2 | 1.22 |
| P04066 | FUCA1 | 1.22 |
| G3V2W1 | SERPINA10 | 1.22 |
| Q03167 | TGFBR3 | 1.22 |
| Q92820 | GGH | 1.22 |
| Q96IY4 | CPB2 | 1.22 |
| P08236 | GUSB | 1.22 |
| P11117 | ACP2 | 1.21 |
| P04085 | PDGFA | 1.21 |
| O76076 | WISP2 | 1.21 |
| P78324 | SIRPA | 1.21 |
| O95841 | ANGPTL1 | 1.21 |
| P36222 | CHI3L1 | 1.21 |
| Q15818 | NPTX1 | 1.21 |
| P08123 | COL1A2 | 1.21 |
| Q99650 | OSMR | 1.21 |
| Q14112 | NID2 | 1.20 |
| A0A087WUM0 | SYNJ2BP-COX16 | 1.20 |
| Q92876 | KLK6 | 1.20 |
| P16278 | GLB1 | 1.20 |
| P18206 | VCL | 1.20 |
| A0A0A0MTS2 | GPI | 1.20 |
| Q15323 | KRT31 | 1.20 |
| P12955 | PEPD | 1.20 |
| Q9HC38 | GLOD4 | 1.20 |
| G8JLG2 | CDSN | 1.20 |
| P16070 | CD44 | 1.20 |

**[Table 19] Types and expression fold changes of proteins whose expression levels were significantly upregulated in PD group**

| Accession number | Gene name | Fold change |
|---|---|---|
| Q86UN3 | RTN4RL2 | 10.88 |
| P55058 | PLTP | 5.95 |
| P04792 | HSPB1 | 4.97 |
| Q02383 | SEMG2 | 4.79 |
| P02745 | C1QA | 3.67 |
| P04279 | SEMG1 | 3.16 |
| P45877 | PPIC | 3.13 |
| P03950 | ANG | 2.71 |
| Q02747 | GUCA2A | 2.52 |
| O95206 | PCDH8 | 2.50 |
| P11684 | SCGB1A1 | 2.42 |
| E7EUW2 | ADGRL3 | 2.38 |
| Q13508 | ART3 | 2.34 |
| Q9NSB2 | KRT84 | 2.27 |
| P52566 | ARHGDIB | 2.24 |
| P09493 | TPM1 | 2.21 |
| P02538 | KRT6A | 2.14 |
| P01824 | IGHV4-39 | 2.06 |
| P01833 | PIGR | 2.03 |
| Q9BXX0 | EMILIN2 | 2.03 |
| P10124 | SRGN | 2.02 |
| Q5D862 | FLG2 | 1.98 |
| O60883 | GPR37L1 | 1.98 |
| Q9NZH8 | IL36G | 1.97 |
| P19013 | KRT4 | 1.94 |
| P62979 | RPS27A | 1.92 |
| O75368 | SH3BGRL | 1.89 |
| P06702 | S100A9 | 1.88 |
| P51858 | HDGF | 1.84 |
| Q86YZ3 | HRNR | 1.83 |
| Q9NQ38 | SPINK5 | 1.71 |
| Q9BX67 | JAM3 | 1.70 |
| H3BSR6 | CX3CL1 | 1.68 |
| P35443 | THBS4 | 1.68 |
| P08236 | GUSB | 1.67 |
| P15090 | FABP4 | 1.65 |
| Q9H299 | SH3BGRL3 | 1.65 |
| P52799 | EFNB2 | 1.64 |
| Q9NZT1 | CALML5 | 1.64 |
| Q6E0U4 | DMKN | 1.63 |
| P13645 | KRT10 | 1.63 |
| A1L4H1 | SSC5D | 1.60 |
| P13647 | KRT5 | 1.60 |
| Q5VU97 | CACHD1 | 1.60 |
| P07492 | GRP | 1.59 |
| Q6UXB2 | CXCL17 | 1.57 |
| P02818 | BGLAP | 1.57 |
| P19438 | TNFRSF1A | 1.55 |
| P12273 | PIP | 1.55 |
| P23490 | LOR | 1.54 |
| P02461 | COL3A1 | 1.53 |
| P0C6S8 | LINGO3 | 1.53 |
| Q14315 | FLNC | 1.53 |
| Q9BWQ8 | FAIM2 | 1.53 |
| Q9Y6X5 | ENPP4 | 1.52 |
| P99999 | CYCS | 1.52 |
| P62328 | TMSB4X | 1.51 |
| Q14050 | COL9A3 | 1.50 |
| P01040 | CSTA | 1.50 |
| Q9NT99 | LRRC4B | 1.50 |
| P35527 | KRT9 | 1.50 |
| O75095 | MEGF6 | 1.50 |
| P30041 | PRDX6 | 1.49 |
| Q13835 | PKP1 | 1.49 |
| P05089 | ARG1 | 1.49 |
| P12645 | BMP3 | 1.49 |
| P08779 | KRT16 | 1.48 |
| H7C2N1 | PTMA | 1.48 |
| P35908 | KRT2 | 1.48 |
| P02766 | TTR | 1.48 |
| P07320 | CRYGD | 1.47 |
| Q6UXI7 | VIT | 1.47 |
| Q86YW7 | GPHB5 | 1.46 |
| P16109 | SELP | 1.46 |
| P42357 | HAL | 1.46 |
| P02760 | AMBP | 1.46 |
| P47929 | LGALS7 | 1.45 |
| P02533 | KRT14 | 1.44 |
| Q92820 | GGH | 1.43 |
| P04156 | PRNP | 1.43 |
| Q96HF1 | SFRP2 | 1.42 |
| P02775 | PPBP | 1.42 |
| Q5T750 | XP32 | 1.42 |
| P22735 | TGM1 | 1.42 |
| Q09666 | AHNAK | 1.42 |
| P19957 | PI3 | 1.42 |
| P04278 | SHBG | 1.41 |
| Q7Z794 | KRT77 | 1.41 |
| P20930 | FLG | 1.41 |
| P28074 | PSMB5 | 1.40 |
| P02792 | FTL | 1.40 |
| Q9H8J5 | MANSC1 | 1.39 |
| P05362 | ICAM1 | 1.39 |
| B4DPQ0 | C1R | 1.39 |
| X6R8A1 | CTSA | 1.39 |
| P00995 | SPINK1 | 1.39 |
| Q15828 | CST6 | 1.39 |
| G3V2W1 | SERPINA10 | 1.39 |
| Q08188 | TGM3 | 1.39 |
| Q9HC56 | PCDH9 | 1.38 |
| Q9BY79 | MFRP | 1.38 |
| A0A0B4J1R4 | HPD | 1.38 |
| Q6UWP8 | SBSN | 1.38 |
| Q8N1N4 | KRT78 | 1.38 |
| Q496H8 | NRN1L | 1.38 |
| P04000 | OPN1LW | 1.37 |
| P12109 | COL6A1 | 1.37 |
| A0A087WUM0 | SYNJ2BP-COX16 | 1.37 |
| P14618 | PKM | 1.37 |
| P04406 | GAPDH | 1.37 |
| O43493 | TGOLN2 | 1.37 |
| P05813 | CRYBA1 | 1.37 |
| P54826 | GAS1 | 1.36 |
| Q9Y5Y7 | LYVE1 | 1.36 |
| O00754 | MAN2B1 | 1.36 |
| P07148 | FABP1 | 1.35 |
| P48163 | ME1 | 1.35 |
| K7ES00 | H3F3B | 1.35 |
| P29508 | SERPINB3 | 1.35 |
| O75223 | GGCT | 1.35 |
| P10646 | TFPI | 1.34 |
| P02452 | COL1A1 | 1.34 |
| Q15113 | PCOLCE | 1.34 |
| P21333 | FLNA | 1.34 |
| P13639 | EEF2 | 1.34 |
| P28300 | LOX | 1.34 |
| P04080 | CSTB | 1.34 |
| Q6P9A2 | GALNT18 | 1.33 |
| P07195 | LDHB | 1.33 |
| P09211 | GSTP1 | 1.33 |
| Q04695 | KRT17 | 1.33 |
| P31947 | SFN | 1.33 |
| A0A0A0MS64 | MEGF11 | 1.33 |
| Q495W5 | FUT11 | 1.33 |
| P14138 | EDN3 | 1.32 |
| G3XAI2 | LAMB1 | 1.32 |
| P29966 | MARCKS | 1.32 |
| K7ERG9 | CFD | 1.32 |
| Q9NZ53 | PODXL2 | 1.32 |
| Q9Y617 | PSAT1 | 1.32 |
| P35754 | GLRX | 1.32 |
| P24592 | IGFBP6 | 1.31 |
| Q14112 | NID2 | 1.31 |
| P35579 | MYH9 | 1.31 |
| P15924 | DSP | 1.31 |
| A0A087WVC6 | PTPRJ | 1.30 |
| Q6EMK4 | VASN | 1.30 |
| Q9NX62 | IMPAD1 | 1.30 |
| O00462 | MANBA | 1.30 |
| Q99941 | ATF6B | 1.30 |
| A0A0U1RRH7 | H2A | 1.30 |
| Q9NPZ5 | B3GAT2 | 1.30 |
| P04155 | TFF1 | 1.30 |
| P00558 | PGK1 | 1.30 |
| P55001 | MFAP2 | 1.29 |
| Q7Z5L0 | VMO1 | 1.29 |
| Q16531 | DDB1 | 1.29 |
| P31944 | CASP14 | 1.29 |
| Q5T1H1 | EYS | 1.29 |
| Q5T749 | KPRP | 1.29 |
| Q08257 | CRYZ | 1.28 |
| X6R8F3 | LCN2 | 1.28 |
| Q969H8 | MYDGF | 1.28 |
| E9PK25 | CFL1 | 1.28 |
| P02743 | APCS | 1.28 |
| P55000 | SLURP1 | 1.28 |
| P22223 | CDH3 | 1.28 |
| Q9NRN5 | OLFML3 | 1.28 |
| O43707 | ACTN4 | 1.28 |
| P16152 | CBR1 | 1.28 |
| O95967 | EFEMP2 | 1.28 |
| P09104 | ENO2 | 1.27 |
| G3V5Z7 | PSMA6 | 1.27 |
| Q14982 | OPCML | 1.27 |
| O15041 | SEMA3E | 1.27 |
| Q9ULX7 | CA14 | 1.26 |
| Q5JRA6 | MIA3 | 1.26 |
| 015335 | CHAD | 1.26 |
| Q9UBG0 | MRC2 | 1.26 |
| Q99523 | SORT1 | 1.26 |
| P13521 | SCG2 | 1.26 |
| Q9HBL6 | LRTM1 | 1.26 |
| P20810 | CAST | 1.26 |
| P22897 | MRC1 | 1.26 |
| A0A0A0MQU6 | SEMA6A | 1.25 |
| Q96P63 | SERPINB12 | 1.25 |
| G8JLG2 | CDSN | 1.25 |
| P14923 | JUP | 1.25 |
| P04259 | KRT6B | 1.24 |
| Q96PQ0 | SORCS2 | 1.24 |
| Q96IY4 | CPB2 | 1.24 |
| P08572 | COL4A2 | 1.24 |
| Q99650 | OSMR | 1.24 |
| Q14393 | GAS6 | 1.24 |
| P04745 | AMY1A | 1.24 |
| P31151 | S100A7 | 1.23 |
| Q8IVA1 | PCP2 | 1.23 |
| Q99674 | CGREF1 | 1.23 |
| Q8TCZ2 | CD99L2 | 1.23 |
| A6NFX8 | NUDT5 | 1.23 |
| P34059 | GALNS | 1.23 |
| P00450 | CP | 1.23 |
| Q08380 | LGALS3BP | 1.23 |
| Q32Q12 | NME1-NME2 | 1.23 |
| P12277 | CKB | 1.23 |
| P08253 | MMP2 | 1.23 |
| P05109 | S100A8 | 1.23 |
| Q9NZK5 | ADA2 | 1.23 |
| P04264 | KRT1 | 1.22 |
| Q9H4F8 | SMOC1 | 1.22 |
| Q9BRK5 | SDF4 | 1.22 |
| P62258 | YWHAE | 1.22 |
| Q9H3G5 | CPVL | 1.22 |
| O00292 | LEFTY2 | 1.22 |
| Q92743 | HTRA1 | 1.22 |
| Q99715 | COL12A1 | 1.22 |
| B7ZL91 | MEP1A | 1.22 |
| P10153 | RNASE2 | 1.22 |
| Q12797 | ASPH | 1.22 |
| Q14956 | GPNMB | 1.21 |
| A0A0G2JLB3 | GBA | 1.21 |
| Q9HC57 | WFDC1 | 1.21 |
| Q6FHJ7 | SFRP4 | 1.21 |
| P08581 | MET | 1.21 |
| Q15847 | ADIRF | 1.21 |
| Q9HCQ7 | NPVF | 1.21 |
| P32004 | L1CAM | 1.21 |
| P36955 | SERPINF1 | 1.21 |
| P07900 | HSP90AA1 | 1.21 |
| P04075 | ALDOA | 1.21 |
| Q15323 | KRT31 | 1.20 |
| Q14055 | COL9A2 | 1.20 |
| P08833 | IGFBP1 | 1.20 |
| P14174 | MIF | 1.20 |
| P04066 | FUCA1 | 1.20 |
| Q03167 | TGFBR3 | 1.20 |
| P07451 | CA3 | 1.20 |
| Q08554 | DSC1 | 1.20 |
| Q92729 | PTPRU | 1.20 |
| Q14574 | DSC3 | 1.20 |

**[Table 20] Types and expression fold changes of proteins whose expression levels were significantly upregulated in CVA group**

| Accession number | Gene name | Fold change |
|---|---|---|
| P45877 | PPIC | 56.49 |
| P02511 | CRYAB | 12.10 |
| E7EUW2 | ADGRL3 | 8.32 |
| P55058 | PLTP | 5.00 |
| P31947 | SFN | 3.65 |
| P51858 | HDGF | 3.34 |
| P06733 | ENO1 | 3.34 |
| P 19013 | KRT4 | 2.74 |
| P02792 | FTL | 2.70 |
| P04066 | FUCA1 | 2.66 |
| P01824 | IGHV4-39 | 2.64 |
| P04792 | HSPB1 | 2.46 |
| Q08257 | CRYZ | 2.37 |
| Q9NT99 | LRRC4B | 2.35 |
| P02489 | CRYAA | 2.29 |
| P52566 | ARHGDIB | 2.25 |
| P00338 | LDHA | 2.08 |
| P02538 | KRT6A | 2.05 |
| O60883 | GPR37L1 | 2.04 |
| P07195 | LDHB | 1.94 |
| P15559 | NQO1 | 1.93 |
| H7C2N1 | PTMA | 1.92 |
| P00558 | PGK1 | 1.89 |
| P00352 | ALDH1A1 | 1.86 |
| P0C6S8 | LINGO3 | 1.84 |
| P20962 | PTMS | 1.83 |
| P02814 | SMR3B | 1.83 |
| P29401 | TKT | 1.83 |
| P22314 | UBA1 | 1.82 |
| P02745 | C1QA | 1.82 |
| P30041 | PRDX6 | 1.81 |
| Q7Z794 | KRT77 | 1.81 |
| P99999 | CYCS | 1.80 |
| P62937 | PPIA | 1.80 |
| Q9GZZ8 | LACRT | 1.80 |
| P62979 | RPS27A | 1.80 |
| P09211 | GSTP1 | 1.80 |
| O43653 | PSCA | 1.78 |
| Q86YZ3 | HRNR | 1.77 |
| P28300 | LOX | 1.76 |
| P13645 | KRT10 | 1.75 |
| P35527 | KRT9 | 1.74 |
| P03950 | ANG | 1.74 |
| P40926 | MDH2 | 1.73 |
| P30838 | ALDH3A1 | 1.72 |
| Q15847 | ADIRF | 1.69 |
| P78417 | GSTO1 | 1.68 |
| P05813 | CRYBA1 | 1.67 |
| Q96LR4 | FAM19A4 | 1.67 |
| Q9BX67 | JAM3 | 1.66 |
| P12645 | BMP3 | 1.65 |
| E5RIM7 | ATOX1 | 1.63 |
| P13647 | KRT5 | 1.62 |
| P22897 | MRC1 | 1.62 |
| P14138 | EDN3 | 1.60 |
| O75368 | SH3BGRL | 1.60 |
| P13646 | KRT13 | 1.60 |
| Q6UXI7 | VIT | 1.59 |
| O75874 | IDH1 | 1.58 |
| P20810 | CAST | 1.58 |
| P16152 | CBR1 | 1.58 |
| P12109 | COL6A1 | 1.57 |
| P02461 | COL3A1 | 1.56 |
| P40394 | ADH7 | 1.55 |
| P22735 | TGM1 | 1.55 |
| P08236 | GUSB | 1.54 |
| B4DPQ0 | C1R | 1.54 |
| P31025 | LCN1 | 1.53 |
| P63104 | YWHAZ | 1.53 |
| Q5D862 | FLG2 | 1.53 |
| Q6UXB2 | CXCL17 | 1.52 |
| P07451 | CA3 | 1.52 |
| Q9NZH8 | IL36G | 1.52 |
| P48163 | ME1 | 1.51 |
| P47929 | LGALS7 | 1.51 |
| O75095 | MEGF6 | 1.51 |
| P61604 | HSPE1 | 1.51 |
| P02533 | KRT14 | 1.49 |
| P04080 | CSTB | 1.49 |
| O00292 | LEFTY2 | 1.49 |
| O43827 | ANGPTL7 | 1.48 |
| P04406 | GAPDH | 1.48 |
| P35754 | GLRX | 1.48 |
| A6NFX8 | NUDT5 | 1.47 |
| P29508 | SERPINB3 | 1.47 |
| P10124 | SRGN | 1.46 |
| Q969H8 | MYDGF | 1.46 |
| Q9NX62 | IMPAD1 | 1.46 |
| Q03167 | TGFBR3 | 1.46 |
| P23490 | LOR | 1.45 |
| P19438 | TNFRSF1A | 1.44 |
| P28074 | PSMB5 | 1.44 |
| P30086 | PEBP1 | 1.44 |
| Q9NPZ5 | B3GAT2 | 1.44 |
| P02766 | TTR | 1.44 |
| P24592 | IGFBP6 | 1.44 |
| Q9BWQ8 | FAIM2 | 1.42 |
| A0A0G2JIW1 | HSPA1B | 1.42 |
| A0A1W2PRB8 | MED17 | 1.42 |
| Q04760 | GLO1 | 1.41 |
| Q9BXX0 | EMILIN2 | 1.41 |
| P43320 | CRYBB2 | 1.41 |
| G3XAI2 | LAMB1 | 1.41 |
| P20930 | FLG | 1.41 |
| P13639 | EEF2 | 1.41 |
| Q9NZ53 | PODXL2 | 1.41 |
| P12277 | CKB | 1.41 |
| P13521 | SCG2 | 1.40 |
| P35443 | THBS4 | 1.40 |
| Q9UBG0 | MRC2 | 1.40 |
| P05362 | ICAM1 | 1.40 |
| P35908 | KRT2 | 1.40 |
| P07900 | HSP90AA1 | 1.39 |
| P14618 | PKM | 1.39 |
| Q5T750 | XP32 | 1.39 |
| P53673 | CRYBA4 | 1.39 |
| Q08188 | TGM3 | 1.39 |
| P15924 | DSP | 1.38 |
| Q8N1N4 | KRT78 | 1.38 |
| P08670 | VIM | 1.38 |
| P07585 | DCN | 1.38 |
| P10451 | SPP1 | 1.38 |
| P42357 | HAL | 1.38 |
| Q96IY4 | CPB2 | 1.38 |
| P02452 | COL1A1 | 1.37 |
| Q99674 | CGREF1 | 1.37 |
| Q14697 | GANAB | 1.37 |
| A0A0A0MRJ7 | F5 | 1.37 |
| P04259 | KRT6B | 1.36 |
| P04179 | SOD2 | 1.36 |
| A0A0A0MTS2 | GPI | 1.36 |
| P23435 | CBLN1 | 1.36 |
| Q9NZT1 | CALML5 | 1.35 |
| Q14574 | DSC3 | 1.35 |
| Q9NRN5 | OLFML3 | 1.35 |
| Q16531 | DDB1 | 1.35 |
| O43707 | ACTN4 | 1.34 |
| P09871 | C1S | 1.34 |
| P31151 | S100A7 | 1.34 |
| P08779 | KRT16 | 1.34 |
| Q15582 | TGFBI | 1.33 |
| P16109 | SELP | 1.33 |
| P01040 | CSTA | 1.33 |
| P12273 | PIP | 1.33 |
| P22792 | CPN2 | 1.32 |
| P14174 | MIF | 1.32 |
| Q9ULI3 | HEGI | 1.32 |
| P09493 | TPM1 | 1.32 |
| P52799 | EFNB2 | 1.32 |
| B1B0D4 | ADAMTSL2 | 1.32 |
| P08493 | MGP | 1.32 |
| Q9Y279 | VSIG4 | 1.32 |
| P05089 | ARG1 | 1.31 |
| Q53RD9 | FBLN7 | 1.31 |
| A1L4H1 | SSC5D | 1.31 |
| P04264 | KRT1 | 1.31 |
| P15121 | AKR1B1 | 1.31 |
| P12955 | PEPD | 1.31 |
| Q9HC38 | GLOD4 | 1.30 |
| Q9Y617 | PSAT1 | 1.30 |
| A6NC48 | BST1 | 1.30 |
| Q86YW7 | GPHB5 | 1.30 |
| P62258 | YWHAE | 1.30 |
| Q5T1H1 | EYS | 1.30 |
| X6R8A1 | CTSA | 1.29 |
| P15090 | FABP4 | 1.29 |
| Q9Y5Z4 | HEBP2 | 1.29 |
| Q14112 | NID2 | 1.29 |
| Q5T749 | KPRP | 1.28 |
| Q04695 | KRT17 | 1.28 |
| O60242 | ADGRB3 | 1.28 |
| Q96KG7 | MEGF10 | 1.28 |
| Q92765 | FRZB | 1.28 |
| Q9BXJ4 | C1QTNF3 | 1.28 |
| P40925 | MDH1 | 1.28 |
| E9PDN6 | CNTNAP4 | 1.28 |
| Q9NRR1 | CYTL1 | 1.27 |
| P04156 | PRNP | 1.27 |
| P00450 | CP | 1.27 |
| Q32Q12 | NME1-NME2 | 1.26 |
| Q96KP4 | CNDP2 | 1.26 |
| P07737 | PFN1 | 1.26 |
| P04278 | SHBG | 1.26 |
| Q02747 | GUCA2A | 1.25 |
| G3V2W1 | SERPINA10 | 1.25 |
| P09104 | ENO2 | 1.25 |
| Q13835 | PKP1 | 1.24 |
| Q8IVA1 | PCP2 | 1.24 |
| P19320 | VCAM1 | 1.24 |
| Q9H299 | SH3BGRL3 | 1.24 |
| O95965 | ITGBL1 | 1.24 |
| O00754 | MAN2B1 | 1.23 |
| P07492 | GRP | 1.23 |
| P04085 | PDGFA | 1.23 |
| P10153 | RNASE2 | 1.23 |
| P15144 | ANPEP | 1.23 |
| P35579 | MYH9 | 1.23 |
| Q496H8 | NRN1L | 1.23 |
| Q14956 | GPNMB | 1.23 |
| P08833 | IGFBP1 | 1.23 |
| Q6EMK4 | VASN | 1.23 |
| P53674 | CRYBB1 | 1.22 |
| Q8TCZ2 | CD99L2 | 1.22 |
| O43493 | TGOLN2 | 1.22 |
| A0A0B4J1R4 | HPD | 1.22 |
| E9PK25 | CFL1 | 1.22 |
| Q14315 | FLNC | 1.22 |
| Q9NZ08 | ERAP1 | 1.22 |
| P55287 | CDH11 | 1.22 |
| Q99715 | COL12A1 | 1.21 |
| P05546 | SERPIND1 | 1.21 |
| Q14055 | COL9A2 | 1.21 |
| P18065 | IGFBP2 | 1.21 |
| P60174 | TPI1 | 1.21 |
| O43278 | SPINT1 | 1.21 |
| Q96HF 1 | SFRP2 | 1.21 |
| 015118 | NPC1 | 1.21 |
| Q15113 | PCOLCE | 1.21 |
| Q14520 | HABP2 | 1.20 |
| P02788 | LTF | 1.20 |
| P10523 | SAG | 1.20 |
| Q08380 | LGALS3BP | 1.20 |
| P01036 | CST4 | 1.20 |
| P14384 | CPM | 1.20 |
| Q6P9A2 | GALNT18 | 1.20 |
| P00742 | F10 | 1.20 |
| O95497 | VNN1 | 1.20 |
| A0A0A0MQU6 | SEMA6A | 1.20 |

**[Table 21] Types and expression fold changes of proteins whose expression levels were significantly upregulated in BT group**

| | | |
|---|---|---|
| Accession number | Gene name | Fold change |
| A0A0G2JIW1 | HSPA1B | 4.72 |
| P01824 | IGHV4-39 | 4.72 |
| P 19013 | KRT4 | 4.56 |
| P01833 | PIGR | 4.29 |
| Q13508 | ART3 | 3.25 |
| P78417 | GSTO1 | 3.23 |
| G3V2W1 | SERPINA10 | 3.17 |
| Q5T750 | XP32 | 3.10 |
| Q02747 | GUCA2A | 3.03 |
| P11684 | SCGB1A1 | 2.86 |
| P45877 | PPIC | 2.77 |
| P23490 | LOR | 2.62 |
| P04066 | FUCA1 | 2.59 |
| Q5T749 | KPRP | 2.57 |
| P35908 | KRT2 | 2.54 |
| Q9BXX0 | EMILIN2 | 2.50 |
| P13645 | KRT10 | 2.39 |
| P12109 | COL6A1 | 2.38 |
| A0A0B4J1R4 | HPD | 2.36 |
| P02760 | AMBP | 2.32 |
| Q9P121 | NTM | 2.30 |
| Q9H299 | SH3BGRL3 | 2.18 |
| P55000 | SLURP1 | 2.10 |
| P31947 | SFN | 2.09 |
| P10124 | SRGN | 2.06 |
| P19957 | PI3 | 2.03 |
| Q5D862 | FLG2 | 1.97 |
| P15559 | NQO1 | 1.96 |
| P16070 | CD44 | 1.96 |
| Q9NQ38 | SPINK5 | 1.94 |
| P07585 | DCN | 1.94 |
| O60883 | GPR37L1 | 1.94 |
| H7C2N1 | PTMA | 1.93 |
| O75368 | SH3BGRL | 1.90 |
| Q08257 | CRYZ | 1.90 |
| P00995 | SPINK1 | 1.89 |
| E5RIM7 | ATOX1 | 1.89 |
| P62979 | RPS27A | 1.89 |
| P62328 | TMSB4X | 1.88 |
| P51858 | HDGF | 1.85 |
| P02775 | PPBP | 1.83 |
| P02745 | C1QA | 1.82 |
| Q9BX67 | JAM3 | 1.81 |
| O75095 | MEGF6 | 1.80 |
| Q12841 | FSTL1 | 1.79 |
| O43653 | PSCA | 1.79 |
| P02511 | CRYAB | 1.78 |
| J3KRP0 | CNDP1 | 1.78 |
| P09493 | TPM1 | 1.76 |
| Q9Y5Y7 | LYVE1 | 1.75 |
| P04792 | HSPB1 | 1.74 |
| Q6UY11 | DLK2 | 1.72 |
| Q8N1N4 | KRT78 | 1.72 |
| P52566 | ARHGDIB | 1.71 |
| P02671 | FGA | 1.71 |
| P07333 | CSF1R | 1.70 |
| Q6E0U4 | DMKN | 1.70 |
| P13647 | KRT5 | 1.69 |
| P15090 | FABP4 | 1.69 |
| P36222 | CHI3L1 | 1.68 |
| Q14315 | FLNC | 1.66 |
| B7ZL91 | MEP1A | 1.66 |
| Q9NT99 | LRRC4B | 1.64 |
| P02461 | COL3A1 | 1.64 |
| P06727 | APOA4 | 1.63 |
| Q08188 | TGM3 | 1.63 |
| Q7Z794 | KRT77 | 1.62 |
| P02538 | KRT6A | 1.62 |
| 015335 | CHAD | 1.61 |
| P16109 | SELP | 1.61 |
| P29401 | TKT | 1.60 |
| P22735 | TGM1 | 1.60 |
| Q9ULI3 | HEG1 | 1.60 |
| P30838 | ALDH3A1 | 1.59 |
| 015041 | SEMA3E | 1.59 |
| Q09666 | AHNAK | 1.58 |
| P40394 | ADH7 | 1.58 |
| P05362 | ICAM1 | 1.58 |
| P02741 | CRP | 1.58 |
| P22792 | CPN2 | 1.56 |
| P14138 | EDN3 | 1.56 |
| P02452 | COL1A1 | 1.56 |
| P04155 | TFF1 | 1.56 |
| P19438 | TNFRSF1A | 1.56 |
| P22897 | MRC1 | 1.56 |
| O95206 | PCDH8 | 1.55 |
| P24592 | IGFBP6 | 1.54 |
| P99999 | CYCS | 1.54 |
| P07148 | FABP1 | 1.53 |
| P05089 | ARG1 | 1.53 |
| P0C6S8 | LINGO3 | 1.53 |
| Q9NZ53 | PODXL2 | 1.52 |
| P02533 | KRT14 | 1.50 |
| Q02383 | SEMG2 | 1.49 |
| P52799 | EFNB2 | 1.49 |
| G3XAI2 | LAMB1 | 1.47 |
| P19320 | VCAM1 | 1.47 |
| Q96HF 1 | SFRP2 | 1.47 |
| Q14050 | COL9A3 | 1.47 |
| Q15828 | CST6 | 1.46 |
| P08493 | MGP | 1.46 |
| B4DPQ0 | C1R | 1.46 |
| A0A087WUM0 | SYNJ2BP-COX16 | 1.45 |
| E9PK25 | CFL1 | 1.45 |
| P05813 | CRYBA1 | 1.45 |
| Q9GZX9 | TWSG1 | 1.45 |
| Q9P0K1 | ADAM22 | 1.44 |
| P20930 | FLG | 1.44 |
| P07195 | LDHB | 1.43 |
| O00468 | AGRN | 1.43 |
| P28074 | PSMB5 | 1.42 |
| O76076 | WISP2 | 1.41 |
| O43493 | TGOLN2 | 1.41 |
| P08572 | COL4A2 | 1.41 |
| Q15847 | ADIRF | 1.40 |
| P02792 | FTL | 1.39 |
| Q6UWP8 | SBSN | 1.39 |
| P07737 | PFN1 | 1.39 |
| O43707 | ACTN4 | 1.39 |
| Q04721 | NOTCH2 | 1.38 |
| P04080 | CSTB | 1.37 |
| P04264 | KRT1 | 1.37 |
| P11047 | LAMC1 | 1.37 |
| P20810 | CAST | 1.37 |
| Q9UBX7 | KLK11 | 1.37 |
| P28838 | LAP3 | 1.36 |
| Q86YZ3 | HRNR | 1.36 |
| Q9BWQ8 | FAIM2 | 1.36 |
| P04000 | OPN1LW | 1.36 |
| Q9UBG0 | MRC2 | 1.35 |
| P98160 | HSPG2 | 1.35 |
| P48163 | ME1 | 1.34 |
| Q9GZP0 | PDGFD | 1.34 |
| P10646 | TFPI | 1.34 |
| P20774 | OGN | 1.34 |
| P03950 | ANG | 1.34 |
| Q9GZZ8 | LACRT | 1.33 |
| Q9NX62 | IMPAD1 | 1.33 |
| P21246 | PTN | 1.33 |
| P28300 | LOX | 1.33 |
| Q496H8 | NRN1L | 1.33 |
| P42357 | HAL | 1.33 |
| P06733 | ENO1 | 1.33 |
| Q969H8 | MYDGF | 1.32 |
| Q9H3G5 | CPVL | 1.32 |
| P16930 | FAH | 1.32 |
| Q92563 | SPOCK2 | 1.31 |
| Q14055 | COL9A2 | 1.31 |
| Q16270 | IGFBP7 | 1.31 |
| O75874 | IDH1 | 1.31 |
| G8JLG2 | CDSN | 1.31 |
| P12955 | PEPD | 1.31 |
| P35579 | MYH9 | 1.31 |
| P12645 | BMP3 | 1.31 |
| K7ERG9 | CFD | 1.31 |
| 014672 | ADAM10 | 1.31 |
| Q969E1 | LEAP2 | 1.30 |
| P05109 | S100A8 | 1.30 |
| Q08380 | LGALS3BP | 1.30 |
| Q5VZE7 | SPINK4 | 1.30 |
| P04085 | PDGFA | 1.30 |
| Q9Y279 | VSIG4 | 1.29 |
| O00754 | MAN2B1 | 1.29 |
| P55001 | MFAP2 | 1.28 |
| Q13835 | PKP1 | 1.28 |
| Q03167 | TGFBR3 | 1.28 |
| Q6UXB2 | CXCL17 | 1.28 |
| P01040 | CSTA | 1.28 |
| P00352 | ALDH1A1 | 1.28 |
| Q8NBJ4 | GOLM1 | 1.28 |
| Q6EMK4 | VASN | 1.28 |
| P08779 | KRT16 | 1.28 |
| Q02985 | CFHR3 | 1.28 |
| P0DJI8 | SAA1 | 1.27 |
| Q15582 | TGFBI | 1.27 |
| P08833 | IGFBP1 | 1.27 |
| P30043 | BLVRB | 1.27 |
| P13639 | EEF2 | 1.27 |
| O95274 | LYPD3 | 1.27 |
| P00558 | PGK1 | 1.27 |
| P09871 | C1S | 1.26 |
| G3V5Z7 | PSMA6 | 1.26 |
| Q08554 | DSC1 | 1.26 |
| P49788 | RARRES1 | 1.25 |
| P02750 | LRG1 | 1.25 |
| B1B0D4 | ADAMTSL2 | 1.25 |
| Q96P63 | SERPINB 12 | 1.25 |
| P23142 | FBLN1 | 1.25 |
| P04179 | SOD2 | 1.25 |
| X6R8F3 | LCN2 | 1.25 |
| Q8IVA1 | PCP2 | 1.24 |
| P02766 | TTR | 1.24 |
| P48745 | NOV | 1.24 |
| Q14314 | FGL2 | 1.23 |
| Q9NRR1 | CYTL1 | 1.23 |
| P21333 | FLNA | 1.23 |
| Q9NPY3 | CD93 | 1.23 |
| 015240 | VGF | 1.23 |
| Q08174 | PCDH1 | 1.23 |
| Q96S96 | PEBP4 | 1.23 |
| Q9BRK5 | SDF4 | 1.22 |
| P04279 | SEMG1 | 1.22 |
| Q14112 | NID2 | 1.22 |
| P07225 | PROS1 | 1.22 |
| Q 14847 | LASP1 | 1.22 |
| Q99983 | OMD | 1.22 |
| P55289 | CDH12 | 1.22 |
| P27918 | CFP | 1.22 |
| A0A087WVC6 | PTPRJ | 1.21 |
| P12273 | PIP | 1.21 |
| P31431 | SDC4 | 1.21 |
| P10153 | RNASE2 | 1.21 |
| P63104 | YWHAZ | 1.21 |
| Q6UXI7 | VIT | 1.21 |
| Q9Y6R7 | FCGBP | 1.21 |
| P35754 | GLRX | 1.21 |
| Q16531 | DDB1 | 1.21 |
| P24043 | LAMA2 | 1.20 |
| P12111 | COL6A3 | 1.20 |
| Q14515 | SPARCL1 | 1.20 |
| P18065 | IGFBP2 | 1.20 |
| P02794 | FTH1 | 1.20 |
| Q14766 | LTBP1 | 1.20 |
| P00338 | LDHA | 1.20 |
| Q92752 | TNR | 1.20 |
| Q8TCZ2 | CD99L2 | 1.20 |
| P02814 | SMR3B | 1.20 |
| P08185 | SERPINA6 | 1.20 |
| Q15113 | PCOLCE | 1.20 |
| Q13231 | CHIT1 | 1.20 |
| 014773 | TPP1 | 1.20 |

**[Table 22] Types and expression fold changes of proteins whose expression levels were significantly downregulated in AD group**

| Accession number | Gene name | Fold change |
|---|---|---|
| P69905 | HBA1 | 0.17 |
| P02790 | HPX | 0.31 |
| P02768 | ALB | 0.33 |
| P19652 | ORM2 | 0.35 |
| P02763 | ORM1 | 0.36 |
| P31946 | YWHAB | 0.36 |
| A0A286YEY1 | IGHA1 | 0.36 |
| Q6PCB0 | VWA1 | 0.38 |
| P00738 | HP | 0.38 |
| P02787 | TF | 0.39 |
| P54802 | NAGLU | 0.41 |
| P07360 | C8G | 0.45 |
| U3KQK0 | HIST1H2BN | 0.45 |
| P14550 | AKR1A1 | 0.45 |
| P68871 | HBB | 0.47 |
| Q06033 | ITIH3 | 0.47 |
| P01009 | SERPINA1 | 0.48 |
| V9GYM3 | APOA2 | 0.48 |
| Q14696 | MESD | 0.48 |
| 015467 | CCL16 | 0.48 |
| P14543 | NID1 | 0.49 |
| P02042 | HBD | 0.52 |
| O43505 | B4GAT1 | 0.52 |
| A0A286YEY4 | IGHG2 | 0.52 |
| O95428 | PAPLN | 0.52 |
| P32119 | PRDX2 | 0.52 |
| P0DOY2 | IGLC2 | 0.53 |
| P02749 | APOH | 0.54 |
| P01008 | SERPINC1 | 0.54 |
| P00915 | CA1 | 0.54 |
| P04004 | VTN | 0.54 |
| D6RF35 | GC | 0.54 |
| P13473 | LAMP2 | 0.56 |
| Q9UBX1 | CTSF | 0.56 |
| P01871 | IGHM | 0.57 |
| P01024 | C3 | 0.57 |
| Q03591 | CFHR1 | 0.57 |
| P01042 | KNG1 | 0.58 |
| P00747 | PLG | 0.58 |
| P02743 | APCS | 0.59 |
| P78504 | JAG1 | 0.59 |
| Q9NQ79 | CRTAC1 | 0.60 |
| Q92563 | SPOCK2 | 0.60 |
| Q9H1Z8 | C2orf40 | 0.60 |
| P25311 | AZGP1 | 0.61 |
| 000391 | QSOX1 | 0.61 |
| Q8WY21 | SORCS1 | 0.61 |
| A0A286YES1 | IGHG3 | 0.61 |
| 095715 | CXCL14 | 0.61 |
| P06681 | C2 | 0.61 |
| P02545 | LMNA | 0.61 |
| A0A286YFJ8 | IGHG4 | 0.61 |
| Q9Y5W5 | WIF1 | 0.62 |
| P01591 | JCHAIN | 0.62 |
| P02741 | CRP | 0.62 |
| P06276 | BCHE | 0.64 |
| A0A0B4J231 | IGLL5 | 0.64 |
| P02765 | AHSG | 0.64 |
| P35858 | IGFALS | 0.64 |
| P00748 | F12 | 0.64 |
| P98172 | EFNB1 | 0.64 |
| P02679 | FGG | 0.65 |
| Q9NZP8 | C1RL | 0.65 |
| P17181 | IFNAR1 | 0.65 |
| P08697 | SERPINF2 | 0.65 |
| P01834 | IGKC | 0.66 |
| Q08629 | SPOCK1 | 0.66 |
| P07996 | THBS1 | 0.66 |
| P22352 | GPX3 | 0.67 |
| P01023 | A2M | 0.67 |
| P15169 | CPN1 | 0.67 |
| Q8NFT8 | DNER | 0.68 |
| B4E1Z4 | CFB | 0.68 |
| P61812 | TGFB2 | 0.69 |
| Q86UN2 | RTN4RL1 | 0.69 |
| Q8TDQ0 | HAVCR2 | 0.70 |
| Q6ZMP0 | THSD4 | 0.70 |
| P04217 | A1BG | 0.70 |
| O75882 | ATRN | 0.71 |
| Q9NPH3 | IL1RAP | 0.71 |
| P34096 | RNASE4 | 0.71 |
| Q13443 | ADAM9 | 0.71 |
| A0A2Q2TTZ9 | IGKV1D-33 | 0.71 |
| P55290 | CDH13 | 0.71 |
| Q8N3J6 | CADM2 | 0.71 |
| O00264 | PGRMC1 | 0.72 |
| P19827 | ITIH1 | 0.72 |
| P01031 | C5 | 0.72 |
| C9JF17 | APOD | 0.72 |
| J3KPA1 | CRISP3 | 0.72 |
| P10643 | C7 | 0.72 |
| Q13867 | BLMH | 0.72 |
| P02774 | GC | 0.72 |
| Q2TAL6 | VWC2 | 0.72 |
| P80108 | GPLD1 | 0.73 |
| Q99435 | NELL2 | 0.73 |
| P01780 | IGHV3-7 | 0.73 |
| Q9UM22 | EPDR1 | 0.74 |
| O00339 | MATN2 | 0.74 |
| P36980 | CFHR2 | 0.74 |
| Q 14624 | ITIH4 | 0.74 |
| Q04756 | HGFAC | 0.74 |
| H0YAC1 | KLKB1 | 0.74 |
| P19823 | ITIH2 | 0.74 |
| P05787 | KRT8 | 0.74 |
| A0A096LPE2 | SAA2-SAA4 | 0.74 |
| 015204 | ADAMDEC1 | 0.74 |
| P02675 | FGB | 0.74 |
| P05543 | SERPINA7 | 0.74 |
| Q13275 | SEMA3F | 0.74 |
| P16519 | PCSK2 | 0.75 |
| Q13421 | MSLN | 0.75 |
| P03951 | F11 | 0.75 |
| Q6YHK3 | CD109 | 0.75 |
| P02647 | APOA1 | 0.75 |
| Q13018 | PLA2R1 | 0.75 |
| 015537 | RS1 | 0.75 |
| P49788 | RARRES1 | 0.75 |
| P27169 | PON1 | 0.76 |
| P05155 | SERPING 1 | 0.76 |
| P07108 | DBI | 0.77 |
| Q9UBM4 | OPTC | 0.77 |
| P30044 | PRDX5 | 0.77 |
| P16870 | CPE | 0.77 |
| P98164 | LRP2 | 0.77 |
| 000451 | GFRA2 | 0.77 |
| P02748 | C9 | 0.77 |
| P08603 | CFH | 0.78 |
| Q14118 | DAG1 | 0.78 |
| P02760 | AMBP | 0.78 |
| P29622 | SERPINA4 | 0.78 |
| A0A0J9YY99 | N/A | 0.78 |
| P06727 | APOA4 | 0.78 |
| 095185 | UNC5C | 0.78 |
| Q8IWV2 | CNTN4 | 0.79 |
| 014793 | MSTN | 0.79 |
| A0A0G2JLV7 | LAIR1 | 0.79 |
| 015394 | NCAM2 | 0.79 |
| Q9BTY2 | FUCA2 | 0.79 |
| O95980 | RECK | 0.79 |
| P00734 | F2 | 0.79 |
| P0DJI8 | SAA1 | 0.79 |
| Q9BRA2 | TXNDC17 | 0.79 |
| P05546 | SERPIND 1 | 0.80 |
| Q16627 | CCL14 | 0.80 |
| P07358 | C8B | 0.80 |
| Q06828 | FMOD | 0.80 |
| P35555 | FBN1 | 0.80 |
| 014498 | ISLR | 0.80 |
| Q6UXB8 | PI16 | 0.80 |
| P00450 | CP | 0.81 |
| P15291 | B4GALT1 | 0.81 |
| P22692 | IGFBP4 | 0.81 |
| P04196 | HRG | 0.81 |
| P00740 | F9 | 0.82 |
| E7ETH0 | CFI | 0.82 |
| Q9UHG2 | PCSK1N | 0.82 |
| A0A0A0MS08 | IGHG 1 | 0.82 |
| O95497 | VNN1 | 0.82 |
| P07315 | CRYGC | 0.82 |
| Q9UGM5 | FETUB | 0.83 |
| O60888 | CUTA | 0.83 |
| Q96PD5 | PGLYRP2 | 0.83 |
| Q9NPZ5 | B3GAT2 | 0.83 |
| Q9HBL6 | LRTM1 | 0.83 |
| Q 14847 | LASP1 | 0.83 |
| Q13740 | ALCAM | 0.83 |
| J3KQ66 | RELN | 0.83 |
| Q99941 | ATF6B | 0.83 |
| P39059 | COL15A1 | 0.83 |

**[Table 23] Types and expression fold changes of proteins whose expression levels were significantly downregulated in PD group**

| Accession number | Gene name | Fold change |
|---|---|---|
| P69905 | HBA1 | 0.20 |
| Q6PCB0 | VWA1 | 0.40 |
| A0A286YEY1 | IGHA1 | 0.41 |
| D6RF35 | GC | 0.43 |
| P14550 | AKR1A1 | 0.43 |
| U3KQK0 | HIST1H2BN | 0.44 |
| P02768 | ALB | 0.45 |
| O43505 | B4GAT1 | 0.47 |
| P02790 | HPX | 0.47 |
| P13473 | LAMP2 | 0.48 |
| P19652 | ORM2 | 0.48 |
| Q06033 | ITIH3 | 0.48 |
| P34096 | RNASE4 | 0.49 |
| P30044 | PRDX5 | 0.49 |
| P31946 | YWHAB | 0.50 |
| Q8TDQ0 | HAVCR2 | 0.51 |
| P06681 | C2 | 0.52 |
| P02042 | HBD | 0.53 |
| P02749 | APOH | 0.54 |
| P05787 | KRT8 | 0.54 |
| P01871 | IGHM | 0.55 |
| P07360 | C8G | 0.55 |
| P02763 | ORM1 | 0.55 |
| P54802 | NAGLU | 0.56 |
| P68871 | HBB | 0.56 |
| P02787 | TF | 0.56 |
| Q9H1Z8 | C2orf40 | 0.57 |
| Q03591 | CFHR1 | 0.59 |
| 015467 | CCL16 | 0.60 |
| A0A286YEY4 | IGHG2 | 0.60 |
| V9GYM3 | APOA2 | 0.60 |
| P14543 | NID1 | 0.61 |
| P00738 | HP | 0.61 |
| P01008 | SERPINC1 | 0.62 |
| Q9UBX1 | CTSF | 0.62 |
| Q9Y5W5 | WIF1 | 0.63 |
| O95428 | PAPLN | 0.63 |
| P01024 | C3 | 0.65 |
| P36980 | CFHR2 | 0.65 |
| P07996 | THBS1 | 0.65 |
| P00915 | CA1 | 0.65 |
| Q9NQ79 | CRTAC1 | 0.66 |
| 014793 | MSTN | 0.66 |
| P00748 | F12 | 0.66 |
| P02679 | FGG | 0.66 |
| Q01459 | CTBS | 0.66 |
| P04004 | VTN | 0.67 |
| P01009 | SERPINA1 | 0.68 |
| Q8NFT8 | DNER | 0.68 |
| P01042 | KNG1 | 0.68 |
| A0A0B4J231 | IGLL5 | 0.69 |
| P00747 | PLG | 0.69 |
| A0A286YES1 | IGHG3 | 0.69 |
| Q9H3S1 | SEMA4A | 0.70 |
| Q8WY21 | SORCS1 | 0.70 |
| P02545 | LMNA | 0.70 |
| P15169 | CPN1 | 0.70 |
| Q9NZP8 | C1RL | 0.71 |
| 014594 | NCAN | 0.71 |
| P0DOY2 | IGLC2 | 0.71 |
| P25311 | AZGP1 | 0.71 |
| P32119 | PRDX2 | 0.71 |
| Q13421 | MSLN | 0.71 |
| P02765 | AHSG | 0.72 |
| Q04756 | HGFAC | 0.72 |
| P06276 | BCHE | 0.72 |
| Q8N3J6 | CADM2 | 0.72 |
| P02741 | CRP | 0.72 |
| P02774 | GC | 0.73 |
| Q86VB7 | CD163 | 0.74 |
| P53634 | CTSC | 0.74 |
| A6NLU5 | VSTM2B | 0.74 |
| Q14624 | ITIH4 | 0.74 |
| 000391 | QSOX1 | 0.74 |
| Q6ZMP0 | THSD4 | 0.74 |
| P16870 | CPE | 0.74 |
| Q14118 | DAG1 | 0.75 |
| P07998 | RNASE1 | 0.75 |
| Q13867 | BLMH | 0.75 |
| P23470 | PTPRG | 0.75 |
| P08174 | CD55 | 0.75 |
| P05155 | SERPING 1 | 0.75 |
| O00339 | MATN2 | 0.75 |
| P08603 | CFH | 0.75 |
| B4E1Z4 | CFB | 0.75 |
| P04217 | A1BG | 0.76 |
| A0A2Q2TTZ9 | IGKV1D-33 | 0.76 |
| P01591 | JCHAIN | 0.76 |
| P29622 | SERPINA4 | 0.76 |
| P01023 | A2M | 0.76 |
| P10599 | TXN | 0.77 |
| P27169 | PON1 | 0.77 |
| P01034 | CST3 | 0.77 |
| Q14696 | MESD | 0.77 |
| P16519 | PCSK2 | 0.77 |
| Q14533 | KRT81 | 0.77 |
| P98172 | EFNB1 | 0.77 |
| Q13275 | SEMA3F | 0.78 |
| P14151 | SELL | 0.78 |
| 015537 | RS1 | 0.78 |
| P03951 | F11 | 0.78 |
| P06703 | S100A6 | 0.78 |
| P35858 | IGFALS | 0.78 |
| P04196 | HRG | 0.79 |
| P08697 | SERPINF2 | 0.79 |
| P07108 | DBI | 0.79 |
| P22914 | CRYGS | 0.79 |
| G3V3D1 | NPC2 | 0.79 |
| J3KPA1 | CRISP3 | 0.80 |
| P61278 | SST | 0.80 |
| H0YAC1 | KLKB1 | 0.80 |
| P19823 | ITIH2 | 0.80 |
| P01834 | IGKC | 0.80 |
| 095715 | CXCL14 | 0.80 |
| P98164 | LRP2 | 0.80 |
| Q9NPH3 | IL1RAP | 0.80 |
| P09382 | LGALS1 | 0.81 |
| A0A0A0MS08 | IGHG 1 | 0.81 |
| P13646 | KRT13 | 0.81 |
| P00734 | F2 | 0.81 |
| P01031 | C5 | 0.81 |
| Q13443 | ADAM9 | 0.81 |
| Q9BRA2 | TXNDC17 | 0.81 |
| P50895 | BCAM | 0.81 |
| Q99538 | LGMN | 0.81 |
| H3BLU2 | LSAMP | 0.81 |
| P00390 | GSR | 0.81 |
| P17643 | TYRP 1 | 0.81 |
| P01780 | IGHV3-7 | 0.81 |
| C9JF17 | APOD | 0.82 |
| P40189 | IL6ST | 0.82 |
| Q99435 | NELL2 | 0.82 |
| K7ES70 | MFAP4 | 0.82 |
| O75882 | ATRN | 0.82 |
| P19022 | CDH2 | 0.82 |
| P19827 | ITIH1 | 0.82 |
| P07355 | ANXA2 | 0.82 |
| P61812 | TGFB2 | 0.83 |
| 095185 | UNC5C | 0.83 |
| P55290 | CDH13 | 0.83 |
| Q96MU8 | KREMEN1 | 0.83 |
| 076061 | STC2 | 0.83 |
| Q8TDF5 | NETO1 | 0.83 |
| P29279 | CTGF | 0.83 |
| 015394 | NCAM2 | 0.83 |
| 014498 | ISLR | 0.83 |
| P19021 | PAM | 0.83 |
| O95980 | RECK | 0.83 |

**[Table 24] Types and expression fold changes of proteins whose expression levels were significantly downregulated in CVA group**

| Accession number | Gene name | Fold change |
|---|---|---|
| P69905 | HBA1 | 0.16 |
| P19652 | ORM2 | 0.27 |
| P02763 | ORM1 | 0.35 |
| Q6PCB0 | VWA1 | 0.37 |
| Q8TDQ0 | HAVCR2 | 0.38 |
| P02787 | TF | 0.38 |
| O95428 | PAPLN | 0.39 |
| P07998 | RNASE1 | 0.41 |
| Q14696 | MESD | 0.41 |
| P02749 | APOH | 0.41 |
| O43505 | B4GAT1 | 0.41 |
| P34096 | RNASE4 | 0.41 |
| P00915 | CA1 | 0.41 |
| P02768 | ALB | 0.42 |
| P13473 | LAMP2 | 0.42 |
| U3KQK0 | HIST1H2BN | 0.44 |
| 015467 | CCL16 | 0.44 |
| A0A286YEY1 | IGHA1 | 0.44 |
| P14550 | AKR1A1 | 0.45 |
| Q9H1Z8 | C2orf40 | 0.46 |
| P02042 | HBD | 0.46 |
| Q9Y5W5 | WIF1 | 0.48 |
| P78504 | JAG1 | 0.49 |
| P36980 | CFHR2 | 0.49 |
| Q01459 | CTBS | 0.50 |
| Q03591 | CFHR1 | 0.51 |
| P02790 | HPX | 0.51 |
| P01008 | SERPINC1 | 0.52 |
| Q06033 | ITIH3 | 0.52 |
| Q9UBX1 | CTSF | 0.52 |
| P01871 | IGHM | 0.53 |
| P31946 | YWHAB | 0.53 |
| P02743 | APCS | 0.55 |
| P04155 | TFF1 | 0.55 |
| P98172 | EFNB1 | 0.55 |
| Q8N3J6 | CADM2 | 0.56 |
| P25311 | AZGP1 | 0.57 |
| P07360 | C8G | 0.57 |
| V9GYM3 | APOA2 | 0.57 |
| P06681 | C2 | 0.58 |
| P68871 | HBB | 0.58 |
| Q13201 | MMRN1 | 0.58 |
| Q8NFT8 | DNER | 0.59 |
| Q8WY21 | SORCS1 | 0.59 |
| Q9HBL6 | LRTM1 | 0.59 |
| P80108 | GPLD1 | 0.59 |
| P01042 | KNG1 | 0.59 |
| Q9HCQ7 | NPVF | 0.60 |
| Q08629 | SPOCK1 | 0.61 |
| O15204 | ADAMDEC1 | 0.61 |
| P02545 | LMNA | 0.61 |
| O00339 | MATN2 | 0.61 |
| Q13275 | SEMA3F | 0.61 |
| P00748 | F12 | 0.62 |
| P01591 | JCHAIN | 0.63 |
| P05787 | KRT8 | 0.63 |
| G3V3D1 | NPC2 | 0.64 |
| A6NLU5 | VSTM2B | 0.64 |
| O75882 | ATRN | 0.64 |
| A0A286YEY4 | IGHG2 | 0.64 |
| P08603 | CFH | 0.64 |
| A0A0G2JLV7 | LAIR1 | 0.64 |
| P02765 | AHSG | 0.64 |
| P04217 | A1BG | 0.64 |
| Q92954 | PRG4 | 0.65 |
| P14543 | NID1 | 0.65 |
| Q6UXB8 | PI16 | 0.65 |
| D6RF35 | GC | 0.65 |
| 095185 | UNC5C | 0.65 |
| Q9H3S1 | SEMA4A | 0.66 |
| P02679 | FGG | 0.66 |
| Q14533 | KRT81 | 0.67 |
| P00738 | HP | 0.67 |
| P01009 | SERPINA1 | 0.67 |
| J3KPA1 | CRISP3 | 0.67 |
| P54802 | NAGLU | 0.67 |
| P16519 | PCSK2 | 0.67 |
| P19022 | CDH2 | 0.67 |
| 014793 | MSTN | 0.67 |
| P49257 | LMAN1 | 0.68 |
| 094910 | ADGRL1 | 0.68 |
| P16870 | CPE | 0.68 |
| P01023 | A2M | 0.68 |
| P27169 | PON1 | 0.68 |
| 015394 | NCAM2 | 0.69 |
| E9PLM6 | MDK | 0.69 |
| Q14118 | DAG1 | 0.70 |
| Q6ZMP0 | THSD4 | 0.70 |
| Q 14624 | ITIH4 | 0.70 |
| P06276 | BCHE | 0.70 |
| P01024 | C3 | 0.71 |
| 095715 | CXCL14 | 0.71 |
| P17181 | IFNAR1 | 0.71 |
| Q16627 | CCL14 | 0.71 |
| P35555 | FBN1 | 0.71 |
| P02818 | BGLAP | 0.72 |
| P25774 | CTSS | 0.72 |
| P30044 | PRDX5 | 0.72 |
| P02675 | FGB | 0.72 |
| Q99435 | NELL2 | 0.73 |
| Q13510 | ASAH1 | 0.73 |
| P50895 | BCAM | 0.73 |
| P55290 | CDH13 | 0.73 |
| P81605 | DCD | 0.73 |
| P00918 | CA2 | 0.73 |
| P15169 | CPN1 | 0.74 |
| P08174 | CD55 | 0.74 |
| Q96MU8 | KREMEN1 | 0.74 |
| P19957 | PI3 | 0.74 |
| P22304 | IDS | 0.74 |
| P53634 | CTSC | 0.74 |
| H3BLU2 | LSAMP | 0.74 |
| Q2TAL6 | VWC2 | 0.74 |
| O95490 | ADGRL2 | 0.74 |
| A0A087WZ82 | FXYD6-FXYD2 | 0.75 |
| Q04756 | HGFAC | 0.75 |
| P98155 | VLDLR | 0.75 |
| A0A0G2JMH6 | HLA-DRA | 0.75 |
| P01034 | CST3 | 0.75 |
| Q13740 | ALCAM | 0.75 |
| A0A286YES1 | IGHG3 | 0.75 |
| P04004 | VTN | 0.75 |
| P0DOY2 | IGLC2 | 0.76 |
| P01833 | PIGR | 0.76 |
| Q13018 | PLA2R1 | 0.76 |
| P48745 | NOV | 0.76 |
| P31431 | SDC4 | 0.76 |
| Q9BRA2 | TXNDC17 | 0.76 |
| Q9UM22 | EPDR1 | 0.77 |
| P05543 | SERPINA7 | 0.77 |
| H0YAC1 | KLKB1 | 0.77 |
| Q9NQ79 | CRTAC1 | 0.77 |
| Q9HAT2 | SIAE | 0.77 |
| 000391 | QSOX1 | 0.77 |
| E7ETH0 | CFI | 0.77 |
| A0A087WYL5 | SEZ6L2 | 0.77 |
| Q96AP7 | ESAM | 0.77 |
| 015537 | RS1 | 0.77 |
| A0A2Q2TTZ9 | IGKV1D-33 | 0.78 |
| P29622 | SERPINA4 | 0.78 |
| P98164 | LRP2 | 0.78 |
| O95980 | RECK | 0.78 |
| P49908 | SELENOP | 0.78 |
| P19021 | PAM | 0.78 |
| A0A0B4J231 | IGLL5 | 0.78 |
| Q14508 | WFDC2 | 0.79 |
| P10253 | GAA | 0.79 |
| P02774 | GC | 0.79 |
| P61812 | TGFB2 | 0.79 |
| Q9UBM4 | OPTC | 0.79 |
| Q9UKB5 | AJAP1 | 0.79 |
| P40189 | IL6ST | 0.79 |
| O75752 | B3GALNT1 | 0.79 |
| P02753 | RBP4 | 0.79 |
| P47972 | NPTX2 | 0.79 |
| B4E1Z4 | CFB | 0.79 |
| P15291 | B4GALT1 | 0.79 |
| 014594 | NCAN | 0.79 |
| P07996 | THBS1 | 0.79 |
| Q96HD 1 | CRELD1 | 0.80 |
| O75787 | ATP6AP2 | 0.80 |
| P04196 | HRG | 0.80 |
| E9PR17 | CD59 | 0.80 |
| 014498 | ISLR | 0.80 |
| P02741 | CRP | 0.80 |
| Q6MZW2 | FSTL4 | 0.80 |
| P20933 | AGA | 0.80 |
| P21246 | PTN | 0.81 |
| P03951 | F11 | 0.81 |
| P17643 | TYRP1 | 0.81 |
| A0A087WWT2 | NRN1 | 0.81 |
| Q9ULB1 | NRXN1 | 0.81 |
| Q8N3Z0 | PRSS35 | 0.81 |
| Q9NP84 | TNFRSF12A | 0.81 |
| P10909 | CLU | 0.81 |
| Q9Y287 | ITM2B | 0.81 |
| Q13421 | MSLN | 0.81 |
| O95206 | PCDH8 | 0.81 |
| P61278 | SST | 0.81 |
| O95390 | GDF11 | 0.82 |
| P11684 | SCGB1A1 | 0.82 |
| P00747 | PLG | 0.82 |
| Q9NZP8 | C1RL | 0.82 |
| P33151 | CDH5 | 0.82 |
| P01780 | IGHV3-7 | 0.82 |
| Q14563 | SEMA3A | 0.82 |
| 076061 | STC2 | 0.82 |
| P35318 | ADM | 0.82 |
| 000115 | DNASE2 | 0.82 |
| Q9UGM5 | FETUB | 0.83 |
| P23470 | PTPRG | 0.83 |
| 043291 | SPINT2 | 0.83 |
| Q8NHP8 | PLBD2 | 0.83 |
| A8MV23 | SERPINE3 | 0.83 |
| Q13308 | PTK7 | 0.83 |
| P61626 | LYZ | 0.83 |
| Q4KMG0 | CDON | 0.83 |
| A0A1B0GV53 | CLEC 19A | 0.83 |

**[Table 25] Types and expression fold changes of proteins whose expression levels were significantly downregulated in BT group**

| Accession number | Gene name | Fold change |
|---|---|---|
| P69905 | HBA1 | 0.13 |
| P19652 | ORM2 | 0.31 |
| Q6PCB0 | VWA1 | 0.35 |
| P30044 | PRDX5 | 0.37 |
| P02787 | TF | 0.38 |
| P31946 | YWHAB | 0.40 |
| 015467 | CCL16 | 0.41 |
| P02763 | ORM1 | 0.41 |
| P14550 | AKR1A1 | 0.41 |
| P68871 | HBB | 0.45 |
| P00915 | CA1 | 0.47 |
| Q14696 | MESD | 0.48 |
| P13473 | LAMP2 | 0.49 |
| Q9Y5W5 | WIF1 | 0.49 |
| P78504 | JAG1 | 0.49 |
| A0A286YEY1 | IGHA1 | 0.49 |
| P04004 | VTN | 0.49 |
| Q06033 | ITIH3 | 0.49 |
| P02768 | ALB | 0.49 |
| P01871 | IGHM | 0.51 |
| P02790 | HPX | 0.52 |
| D6RF35 | GC | 0.52 |
| U3KQK0 | HIST1H2BN | 0.53 |
| P02749 | APOH | 0.53 |
| Q8TDQ0 | HAVCR2 | 0.53 |
| O95428 | PAPLN | 0.54 |
| O43505 | B4GAT1 | 0.54 |
| P17181 | IFNAR1 | 0.55 |
| P02042 | HBD | 0.55 |
| P01008 | SERPINC1 | 0.55 |
| P54802 | NAGLU | 0.55 |
| Q9H1Z8 | C2orf40 | 0.56 |
| 014793 | MSTN | 0.57 |
| P07360 | C8G | 0.57 |
| P34096 | RNASE4 | 0.58 |
| Q01459 | CTBS | 0.58 |
| Q8WY21 | SORCS1 | 0.58 |
| P14543 | NID1 | 0.58 |
| Q9UBX1 | CTSF | 0.58 |
| P05787 | KRT8 | 0.58 |
| Q8NFT8 | DNER | 0.59 |
| P00738 | HP | 0.59 |
| P00748 | F12 | 0.59 |
| P01034 | CST3 | 0.60 |
| P15291 | B4GALT1 | 0.61 |
| A0A286YEY4 | IGHG2 | 0.61 |
| Q03591 | CFHR1 | 0.61 |
| P98172 | EFNB1 | 0.61 |
| J3KPA1 | CRISP3 | 0.62 |
| A0A087WZ82 | FXYD6-FXYD2 | 0.63 |
| Q9H3S1 | SEMA4A | 0.64 |
| 095715 | CXCL14 | 0.64 |
| P02765 | AHSG | 0.65 |
| P01591 | JCHAIN | 0.65 |
| P04217 | A1BG | 0.65 |
| O00339 | MATN2 | 0.65 |
| 015394 | NCAM2 | 0.66 |
| Q13275 | SEMA3F | 0.67 |
| P01042 | KNG1 | 0.67 |
| P01024 | C3 | 0.68 |
| P00492 | HPRT1 | 0.68 |
| P10909 | CLU | 0.68 |
| P25311 | AZGP1 | 0.68 |
| P08174 | CD55 | 0.68 |
| H3BLU2 | LSAMP | 0.69 |
| Q6ZMP0 | THSD4 | 0.69 |
| 000391 | QSOX1 | 0.70 |
| Q8N3J6 | CADM2 | 0.70 |
| P07996 | THBS1 | 0.70 |
| Q04756 | HGFAC | 0.70 |
| P08603 | CFH | 0.70 |
| P81605 | DCD | 0.70 |
| Q6UXB8 | PI16 | 0.70 |
| P80108 | GPLD1 | 0.70 |
| P0DOY2 | IGLC2 | 0.71 |
| Q96DR8 | MUCL1 | 0.71 |
| Q9NQ79 | CRTAC1 | 0.71 |
| P02545 | LMNA | 0.71 |
| P98155 | VLDLR | 0.71 |
| O60888 | CUTA | 0.71 |
| P04196 | HRG | 0.71 |
| O75752 | B3GALNT1 | 0.71 |
| P22304 | IDS | 0.72 |
| P16519 | PCSK2 | 0.72 |
| 015537 | RS1 | 0.72 |
| P19021 | PAM | 0.72 |
| O75787 | ATP6AP2 | 0.72 |
| P15121 | AKR1B1 | 0.72 |
| Q99969 | RARRES2 | 0.72 |
| P40189 | IL6ST | 0.72 |
| A0A087WYL5 | SEZ6L2 | 0.72 |
| P61278 | SST | 0.72 |
| P04075 | ALDOA | 0.72 |
| P06396 | GSN | 0.73 |
| Q16568 | CARTPT | 0.73 |
| A6NLU5 | VSTM2B | 0.73 |
| Q6MZW2 | FSTL4 | 0.73 |
| P61812 | TGFB2 | 0.73 |
| P26447 | S100A4 | 0.73 |
| Q13421 | MSLN | 0.73 |
| P22914 | CRYGS | 0.73 |
| O95980 | RECK | 0.74 |
| Q 14624 | ITIH4 | 0.74 |
| P14625 | HSP90B1 | 0.74 |
| Q8N3Z0 | PRSS35 | 0.74 |
| Q2TAL6 | VWC2 | 0.74 |
| P53634 | CTSC | 0.74 |
| P32119 | PRDX2 | 0.74 |
| P08670 | VIM | 0.75 |
| P36980 | CFHR2 | 0.75 |
| O95390 | GDF11 | 0.75 |
| Q86SF2 | GALNT7 | 0.75 |
| Q9UJJ9 | GNPTG | 0.75 |
| Q9NZP8 | C1RL | 0.75 |
| 014594 | NCAN | 0.75 |
| Q86VB7 | CD163 | 0.75 |
| Q8NFY4 | SEMA6D | 0.76 |
| P16870 | CPE | 0.76 |
| P02743 | APCS | 0.76 |
| P27169 | PON1 | 0.76 |
| Q7Z7H5 | TMED4 | 0.76 |
| P02679 | FGG | 0.76 |
| P55290 | CDH13 | 0.76 |
| P25774 | CTSS | 0.77 |
| P98164 | LRP2 | 0.77 |
| Q13018 | PLA2R1 | 0.77 |
| Q9UHG2 | PCSK1N | 0.77 |
| G3V3D1 | NPC2 | 0.77 |
| Q9UGM5 | FETUB | 0.77 |
| P29622 | SERPINA4 | 0.77 |
| Q13443 | ADAM9 | 0.77 |
| P13646 | KRT13 | 0.77 |
| Q9Y646 | CPQ | 0.77 |
| V9GYM3 | APOA2 | 0.77 |
| Q9Y2I2 | NTNG1 | 0.77 |
| Q9UM22 | EPDR1 | 0.77 |
| P01834 | IGKC | 0.78 |
| A0A2Q2TTZ9 | IGKV1D-33 | 0.78 |
| P40967 | PMEL | 0.78 |
| P01009 | SERPINA1 | 0.78 |
| P07451 | CA3 | 0.78 |
| J3KNP4 | SEMA4B | 0.78 |
| P49257 | LMAN1 | 0.78 |
| O95336 | PGLS | 0.78 |
| Q14533 | KRT81 | 0.78 |
| P07998 | RNASE1 | 0.78 |
| P00441 | SOD1 | 0.78 |
| P01023 | A2M | 0.78 |
| Q96HD 1 | CRELD1 | 0.78 |
| 076061 | STC2 | 0.78 |
| Q4KMG0 | CDON | 0.78 |
| P03951 | F11 | 0.79 |
| P01780 | IGHV3-7 | 0.79 |
| A0A0G2JMH6 | HLA-DRA | 0.79 |
| P10586 | PTPRF | 0.79 |
| Q86UD1 | OAF | 0.79 |
| P41222 | PTGDS | 0.80 |
| A6NGN9 | IGLON5 | 0.80 |
| P02774 | GC | 0.80 |
| P42857 | NSG1 | 0.80 |
| A0A0A0MS08 | IGHG 1 | 0.80 |
| Q9BRA2 | TXNDC17 | 0.80 |
| P47972 | NPTX2 | 0.80 |
| A0A286YES1 | IGHG3 | 0.80 |
| P19827 | ITIHI | 0.80 |
| Q96AP7 | ESAM | 0.80 |
| F5GWQ8 | CLUL1 | 0.80 |
| Q9UKB5 | AJAP1 | 0.80 |
| Q8N436 | CPXM2 | 0.81 |
| Q96FE5 | LINGO1 | 0.81 |
| A0A0J9YY99 | N/A | 0.81 |
| P10599 | TXN | 0.81 |
| Q495W5 | FUT11 | 0.81 |
| Q13740 | ALCAM | 0.81 |
| K7ES70 | MFAP4 | 0.81 |
| Q99941 | ATF6B | 0.81 |
| A0A286YFJ8 | IGHG4 | 0.81 |
| Q658N2 | WSCD1 | 0.81 |
| Q5JS37 | NHLRC3 | 0.81 |
| P10253 | GAA | 0.82 |
| Q8IWV2 | CNTN4 | 0.82 |
| Q99784 | OLFM1 | 0.82 |
| Q14563 | SEMA3A | 0.82 |
| P35858 | IGFALS | 0.82 |
| Q8NFP4 | MDGA1 | 0.82 |
| O95490 | ADGRL2 | 0.82 |
| P08697 | SERPINF2 | 0.82 |
| Q99538 | LGMN | 0.82 |
| P00740 | F9 | 0.82 |
| Q6YHK3 | CD109 | 0.82 |
| Q96JP9 | CDHR1 | 0.82 |
| P08758 | ANXA5 | 0.82 |
| Q92484 | SMPDL3A | 0.82 |
| Q16849 | PTPRN | 0.82 |
| Q8WXD2 | SCG3 | 0.82 |
| Q9NPZ5 | B3GAT2 | 0.82 |
| 014498 | ISLR | 0.83 |
| P00918 | CA2 | 0.83 |
| Q99435 | NELL2 | 0.83 |
| O75326 | SEMA7A | 0.83 |
| Q86VZ4 | LRP11 | 0.83 |
| P02649 | APOE | 0.83 |
| Q 17R60 | IMPG1 | 0.83 |
| Q9UNW1 | MINPP1 | 0.83 |
| Q08629 | SPOCK1 | 0.83 |
| P00734 | F2 | 0.83 |
| P08294 | SOD3 | 0.83 |
| P07355 | ANXA2 | 0.83 |
| P15848 | ARSB | 0.83 |
| 015204 | ADAMDEC 1 | 0.83 |

Thereafter, analysis was made as to whether the AH DEPs whose expression levels were significantly upregulated in the AD, PD, CVA and BT groups overlap between the groups, and the results are shown in FIG. 5. The list of biomarker genes belonging to each group in FIG. 5 is summarized, and the results are shown in Table 26 below.

**[Table 26]**

| Gene name | | | | | | | | | | | | | | |
|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 |
| PRD X1 | CRY AA | CRY AB | FT H1 | OPNIL W | HSPB 1 | YWH AE | RTN4 RL2 | CA CH D1 | ADG RL3 | EMI LIN2 | SEM G2 | NTM | PSC A | PPIA |
| OMG | CRY BA4 | NQ 01 | PT N | TMSB4X | SFN | PKM | GGCT | PC DH 9 | THB S4 | SH3 BGR L3 | SEM G1 | FSTL 1 | GST O1 | FAM 19A4 |
| COL 2A1 | CRY BBI | TKT | TN R | FLNA | KRT4 | LGAL S7 | A0A0 U1RR H7 | GA S1 | GRP | EFN B2 | SCGB 1A1 | FGA | ATO X1 | KRT 13 |
| NCA N | PTM S | ALD H3A 1 | DL K2 | COL9A3 | C1QA | GAPD H | SORC S2 | H3F 3B | GPH B5 | CXC L17 | ART3 | CSF1 R | DCN | HSP E1 |
| CCL 2 | CRY BB2 | ENO 1 | CN DP1 | TFPI | IGHV 4-39 | KRT9 | MMP2 | FU T11 | SERP INB3 | FLN C | PIGR | APO A4 | SOD 2 | ANG PTL7 |
| FAB P5 | GA NAB | YW HAZ | LA P3 | CHAD | HDGF | GSTP 1 | KRT84 | AT F6B | B3G AT2 | NRN 1L | SPIN K5 | CRP | TGF BI | VIM |
| FAM 198A | CBL N1 | HEG 1 | FG L2 | PSMA6 | GPR3 7L1 | PRDX 6 | GAS6 | AP CS | ENO 2 | COL 1A1 | DMK N | TWS G1 | ADA MTS L2 | SPP1 |
| EGF LAM | HEB P2 | AD H7 | NO TC H2 | PCDH8 | CRYB A1 | KRT1 7 | ALDO A | OP CM L | KRT 6B | PCO LCE | AMB P | ADA M22 | MGP | F5 |
| ACH E | C1Q TNF 3 | SMR 3B | FC GB P | S100A8 | ANG | NUD T5 | ENPP4 | LR TM 1 | S100 A7 | LOX | PPBP | AGR N | VSIG 4 | FBL N7 |
| NCA M1 | NPC 1 | HSP A1B | PE BP4 | CPVL | CRYZ | CTSA | HTRA 1 | AM Y1 A | CGR EF1 | CST B | AHN AK | LAM C1 | CYT L1 | AKR 1B1 |
| ATP6 AP1 | SAG | ALD H1A 1 | WI SP2 | SDF4 | HRN R | SEM A6A | MARC KS | GA LN S | CP | EDN 3 | PI3 | KLK 11 | VCA M1 | BST1 |
| FOL R1 | PEB P1 | IDH 1 | CHI 3L1 | LYVE1 | KRT1 0 | PLTP | VMO1 | AD A2 | CKB | LAM B1 | SPIN K1 | HSP G2 | IGFB P2 | ADG RB3 |
| GRI A4 | LTF | LAC RT | CD 44 | SYNJ2B P-COX16 | PTM A | SCG2 | MANS C1 | SM OC 1 | GPN MB | MYD GF | CST6 | PDG FD | | MDH 1 |
| GDI2 | FRZ B | PFN 1 | | CDSN | KRT6 A | LEFT Y2 | BGLA P | AS PH | DSC 3 | LGA LS3B P | SBSN | OGN | | CNT NAP 4 |
| OLF M2 | ME GF1 0 | CPN 2 | | | JAM3 | HSP9 0AA1 | L1CA M | WF DC 1 | | RNA SE2 | FABP 1 | FAH | | CND P2 |
| SOD 1 | LCN 1 | LDH A | | | LOR | NME1 - NME2 | GBA | SFR P4 | | COL 9A2 | CFD | SPO CK2 | | ITGB L1 |
| C1Q L1 | GLO 1 | C1S | | | KRT7 7 | CAL ML5 | MEGF 11 | NP VF | | IGFB P1 | PTPR J | IGFB P7 | | ANP EP |
| NEU 1 | MD H2 | PDG FA | | | COL6 A1 | MIF | CDH3 | | | | TFF1 | ADA M10 | | ERA P1 |
| PTPR Z1 | ME D17 | PEP D | | | CYCS | DSP | EFEM P2 | | | | MFAP 2 | LEA P2 | | CDH 11 |
| SLPI | UBA 1 | | | | MEG F6 | IL36G | CX3C L1 | | | | LCN2 | SPIN K4 | | SERP IND1 |
| SEZ6 | CST 4 | | | | KRT5 | PRNP | CRYG D | | | | SLUR P1 | GOL M1 | | TPI1 |
| BTD | GPI | | | | COL3 A1 | PSAT 1 | SORT 1 | | | | SEM A3E | CFH R3 | | SPIN T1 |
| RNA | GLO | | | | KPRP | OLF | S100A | | | | SERPI | SAA | | HAB |
| SET2 | D4 | | | | | ML3 | 9 | | | | NB12 | 1 | | P2 |
| PSM A7 | | | | | TGM1 | CBR1 | MFRP | | | | COL4 A2 | BLV RB | | CPM |
| POM GNT 1 | | | | | GUC A2A | GAL NT18 | JUP | | | | MEP1 A | LYP D3 | | F10 |
| ANX A1 | | | | | PPIC | COL1 2A1 | MIA3 | | | | DSC1 | RAR RES1 | | VNN 1 |
| SERP INI1 | | | | | BMP3 | SHBG | CASP1 4 | | | | | LRG 1 | | |
| MTP N | | | | | FAIM 2 | CA3 | MET | | | | | FBL N1 | | |
| COT L1 | | | | | XP32 | SSC5 D | MANB A | | | | | NOV | | |
| PTPR D | | | | | TPM1 | EYS | SERPI NF1 | | | | | CD93 | | |
| RBP3 | | | | | SH3B GRL | CPB2 | CA14 | | | | | VGF | | |
| EGF R | | | | | TGM3 | GUSB | PTPR U | | | | | PCD H1 | | |
| CAL R | | | | | FLG2 | | GGH | | | | | PRO S1 | | |
| NRX N2 | | | | | ADIR F | | OSMR | | | | | LAS P1 | | |
| EEF1 | | | | | TNFR | | KRT31 | | | | | OMD | | |
| A1 | | | | | SF1A | | | | | | | | | |
| GFR A1 | | | | | ME1 | | | | | | | CDH 12 | | |
| CLE C19A | | | | | ICAM 1 | | | | | | | CFP | | |
| END OD1 | | | | | KRT1 4 | | | | | | | SDC 4 | | |
| ACT B | | | | | SRGN | | | | | | | LAM A2 | | |
| CTS L | | | | | RPS2 7A | | | | | | | COL 6A3 | | |
| HSP A5 | | | | | KRT7 8 | | | | | | | SPA RCL 1 | | |
| PGA M1 | | | | | KRT2 | | | | | | | LTB P1 | | |
| PCM T1 | | | | | PIP | | | | | | | SERP INA6 | | |
| LTB P3 | | | | | ARH GDIB | | | | | | | CHIT 1 | | |
| VST M2A | | | | | VIT | | | | | | | TPP1 | | |
| CNT NAP 2 | | | | | CAST | | | | | | | | | |
| ACP 2 | | | | | PGK1 | | | | | | | | | |
| SIRP A | | | | | PODX L2 | | | | | | | | | |
| ANG PTL1 | | | | | EEF2 | | | | | | | | | |
| NPT X1 | | | | | KRT1 6 | | | | | | | | | |
| COL 1A2 | | | | | ARG1 | | | | | | | | | |
| KLK 6 | | | | | FLG | | | | | | | | | |
| GLB 1 | | | | | MRC2 | | | | | | | | | |
| VCL | | | | | HAL | | | | | | | | | |
| | | | | | PSMB 5 | | | | | | | | | |
| | | | | | GLRX | | | | | | | | | |
| | | | | | LDHB | | | | | | | | | |
| | | | | | PKP1 | | | | | | | | | |
| | | | | | PCP2 | | | | | | | | | |
| | | | | | CFL1 | | | | | | | | | |
| | | | | | KRT1 | | | | | | | | | |
| | | | | | IGFB | | | | | | | | | |
| | | | | | P6 | | | | | | | | | |
| | | | | | CD99 L2 | | | | | | | | | |
| | | | | | LING O3 | | | | | | | | | |
| | | | | | MAN 2B1 | | | | | | | | | |
| | | | | | TGOL N2 | | | | | | | | | |
| | | | | | FTL | | | | | | | | | |
| | | | | | C1R | | | | | | | | | |
| | | | | | IMPA D1 | | | | | | | | | |
| | | | | | VASN | | | | | | | | | |
| | | | | | MYH 9 | | | | | | | | | |
| | | | | | MRC1 | | | | | | | | | |
| | | | | | FABP 4 | | | | | | | | | |
| | | | | | DDB1 | | | | | | | | | |
| | | | | | SFRP 2 | | | | | | | | | |
| | | | | | LRRC 4B | | | | | | | | | |
| | | | | | CSTA | | | | | | | | | |
| | | | | | TTR | | | | | | | | | |
| | | | | | ACTN 4 | | | | | | | | | |
| | | | | | HPD | | | | | | | | | |
| | | | | | SELP | | | | | | | | | |
| | | | | | FUCA 1 | | | | | | | | | |
| | | | | | SERPI NA10 | | | | | | | | | |
| | | | | | TGFB R3 | | | | | | | | | |
| | | | | | NID2 | | | | | | | | | |

In addition, analysis was made as to whether the AH DEPs whose expression levels were significantly downregulated in the AD, PD, CVA and BT groups overlap between the groups, and the results are shown in FIG. 6. The list of biomarker genes belonging to each group in FIG. 6 is summarized, and the results are shown in Table 27 below.

**[Table 27]**

| Gene name | | | | | | | | | | | | | | |
|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 |
| SPOC K2 | FGB | APCS | IGHG4 | PRD X2 | HBA1 | PLG | IL1IR AP | SEL L | PTP RG | CT BS | CD 163 | HPR T1 | LMAN1 | TFF1 |
| GPX3 | SERP | JAG1 | CD109 | IGFA | HPX | C2 | C5 | S10 | BCA | SE | TX | MU | CTSS | MMR |
| | INA7 | | | LS | | | | 0A6 | M | MA 4A | N | CL1 | | N1 |
| RTN4 RL1 | OPTC | IFNA R1 | A0A0J 9YY99 | SERP INF2 | ALB | CRP | APO D | LG ALS 1 | TYR P1 | NC AN | CR YG S | AKR 1B1 | DCD | NPVF |
| PGRM C1 | LAIR 1 | SPOC K1 | CNTN4 | IGKC | ORM2 | BC HE | BLM H | GS R | KRE MEN 1 | CTS C | KR T13 | RAR RES 2 | CA2 | PRG4 |
| C7 | CCL1 4 | VWC 2 | F9 | ADA M9 | ORM1 | IGL L5 | ITIH2 | NE TO1 | | VST M2 B | LG MN | ALD OA | IDS | ADGR L1 |
| SAA2-SAA4 | FBN1 | GPL D1 | PCSK1 N | ITIH1 | YWH AB | CP N1 | SERP ING1 | CT GF | | RN ASE 1 | MF AP 4 | GSN | ADGRL 2 | MDK |
| APOA 1 | CFI | EPDR 1 | CUTA | F2 | IGHA 1 | CD H2 | DBI | | | CD5 5 | AN XA 2 | CAR TPT | FXYD6 FXYD2 | BGLA P |
| RARR ES1 | LRT M1 | ADA MDE C1 | B3GAT 2 | IGHG 1 | VWA1 | B4E 1Z4 | | | | CST 3 | | S100 A4 | VLDLR | ASAH 1 |
| GFRA 2 | | PLA2 R1 | ATF6B | | HP | AT RN | | | | KR T81 | | HSP 90B1 | HLA-DRA | PI3 |
| C9 | | PI16 | | | TF | KL KB1 | | | | NP C2 | | VIM | SEZ6L2 | PIGR |
| AMBP | | B4G | | | NAGL | DA | | | | SST | | GAL | ESAM | NOV |
| | | ALT1 | | | U | G1 | | | | | | NT7 | | |
| APOA 4 | | FETU B | | | C8G | UN C5C | | | | LSA MP | | GNP TG | GAA | SDC4 |
| FUCA 2 | | ALC AM | | | HIST1 H2BN | | | | | IL6 ST | | SEM A6D | AJAP1 | SIAE |
| SAA1 | | | | | AKR1 A1 | | | | | STC 2 | | TME D4 | B3GAL NT1 | SELE NOP |
| SERPI ND1 | | | | | HBB | | | | | PA M | | CPQ | NPTX2 | WFDC 2 |
| C8B | | | | | ITIH3 | | | | | | | NTN G1 | CRELD 1 | RBP4 |
| FMOD | | | | | SERPI NA1 | | | | | | | PME L | ATP6A P2 | CD59 |
| CP | | | | | APOA 2 | | | | | | | CA3 | FSTL4 | AGA |
| IGFBP 4 | | | | | MESD | | | | | | | SEM A4B | PRSS35 | PTN |
| VNN1 | | | | | CCL1 6 | | | | | | | PGL S | CLU | NRN1 |
| CRYG C | | | | | NID1 | | | | | | | SOD 1 | GDF11 | NRXN 1 |
| PGLY RP2 | | | | | HBD | | | | | | | PTP RF | SEMA3 A | TNFR SF12A |
| LASP 1 | | | | | B4GA T1 | | | | | | | OAF | CDON | ITM2 B |
| RELN | | | | | IGHG 2 | | | | | | | PTG DS | | PCDH 8 |
| COL1 5A1 | | | | | PAPL N | | | | | | | IGL ON5 | | SCGB 1A1 |
| | | | | | IGLC2 | | | | | | | NSG 1 | | CDH5 |
| | | | | | APOH | | | | | | | CLU L1 | | ADM |
| | | | | | SERPI NC1 | | | | | | | CPX M2 | | DNAS E2 |
| | | | | | CA1 | | | | | | | LIN GO1 | | SPINT 2 |
| | | | | | VTN | | | | | | | FUT 11 | | PLBD 2 |
| | | | | | GC | | | | | | | WSC D1 | | SERPI NE3 |
| | | | | | LAMP 2 | | | | | | | NHL RC3 | | PTK7 |
| | | | | | CTSF | | | | | | | OLF M1 | | LYZ |
| | | | | | IGHM | | | | | | | MD GA1 | | CLEC 19A |
| | | | | | C3 | | | | | | | CDH R1 | | |
| | | | | | CFHR | | | | | | | ANX | | |
| | | | | | 1 | | | | | | | A5 | | |
| | | | | | KNG1 | | | | | | | SMP DL3 A | | |
| | | | | | CRTA C1 | | | | | | | PTP RN | | |
| | | | | | C2orf4 0 | | | | | | | SCG 3 | | |
| | | | | | AZGP 1 | | | | | | | SEM A7A | | |
| | | | | | QSOX 1 | | | | | | | LRP 11 | | |
| | | | | | SORC S1 | | | | | | | APO E | | |
| | | | | | IGHG 3 | | | | | | | IMP G1 | | |
| | | | | | CXCL 14 | | | | | | | MIN PP1 | | |
| | | | | | LMN A | | | | | | | SOD 3 | | |
| | | | | | WIF1 | | | | | | | ARS B | | |
| | | | | | JCHAI N | | | | | | | | | |
| | | | | | AHSG | | | | | | | | | |
| | | | | | F12 | | | | | | | | | |
| | | | | | EFNB 1 | | | | | | | | | |
| | | | | | FGG | | | | | | | | | |
| | | | | | C1RL | | | | | | | | | |
| | | | | | THBS 1 | | | | | | | | | |
| | | | | | A2M | | | | | | | | | |
| | | | | | DNER | | | | | | | | | |
| | | | | | TGFB 2 | | | | | | | | | |
| | | | | | HAVC R2 | | | | | | | | | |
| | | | | | THSD 4 | | | | | | | | | |
| | | | | | A1BG | | | | | | | | | |
| | | | | | RNAS E4 | | | | | | | | | |
| | | | | | IGKV 1D-33 | | | | | | | | | |
| | | | | | CDH1 3 | | | | | | | | | |
| | | | | | CAD M2 | | | | | | | | | |
| | | | | | CRISP 3 | | | | | | | | | |
| | | | | | GC | | | | | | | | | |
| | | | | | NELL 2 | | | | | | | | | |
| | | | | | IGHV 3-7 | | | | | | | | | |
| | | | | | MAT N2 | | | | | | | | | |
| | | | | | CFHR 2 | | | | | | | | | |
| | | | | | ITIH4 | | | | | | | | | |
| | | | | | HGFA C | | | | | | | | | |
| | | | | | KRT8 | | | | | | | | | |
| | | | | | SEMA 3F | | | | | | | | | |
| | | | | | PCSK 2 | | | | | | | | | |
| | | | | | MSLN | | | | | | | | | |
| | | | | | F11 | | | | | | | | | |
| | | | | | RS1 | | | | | | | | | |
| | | | | | PON1 | | | | | | | | | |
| | | | | | PRDX | | | | | | | | | |
| | | | | | 5 | | | | | | | | | |
| | | | | | CPE | | | | | | | | | |
| | | | | | LRP2 | | | | | | | | | |
| | | | | | CFH | | | | | | | | | |
| | | | | | SERPI NA4 | | | | | | | | | |
| | | | | | MSTN | | | | | | | | | |
| | | | | | NCA M2 | | | | | | | | | |
| | | | | | RECK | | | | | | | | | |
| | | | | | TXND C17 | | | | | | | | | |
| | | | | | ISLR | | | | | | | | | |
| | | | | | HRG | | | | | | | | | |

In addition, based on FIGS. 5 and 6, AH biomarker proteins specific to each brain and nervous system disease were selected, and it was confirmed that, among AD-related AH markers, 54 UP-DEPs and 26 DN-DEPs were expressed specifically in the AH of the corresponding brain and nervous system disease group (FIGS. 7 and 8). Among them, proteins with large expression differences were additionally selected (UP-DEP: PRDX1, DMG, COL2A1, NCAN, CCL2, FABP5, FAM198A, EGFLAM, CHE, NCAM1, NEU1, and EEF1A1; and DN-DEP: SPOCK2, GPX3, RTN4RL1, PGRMC1, C7, SAA2-SAA4, APOA1, RARRES1, GFRA2, C9, and RELN). It was confirmed that, among PD-related AH markers, 17 UP-DEPs and 6 DN-DEPs were expressed specifically in the AH of the corresponding brain and nervous system disease group (FIGS. 9 and 10). Among them, top 6 proteins with the greatest expression differences were selected (UP-DEP: CACHD1, PCDH9, GAS1, H3F3B, FUT11, and ATF6B; and DN-DEP: SELL, S100A6, LGALS1, GSR, NETO1, and CTGF).

In addition, it was confirmed that, among CVA-related AH markers, 26 UP-DEPs and 34 DN-DEPs were expressed specifically in the AH of the corresponding brain and nervous system disease group (FIGS. 11 and 12). Amon them, top six proteins with the greatest expression differences were selected (UP-DEP: PPIA, FAM19A4, KRT13, HSPE1, ANGPTL7, and VIM; and DN-DEP: TFF1, MMRN1, NPVF, PRG4, ADGRL1, and MDK).

Finally, it was confirmed that, among BT-related AH markers, 45 UP-DEPs and 46 DN-DEPs were expressed specifically in the AH of the corresponding brain and nervous system disease (FIGS. 13 and 14). Amon them, top six proteins with the greatest expression differences were selected (UP-DEP: NTM, FSTL1, FGA, CSF1R, APOA4, and CRP; and DN-DEP: HPRT1, MUCL1, AKR1B1, RARRES2, ALDOA, and GSN).

### [Example 2] Screening of Aqueous Humor Biomarkers for Diagnosing Alzheimer's Disease

In relation to AD, validation of more diverse and precise AH biomarkers was performed. First, the correlation between the DEPs of MCI, which is a pre-stage of AD, and the DEPs of AD was checked. Among AH DEPs, each showing a difference in expression compared to CON, UP-DEPs and DN-DEPs having the same directionality were checked. Among them, 119 UP-DEPs and 118 DN-DEPs in AH crossed (FIG. 15).

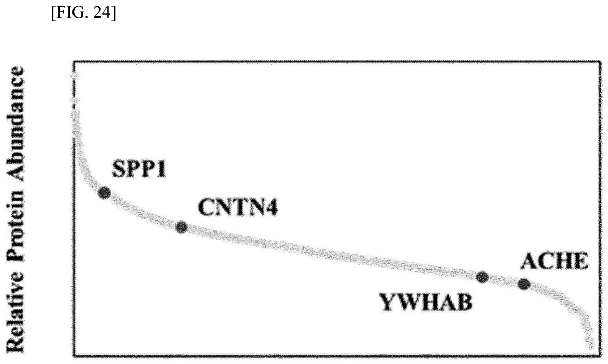

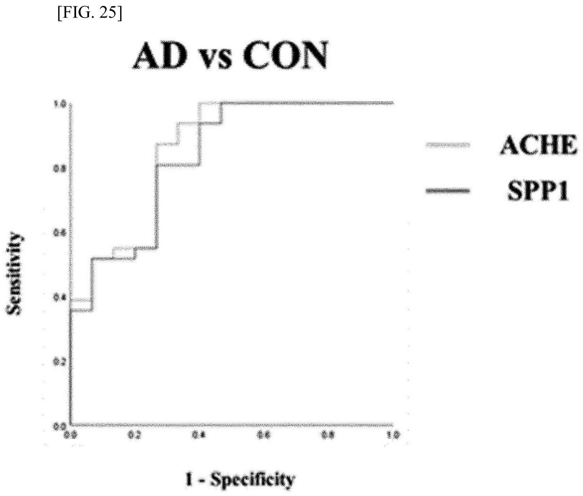

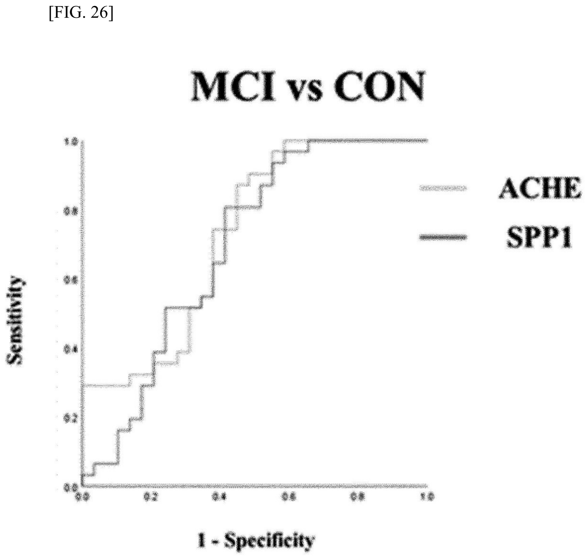

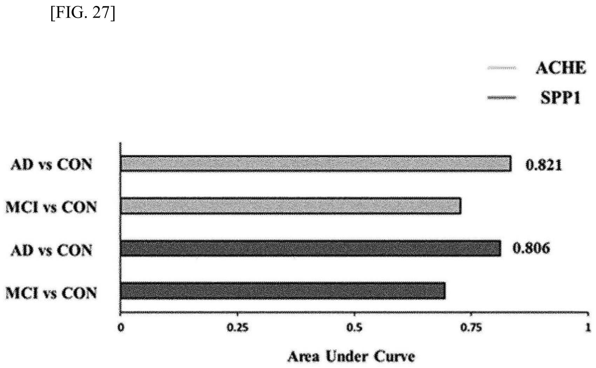

To validate AH markers associated with AD and MCI which is a pre-stage of AD, trace amounts of AH were validated in each individual patient. To this end, a parallel reaction monitoring (PRM) analysis method was applied to ensure that no protein was missed during the one-time aqueous humor analysis. For this validation experiment, 20 AD patients, 47 MCI patients and 52 CON persons were additionally recruited. As a result of performing the individual validation analysis, as shown in FIGS. 16 to 23, the following AH marker proteins could be finally derived: six AH marker proteins (ACHE, SPP1, EEF2, PRDX1 and CES1) with increased expression levels in AH; and three AH marker proteins (YWHAB, CNTN4 and BCHE) with decreased expression levels in AH. These proteins showed no significant difference in abundance, and among them, ACHE and SPP1 showed AD diagnosis rates of 82.1% and 80.6%, respectively, in aqueous humor when analyzing the ROC curves (FIGS. 24 to 27).

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The present invention relates to a method of diagnosing brain and nervous system diseases using various biomarkers.
In particular, the present invention pertains to the following:
1. A biomarker for diagnosing a brain and nervous system disease comprising: at least one gene selected from the group shown in Table 1 below; or a protein encoded thereby: 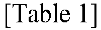 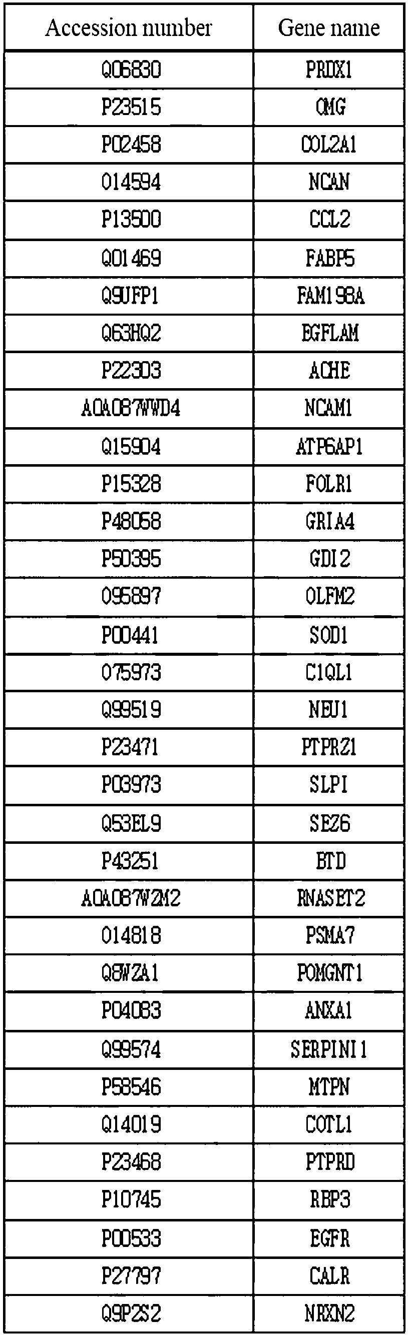 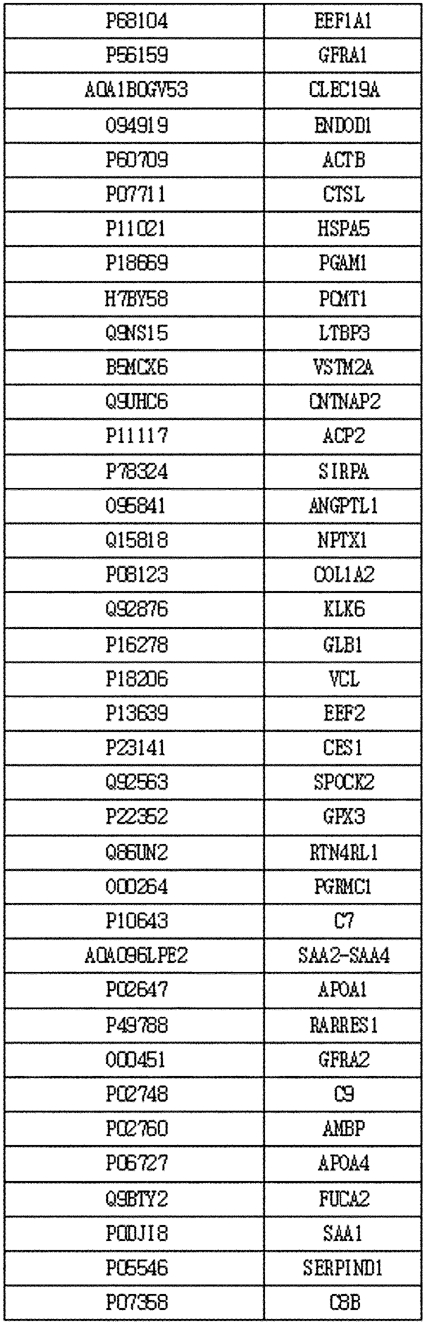 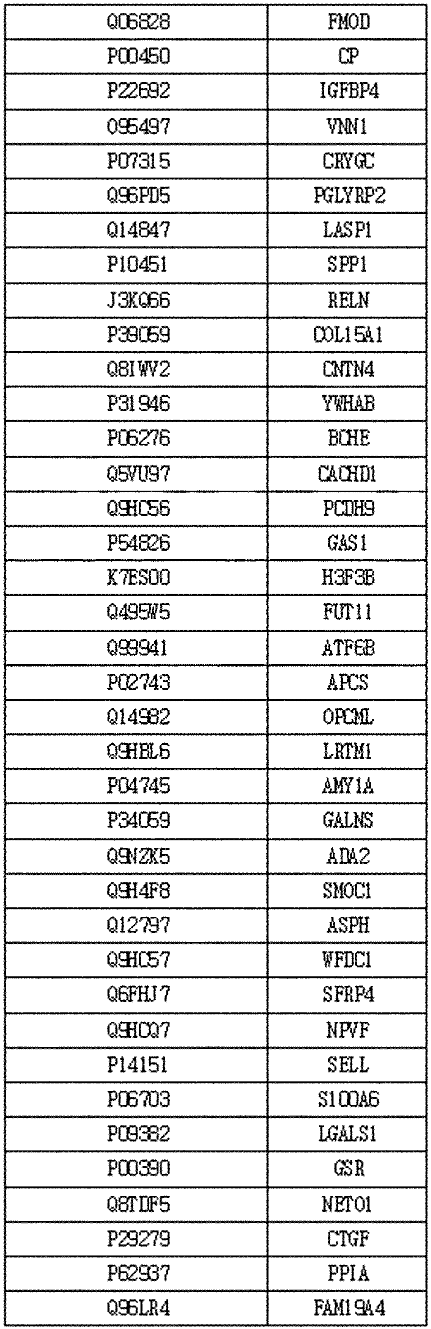 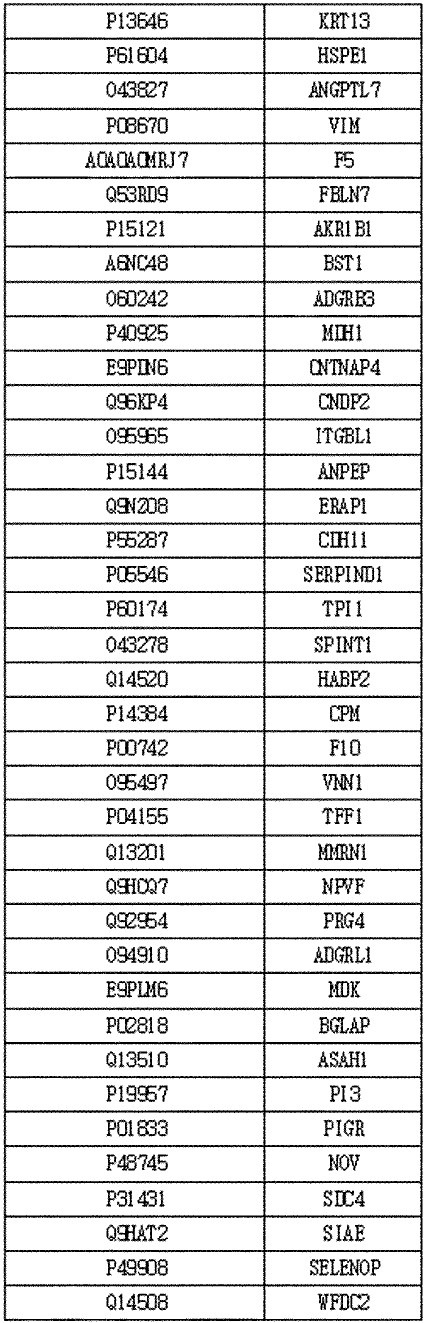 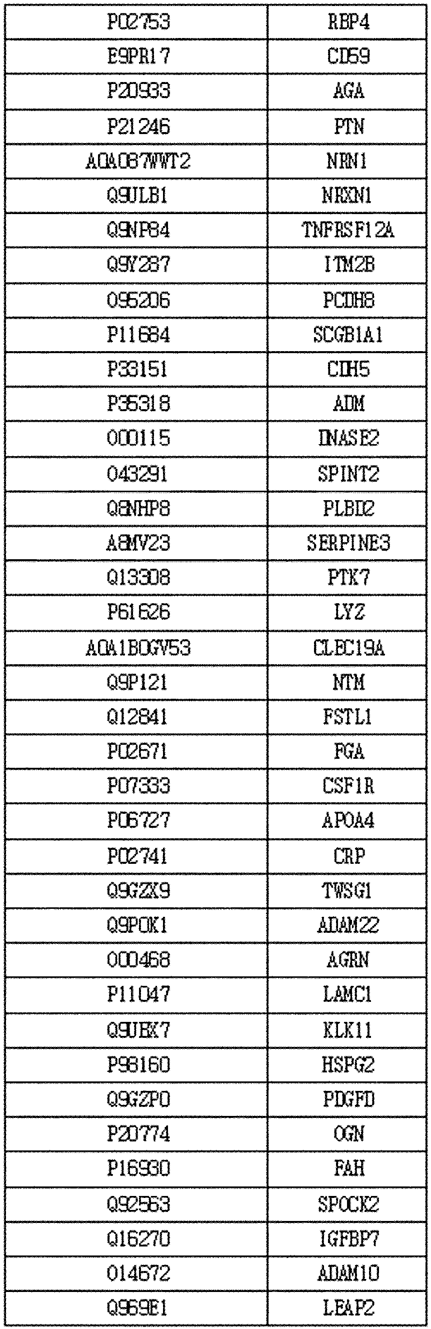 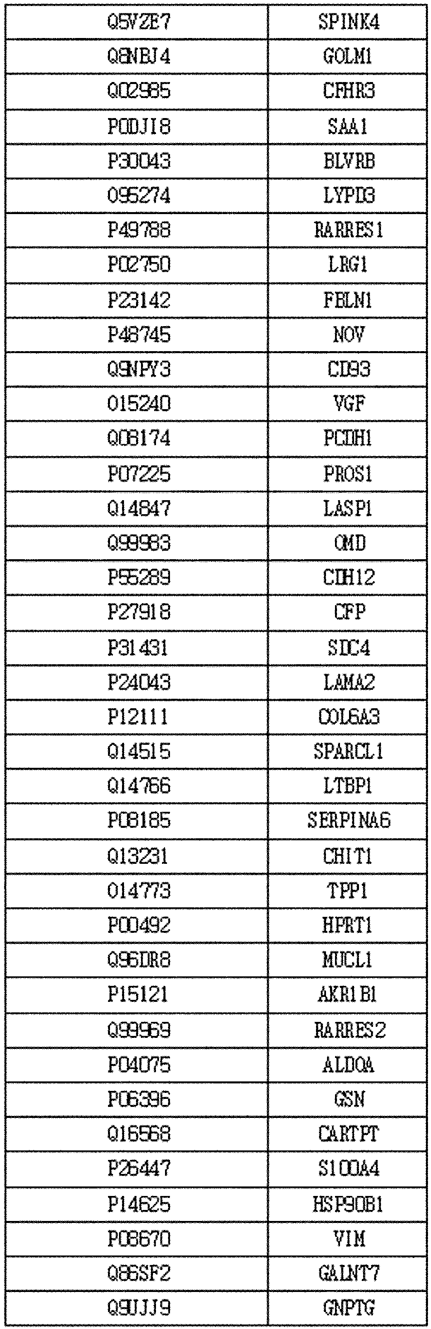 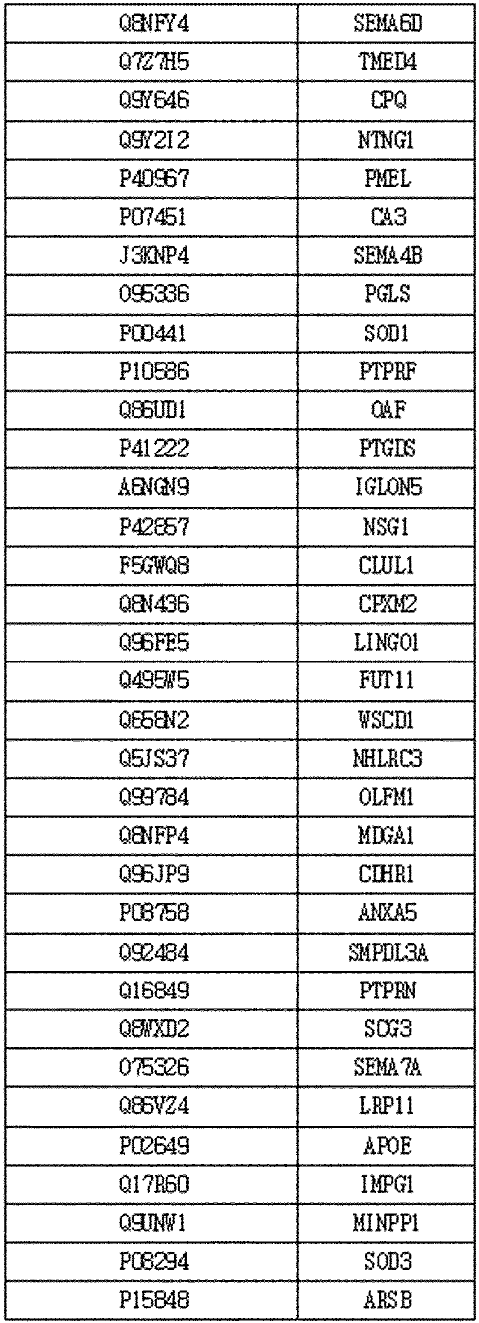
2. The biomarker of item 1, wherein the biomarker is present in aqueous humor of an eye.
3. The biomarker of item 1, wherein:
   a) the brain and nervous system disease is Alzheimer's disease, and the biomarker comprises either at least one gene selected from the group consisting of ACHE, SPP1, EEF2, PRDX1, CES1, YWHAB, CNTN4 and BCHE, or a protein encoded thereby; or
   b) the brain and nervous system disease is Parkinson's disease, and the biomarker comprises either at least one gene selected from the group consisting of CACHD1, PCDH9, GAS1, H3F3B, FUT11, ATF6B, SELL, S100A6, LGALS1, GSR, NETO1 and CTGF, or a protein encoded thereby; or
   c) the brain and nervous system disease is cerebrovascular attack, and the biomarker comprises either at least one gene selected from the group consisting of PPIA, FAM19A4, KRT13, HSPE1, ANGPTL7, VIM, TFF1, MMRN1, NPVF, PRG4, ADGRL1 and MDK, or a protein encoded thereby; or
   d) the brain and nervous system disease is brain tumor, and the biomarker comprises either at least one gene selected from the group consisting of NTM, FSTL1, FGA, CSF1R, APOA4, CRP, HPRT1, MUCL1, AKRIB1, RARRES2, ALDOA and GSN, or a protein encoded thereby.
4. The biomarker of item 3, wherein the biomarker in a) further comprises either at least one gene selected from the group shown in Table 28 below, or a protein encoded thereby: 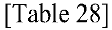 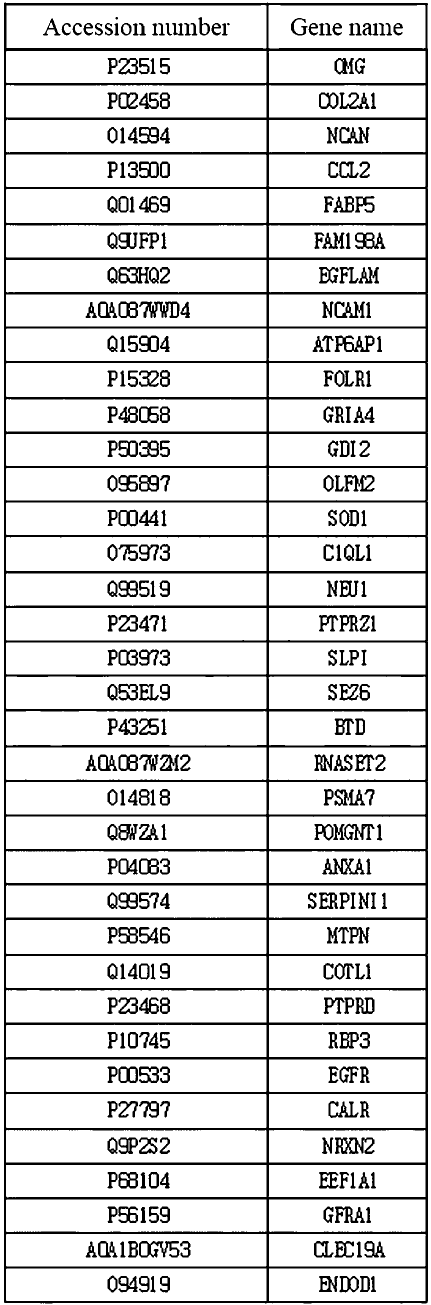 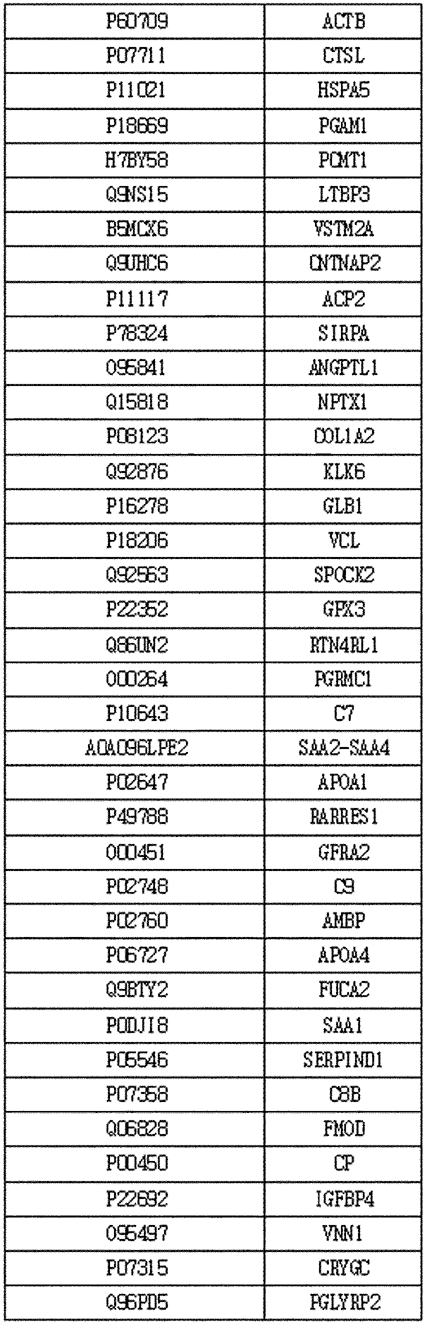 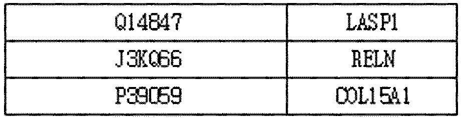
5. The biomarker of item 3, wherein the biomarker in b) further comprises either at least one gene selected from the group consisting of APCS, OPCML, LRTM1, AMY1A, GALNS, ADA2, SMOC1, ASPH, WFDC1, SFRP4 and NPVF, or a protein encoded thereby.
6. The biomarker of item 3, wherein the biomarker in c) further comprises either at least one gene selected from the group shown in Table 29 below, or a protein encoded thereby: 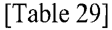 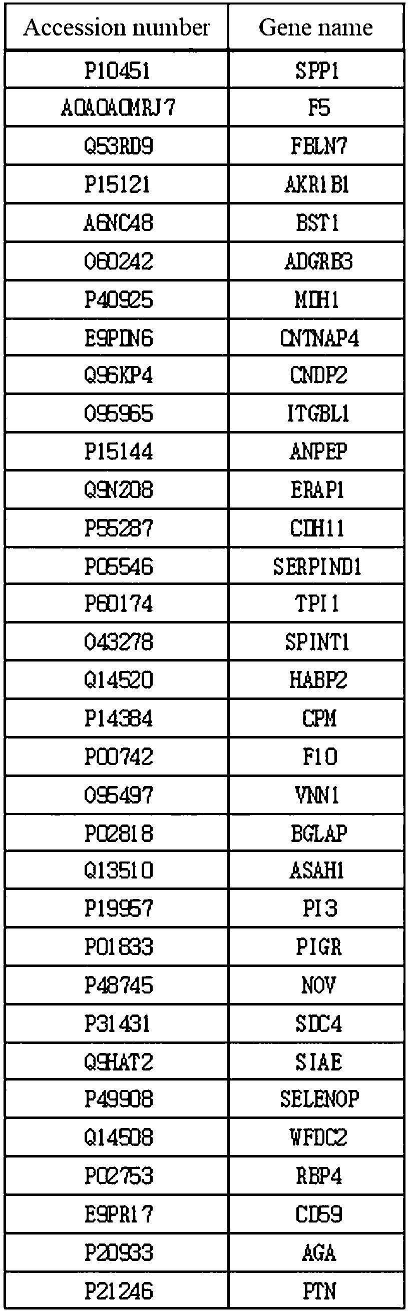 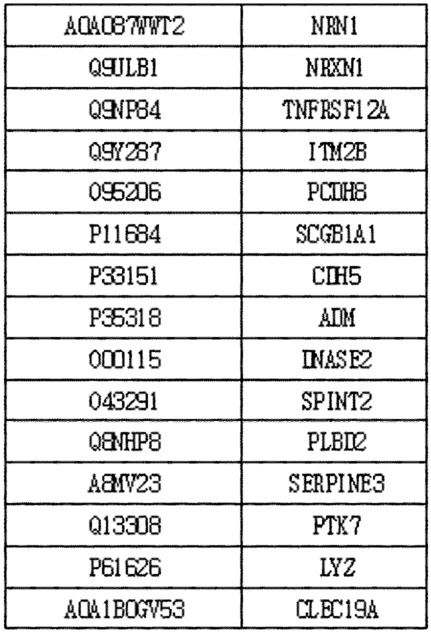
7. The biomarker of item 3, wherein the biomarker in d) further comprises either at least one gene selected from the group shown in Table 30 below, or a protein encoded thereby: 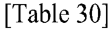 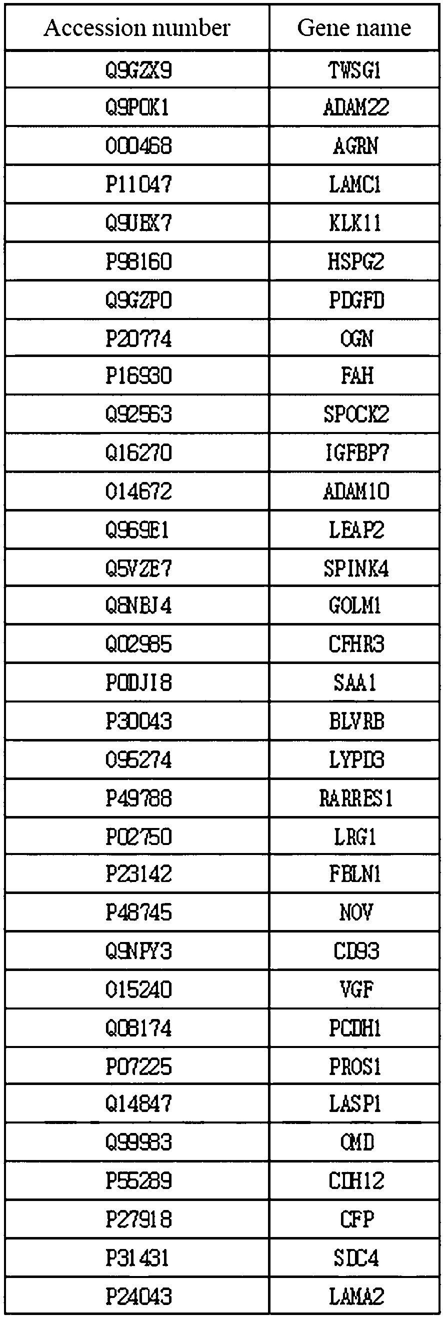 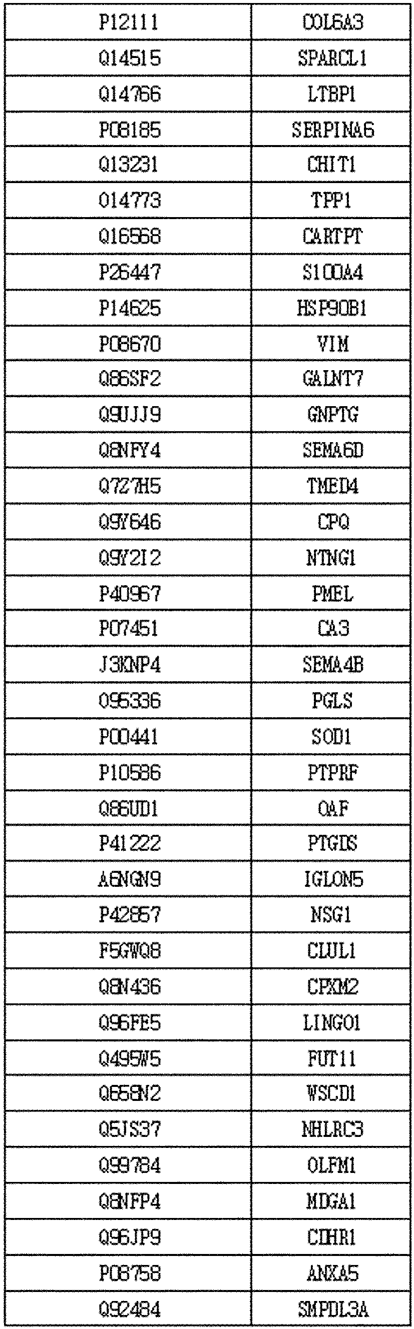 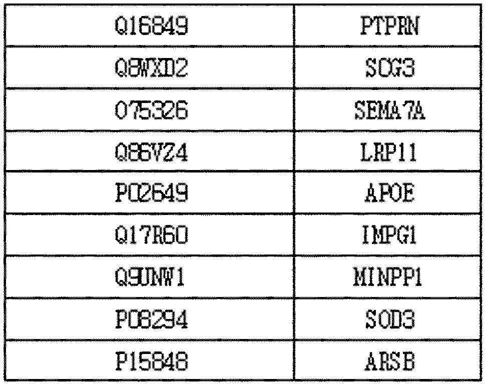
8. A composition for diagnosing a brain and nervous system disease containing an agent for measuring an expression level of either at least one gene selected from the group shown in Table 1 below, or a protein encoded thereby: 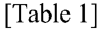 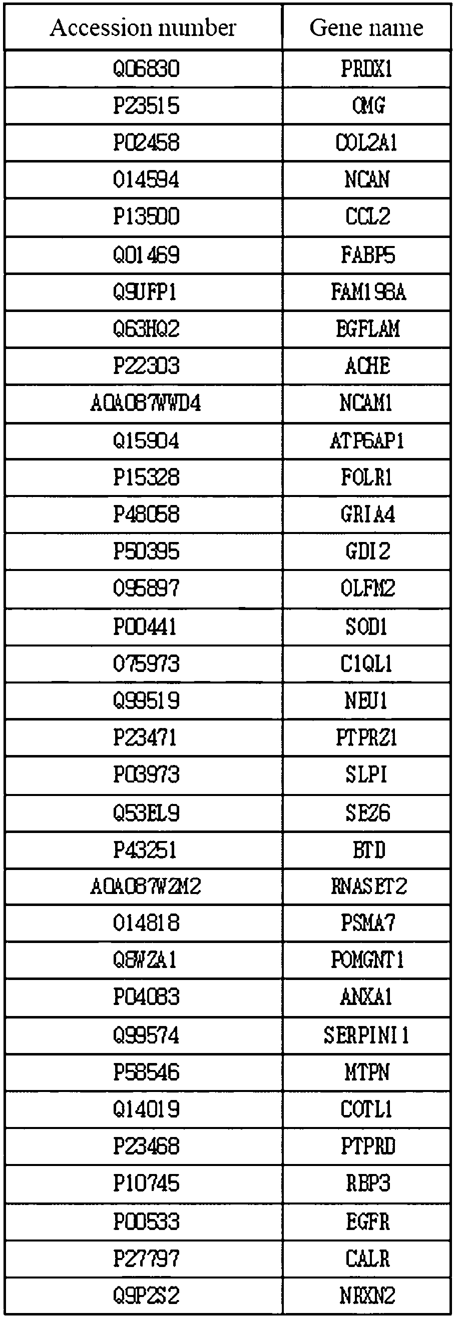 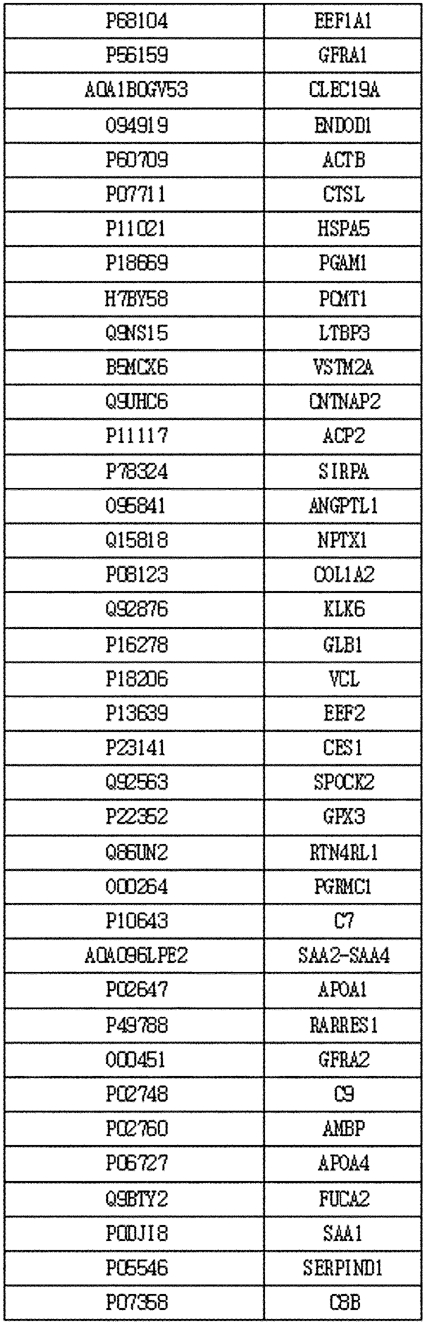 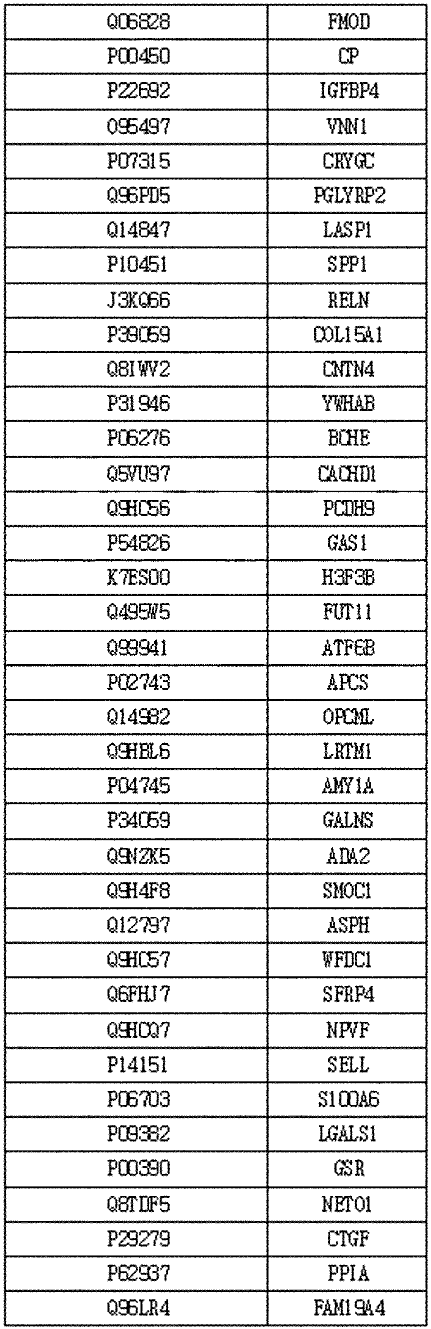 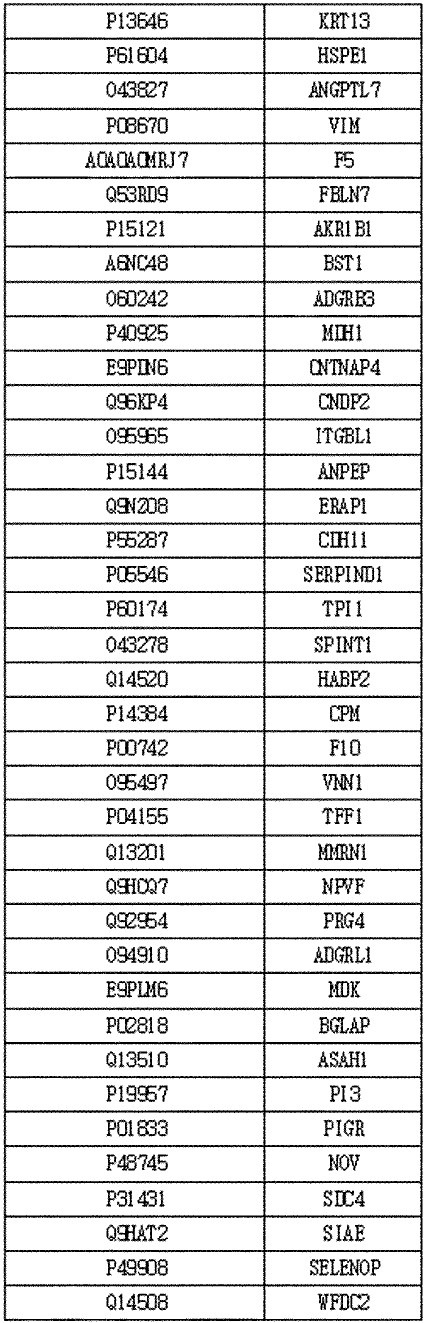 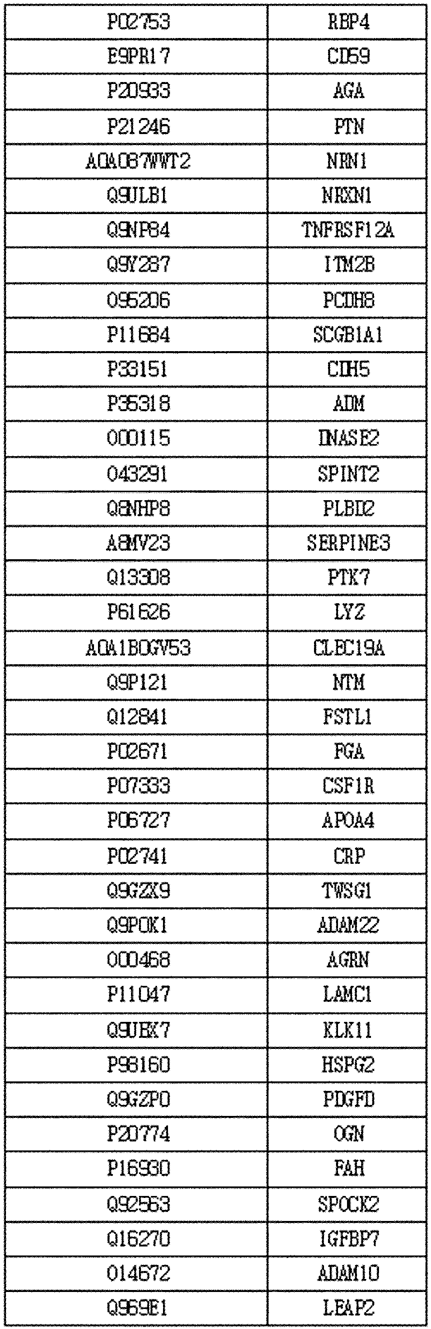 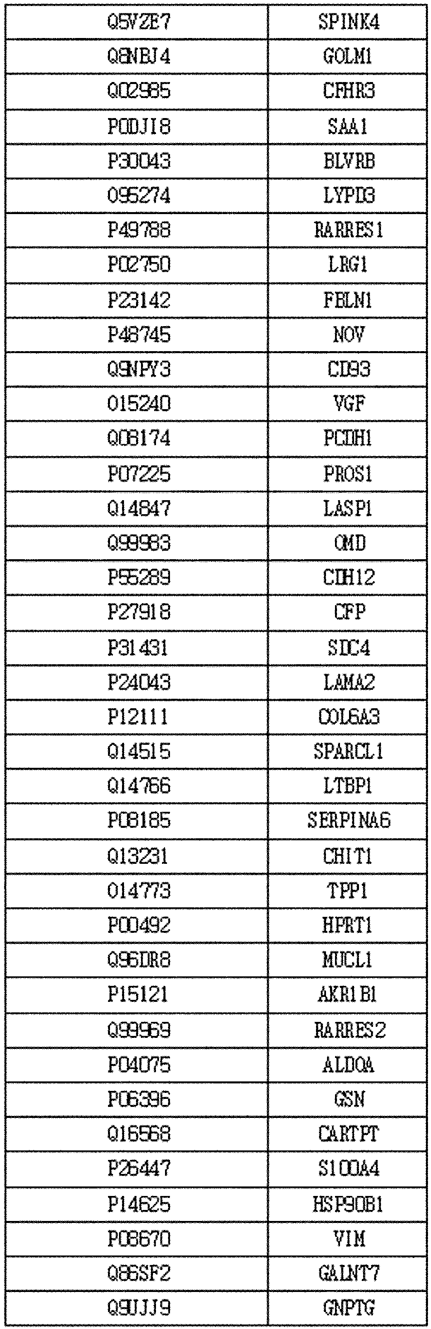 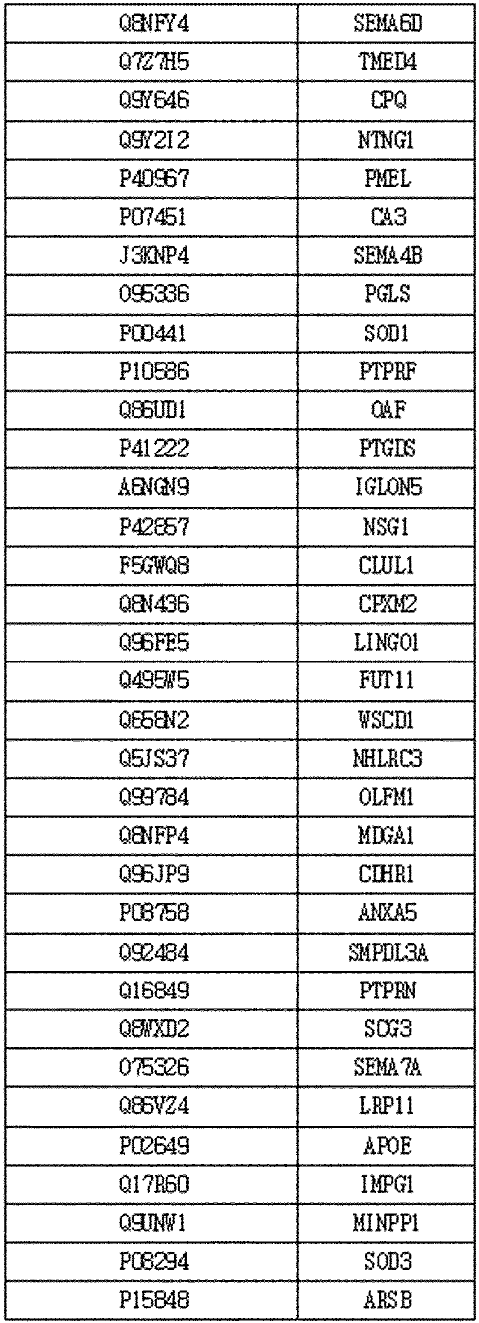
9. The composition of item 8, wherein the gene or the protein is present in aqueous humor of an eye.
10. The composition of item 8, wherein the agent for measuring the expression level of the protein comprises at least one selected from the group consisting of antibodies, oligopeptides, ligands, PNA (peptide nucleic acids) and aptamers, which bind specifically to the protein.
11. The composition of item 8, wherein the agent for measuring the expression level of the gene comprises at least one selected from the group consisting of primers, probes and antisense oligonucleotides, which bind specifically to the gene.
12. The composition of item 8, wherein:
   a) the brain and nervous system disease is Alzheimer's disease, and the agent for measuring the expression level is an agent for measuring the expression level of either at least one gene selected from the group consisting of ACHE, SPP1, EEF2, PRDX1, CES1, YWHAB, CNTN4 and BCHE, or a protein encoded thereby; or
   b) the brain and nervous system disease is Parkinson's disease, and the agent for measuring the expression level is an agent for measuring the expression level of either at least one gene selected from the group consisting of CACHD1, PCDH9, GAS1, H3F3B, FUT11, ATF6B, SELL, S100A6, LGALS1, GSR, NETO1 and CTGF, or a protein encoded thereby; or
   c) the brain and nervous system disease is cerebrovascular attack, and the agent for measuring the expression level is an agent for measuring the expression level of at least one gene selected from the group consisting of PPIA, FAM19A4, KRT13, HSPE1, ANGPTL7, VIM, TFF1, MMRN1, NPVF, PRG4, ADGRL1 and MDK, or a protein encoded thereby; or
   d) the brain and nervous system disease is brain tumor, and the agent for measuring the expression level is an agent for measuring the expression level of either at least one gene selected from the group consisting of NTM, FSTL1, FGA, CSF1R, APOA4, CRP, HPRT1, MUCL1, AKR1B1, RARRES2, ALDOA and GSN, or a protein encoded thereby.
13. The composition of item 12, wherein the composition in a) further comprises an agent for measuring the expression level of either at least one gene selected from the group shown in Table 28 below, or a protein encoded thereby: 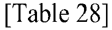 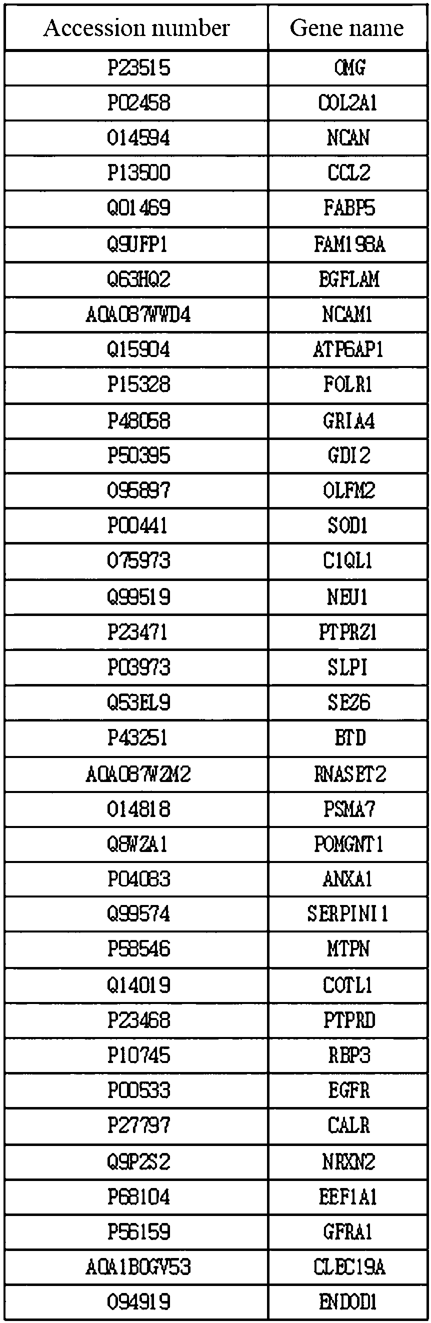 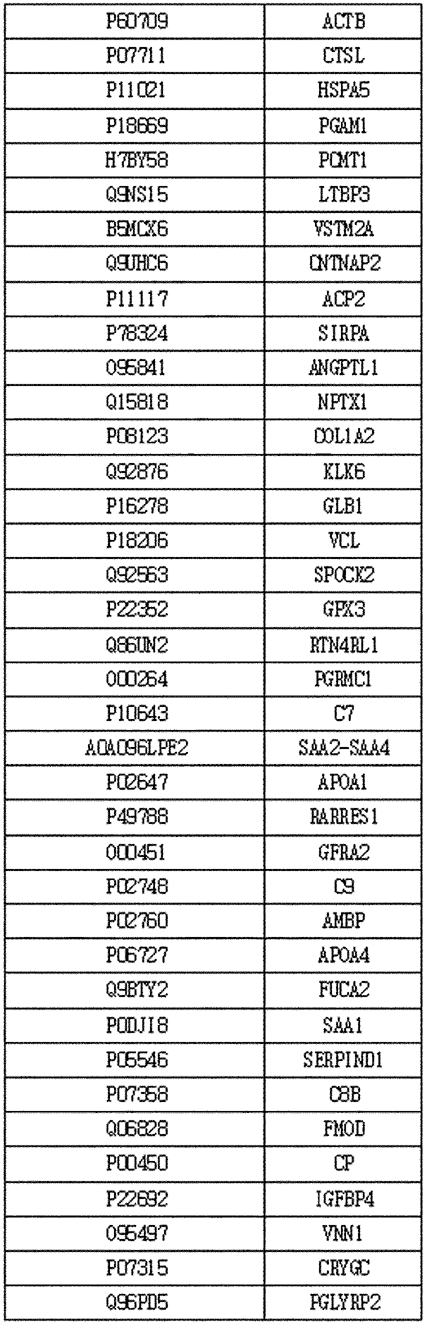 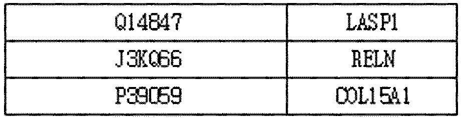
14. The composition of item 12, wherein the composition in b) further comprises an agent for measuring the expression of either at least one gene selected from the group consisting of APCS, OPCML, LRTM1, AMY1A, GALNS, ADA2, SMOC1, ASPH, WFDC1, SFRP4 and NPVF, or a protein encoded thereby.
15. The composition of item 12, wherein the composition in c) further comprises an agent for measuring the expression of either at least one gene selected from the group shown in Table 29 below, or a protein encoded thereby: 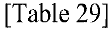 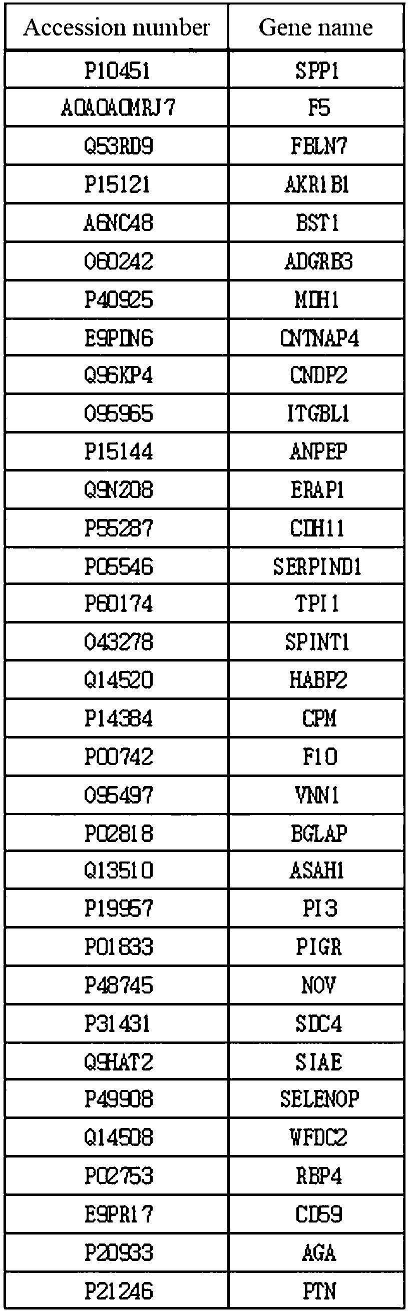 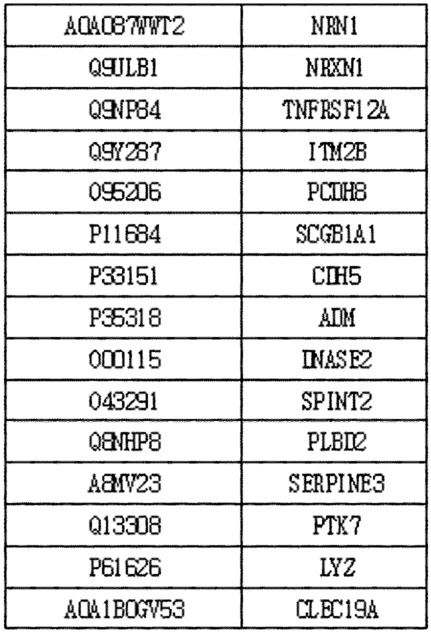
16. The composition of item 12, wherein the composition in d) further comprises an agent for measuring the expression level of either at least one gene selected from the group shown in Table 30 below, or a protein encoded thereby: 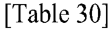 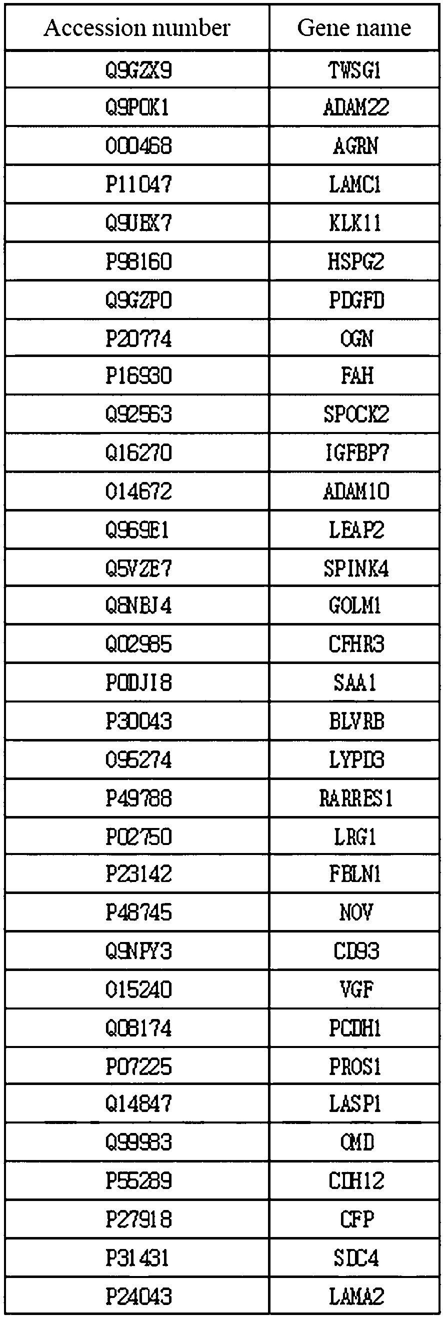 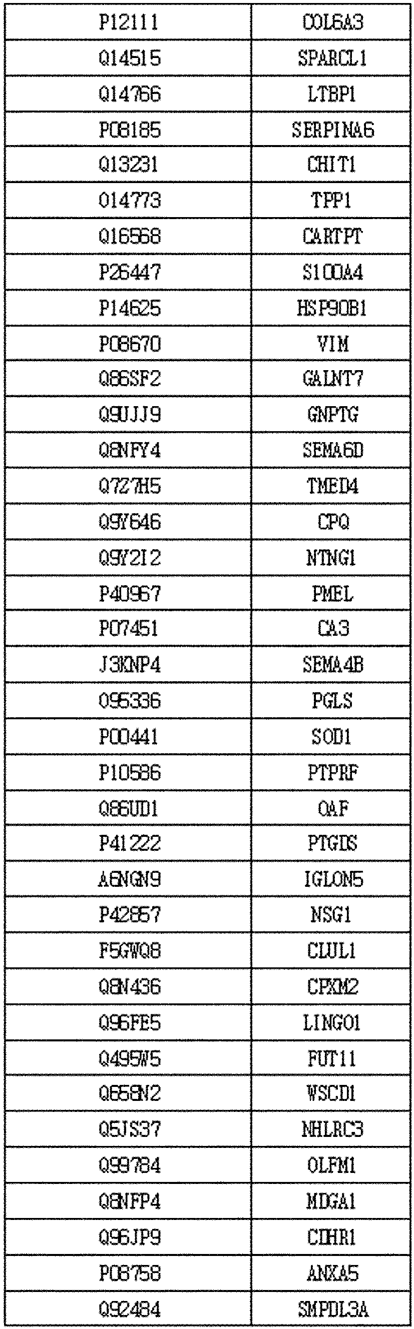 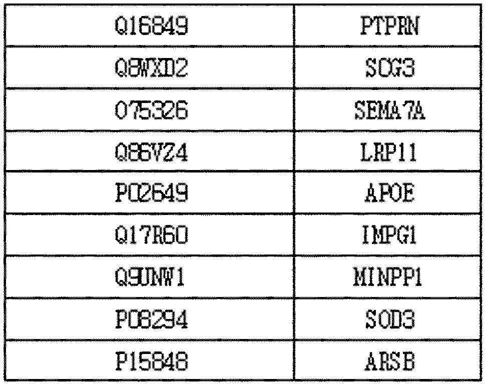
17. A kit for diagnosing a brain and nervous system disease comprising the composition of any one of items 8 to 16.
18. The kit of item 17, which is an RT-PCR kit, a DNA chip kit, an ELISA kit, a protein chip kit, a rapid kit, or a multiple-reaction monitoring (MRM) kit.
19. A method for providing information for diagnosing a brain and nervous system disease, the method comprising a step of measuring an expression level of either at least one gene selected from the group shown in Table 1 below, or a protein encoded thereby, in a biological sample isolated from a subject of interest. 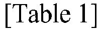 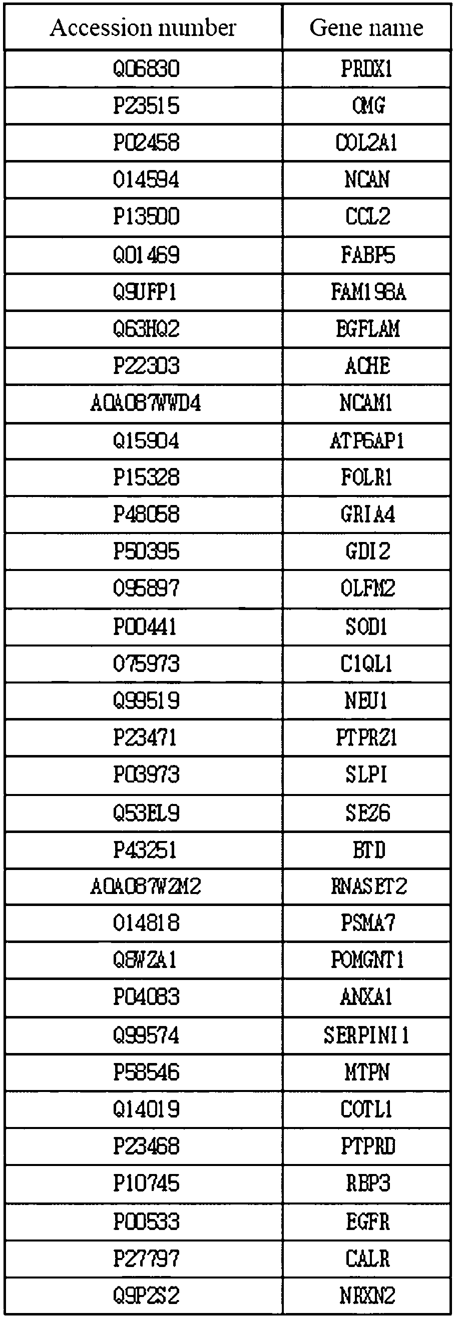 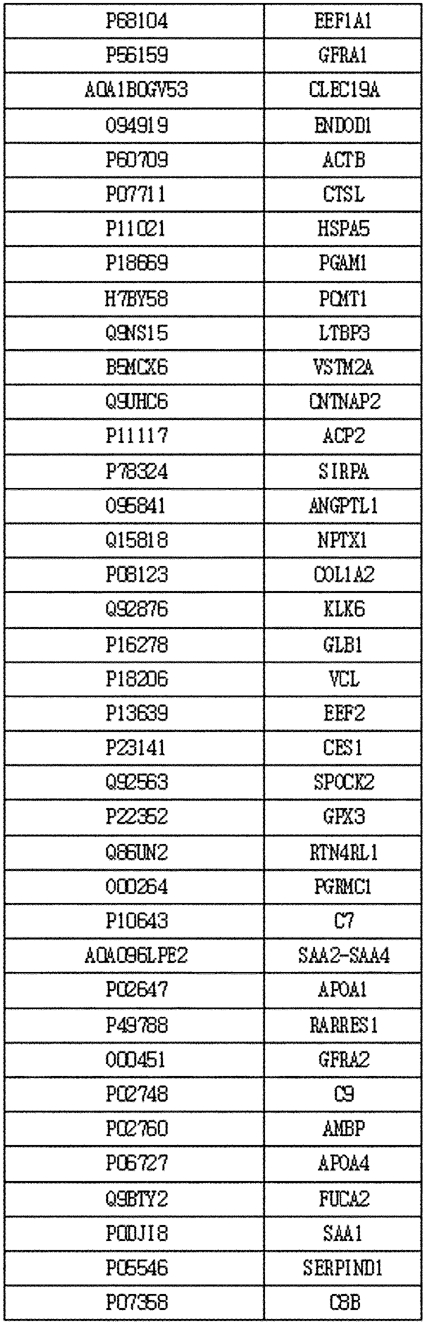 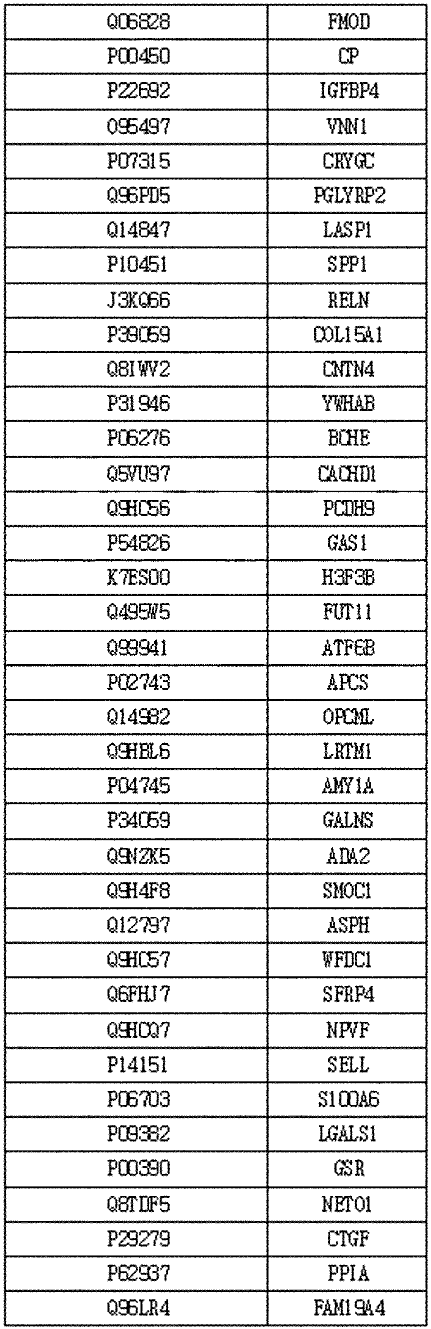 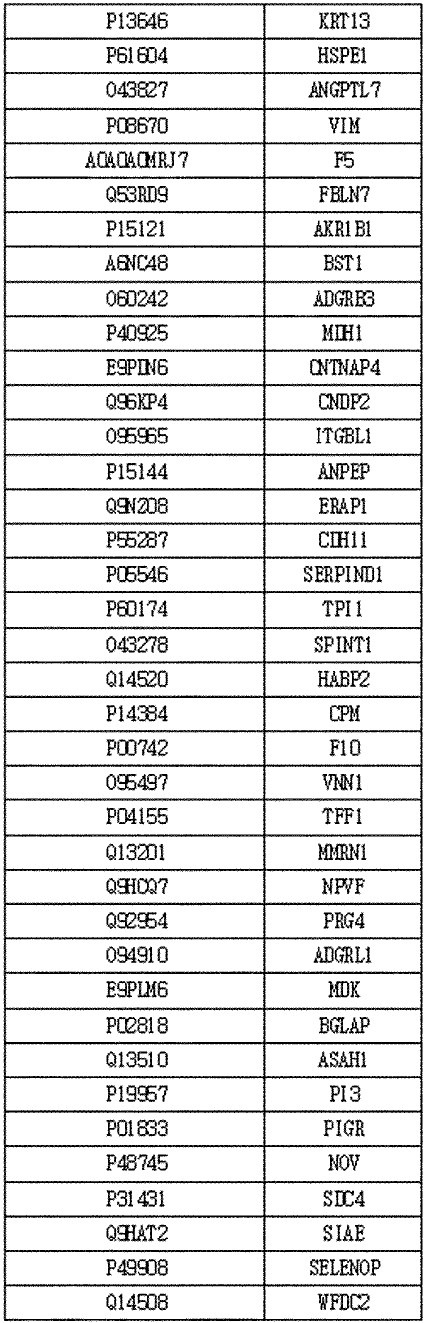 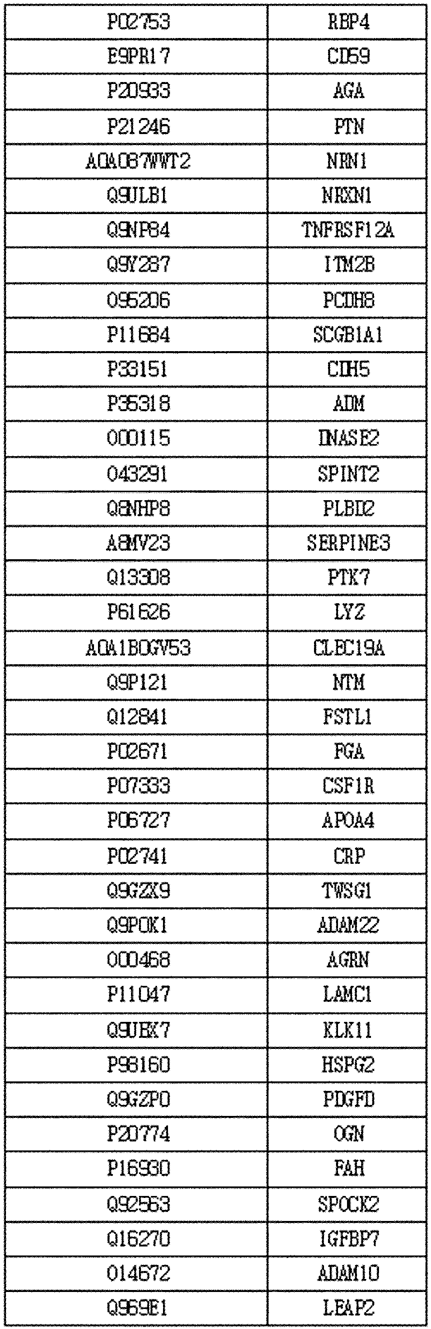 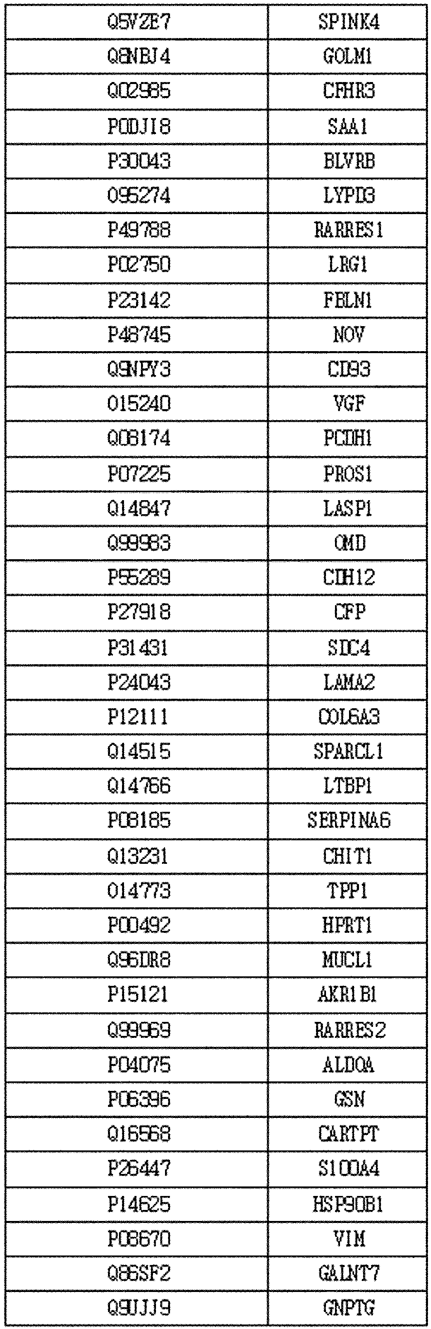 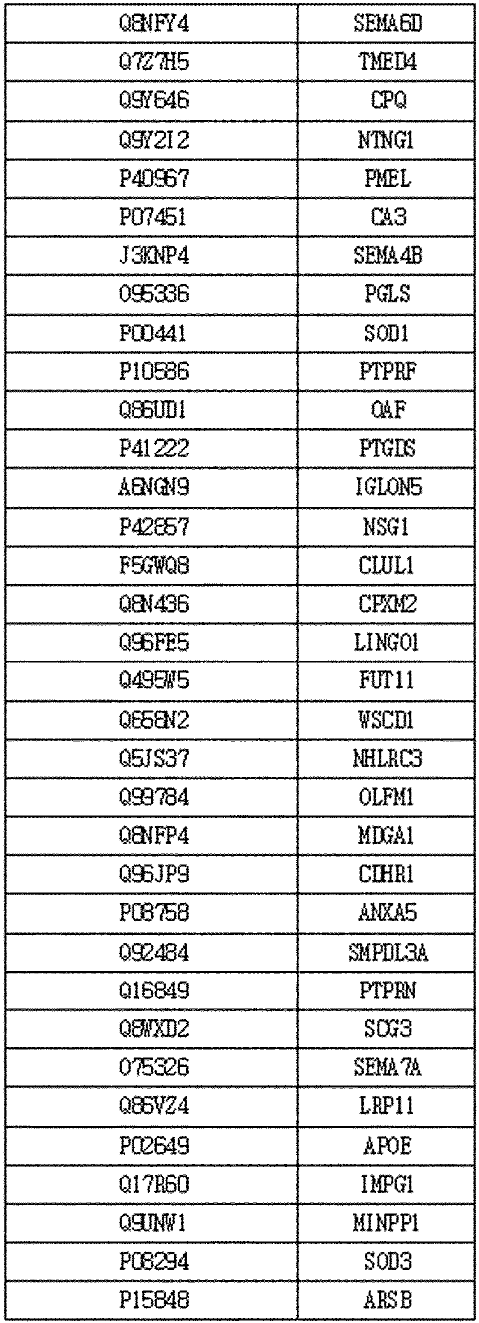
20. The method of item 19, wherein the biological sample is aqueous humor of an eye.
21. The method of item 19, wherein an agent for measuring the expression level of the protein comprises at least one selected from the group consisting of antibodies, oligopeptides, ligands, peptide nucleic acids (PNAs) and aptamers, which bind specifically to the protein.
22. The method of item 19, wherein a method of measuring the expression level of the protein is protein chip analysis, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) assay, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, or enzyme-linked immunosorbent assay (ELISA).
23. The method of item 19, wherein an agent for measuring the expression level of the gene comprises at least one selected from the group consisting of primers, probes and antisense oligonucleotides, which bind specifically to the gene.
24. The method of item 19, wherein a method of measuring the expression level of the gene is reverse transcription polymerase chain reaction (RT-PCR), competitive RT-PCR, real-time RT-PCR, RNase protection assay (RPA), Northern blotting, or DNA chip assay.
25. The method of item 19, wherein:
   a) the brain and nervous system disease is Alzheimer's disease, and the step of measuring the expression level comprises measuring the expression level of either at least one gene selected from the group consisting of ACHE, SPP1, EEF2, PRDX1, CES1, YWHAB, CNTN4 and BCHE, or a protein encoded thereby; or
   b) the brain and nervous system disease is Parkinson's disease, and the step of measuring the expression level comprises measuring the expression level of either at least one gene selected from the group consisting of CACHD1, PCDH9, GAS1, H3F3B, FUT11, ATF6B, SELL, S100A6, LGALS1, GSR, NETO1 and CTGF, or a protein encoded thereby; or
   c) the brain and nervous system disease is cerebrovascular attack, and the step of measuring the expression level comprises measuring the expression level of either at least one gene selected from the group consisting of PPIA, FAM19A4, KRT13, HSPE1, ANGPTL7, VIM, TFF1, MMRN1, NPVF, PRG4, ADGRL1 and MDK, or a protein encoded thereby; or
   d) the brain and nervous system disease is brain tumor, and the step of measuring the expression level comprises measuring the expression level of either at least one gene selected from the group consisting of NTM, FSTL1, FGA, CSF1R, APOA4, CRP, HPRT1, MUCL1, AKR1B1, RARRES2, ALDOA and GSN, or a protein encoded thereby.
26. The method of item 25, wherein, in a), the step of measuring the expression further comprises a step of measuring the expression level of either at least one gene selected from the group shown in Table 28 below, or a protein encoded thereby: 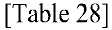 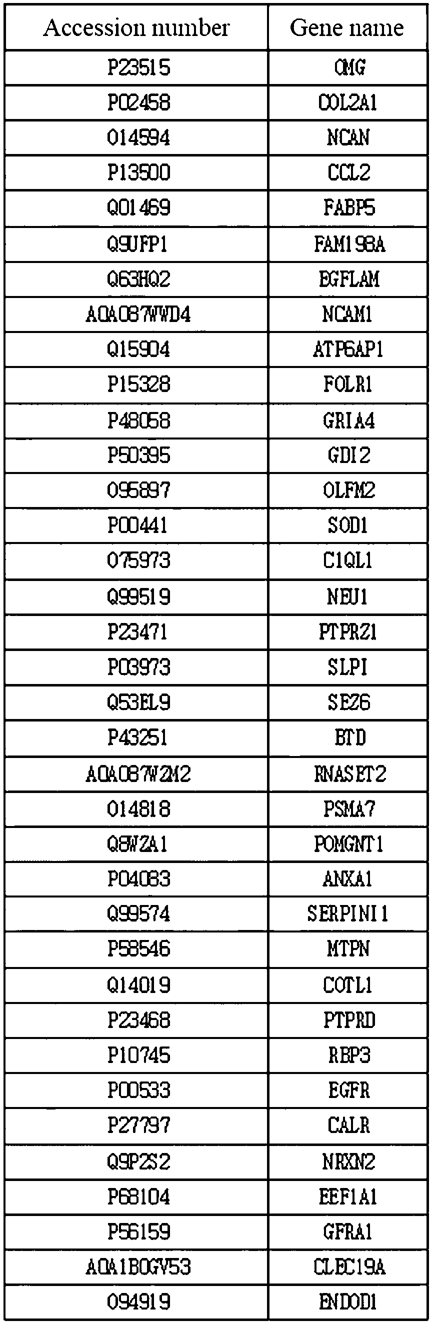 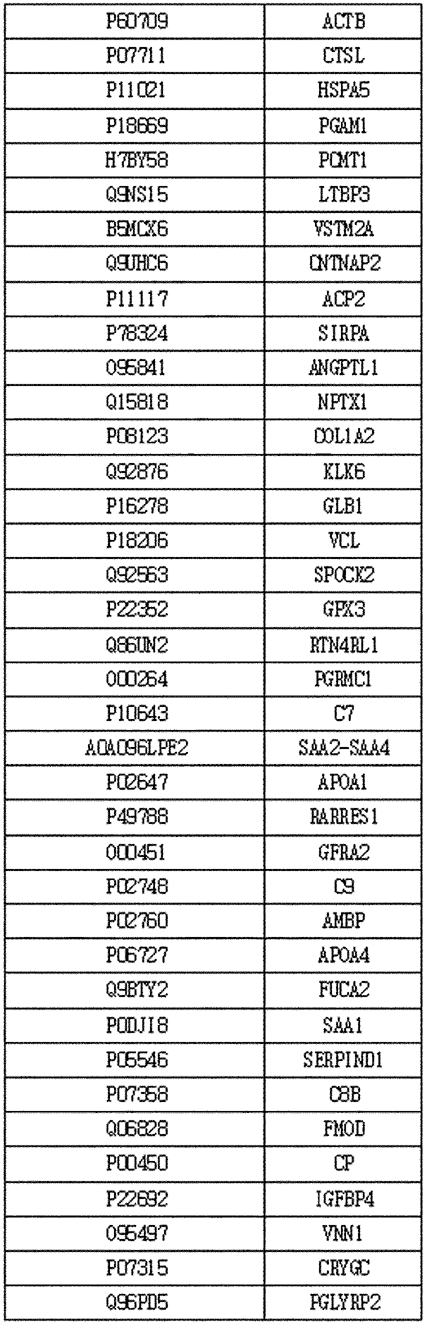 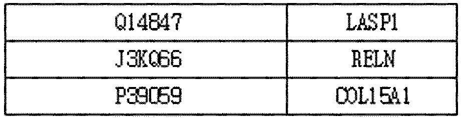
27. The method of item 25, wherein, in a), when the measured expression level of either at least one gene selected from the group consisting of ACHE, SPP1, EEF2, PRDX1 and CES1, or a protein encoded thereby is higher than that in a normal control group, or when the measured expression level of either at least one gene selected from the group consisting of YWHAB, CNTN4 and BCHE, or a protein encoded thereby is lower than that in the normal control group, it is predicted that the likelihood of developing Alzheimer's disease is high.
28. The method of item 25, wherein, in b), the step of measuring the expression level further comprises a step of measuring the expression level of either at least one gene selected from the group consisting of APCS, OPCML, LRTM1, AMY1A, GALNS, ADA2, SMOC1, ASPH, WFDC1, SFRP4 and NPVF, or a protein encoded thereby.
29. The method of item 25, wherein, in b), when the measured expression level of either at least one gene selected from the group consisting of CACHD1, PCDH9, GAS1, H3F3B, FUT11 and ATF6B, or a protein encoded thereby is higher than that in a normal control group, or when the measured expression level of at least one gene selected from the group consisting of SELL, S100A6, LGALS1, GSR, NETO1 and CTGF, or a protein encoded thereby is lower than that in the normal control group, it is predicted that the likelihood of developing Parkinson's disease is high.
30. The method of item 25, wherein, in c), the step of measuring the expression level further comprises a step of measuring the expression level of either at least one gene selected from the group shown in Table 29 below, or a protein encoded thereby: 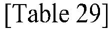 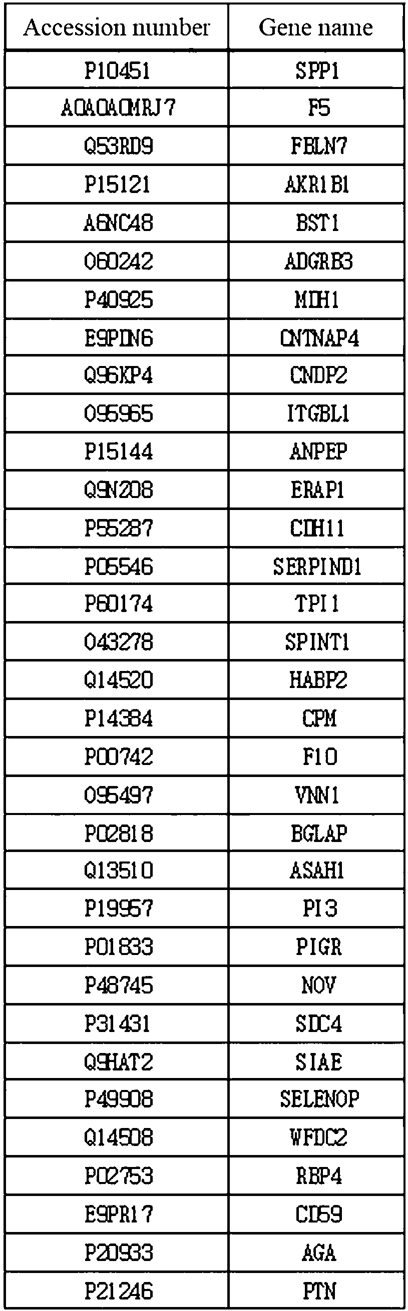 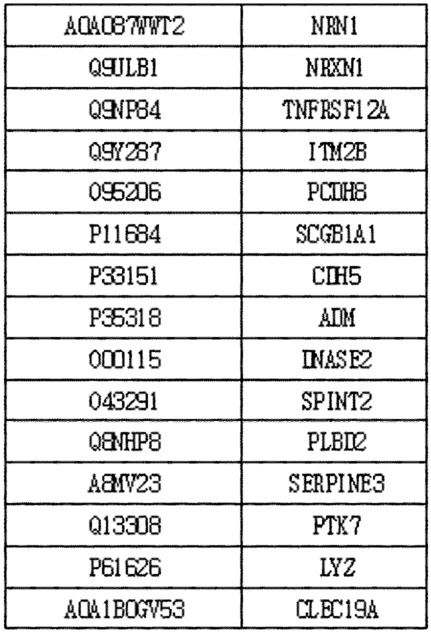
31. The method of item 25, wherein, in c), when the measured expression level of either at least one gene selected from the group consisting of PPIA, FAM19A4, KRT13, HSPE1, ANGPTL7 and VIM, or a protein encoded thereby is higher than that in a normal control group, or when the measured expression level of either at least one gene selected from the group consisting of TFF1, MMRN1, NPVF, PRG4, ADGRL1 and MDK, or a protein encoded thereby is lower than that in the normal control group, it is predicted that the likelihood of developing cerebrovascular attack is high.
32. The method of item 25, wherein, in d), the step of measuring the expression level further comprises a step of measuring the expression level of either at least one gene selected from the group shown in Table 30 below, or a protein encoded thereby: 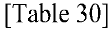 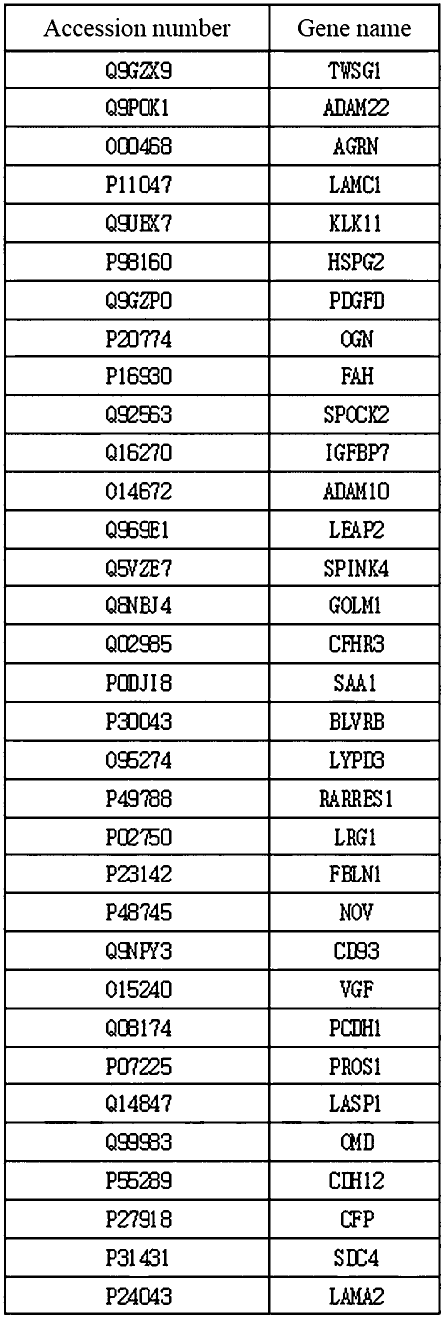 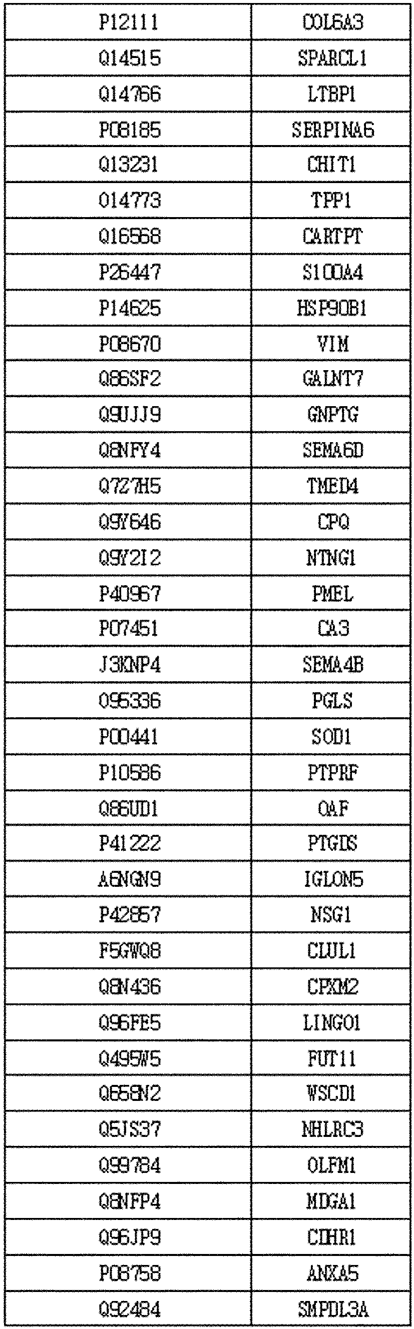 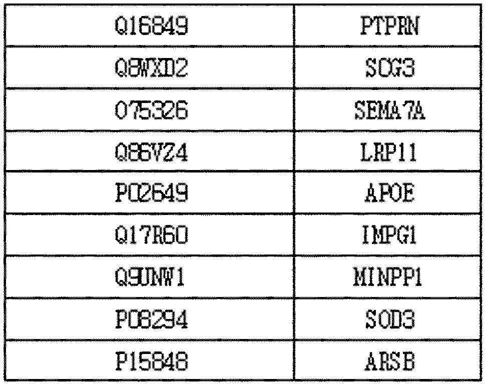
33. The method of item 25, wherein, in d), when the measured expression level of either at least one gene selected from the group consisting of NTM, FSTL1, FGA, CSF1R, APOA4 and CRP, or a protein encoded thereby is higher than that in a normal control group, or when the measured expression level of either at least one gene selected from the group consisting of HPRT1, MUCL1, AKR1B1, RARRES2, ALDOA and GSN, or a protein encoded thereby is lower than that in the normal control group, it is predicted that the likelihood of developing brain tumor is high.
34. A method for screening a drug that induces a brain and nervous system disease, the method comprising steps of:
   treating an isolated biological sample with a candidate substance expected to induce the brain and nervous system disease; and
   measuring an expression level of either at least one gene selected from the group shown in Table 1 below, or a protein encoded thereby, in the biological sample treated with the candidate sample: 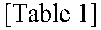 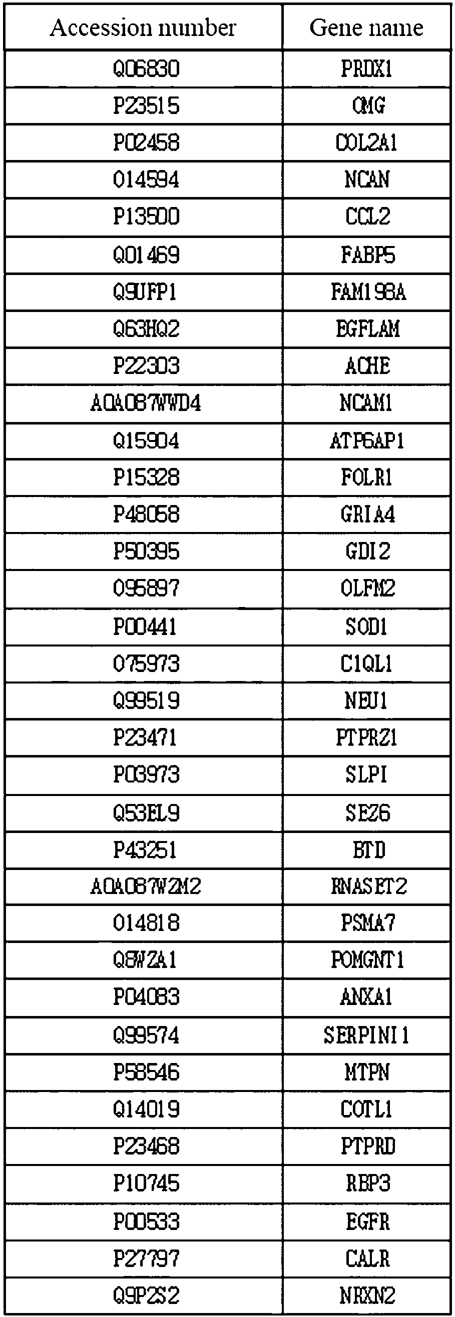 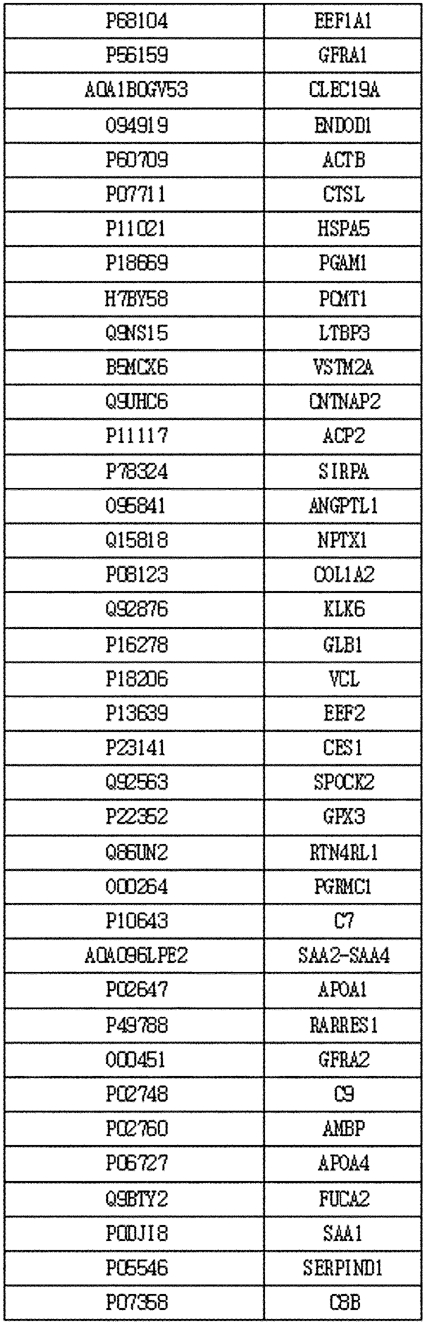 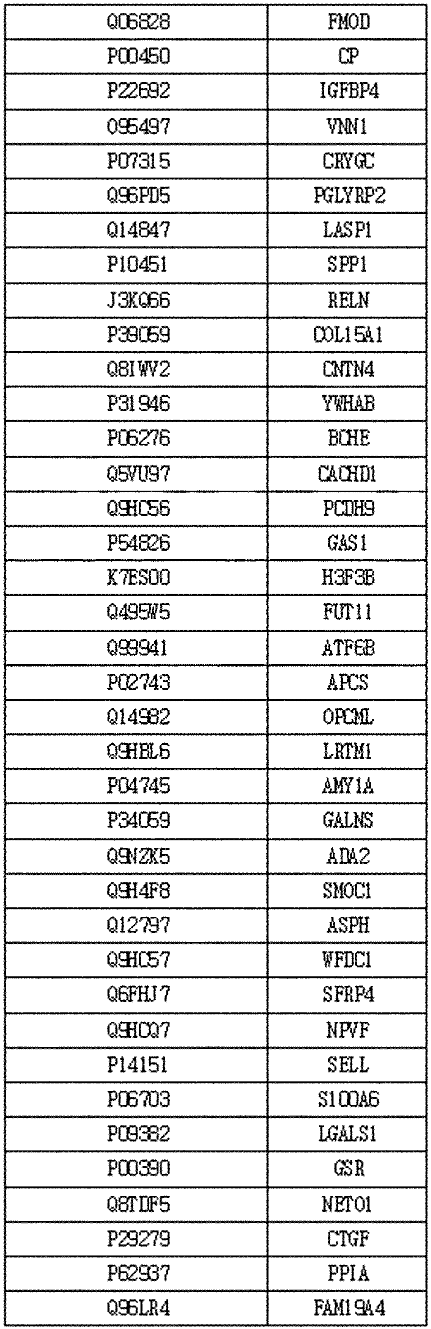 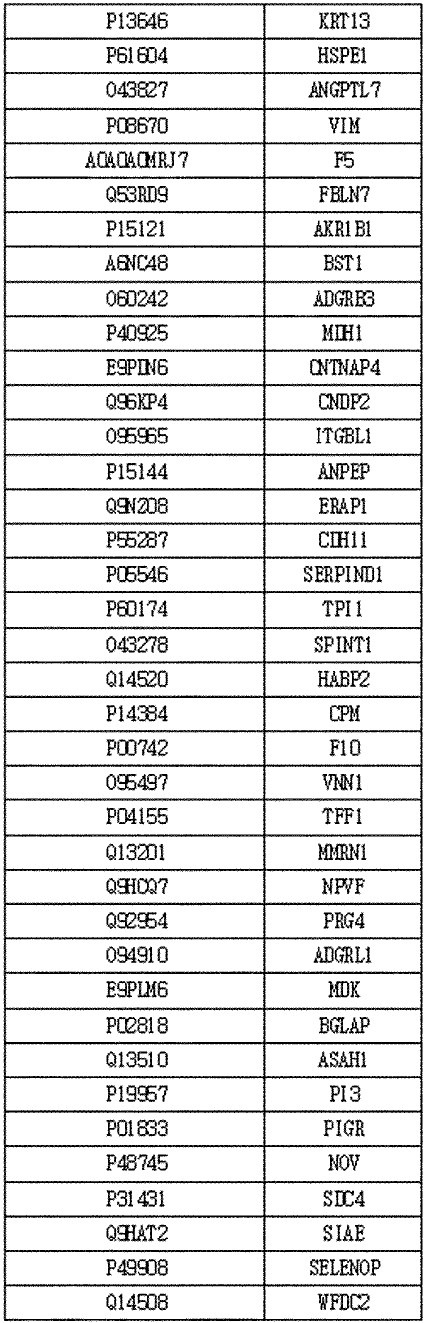 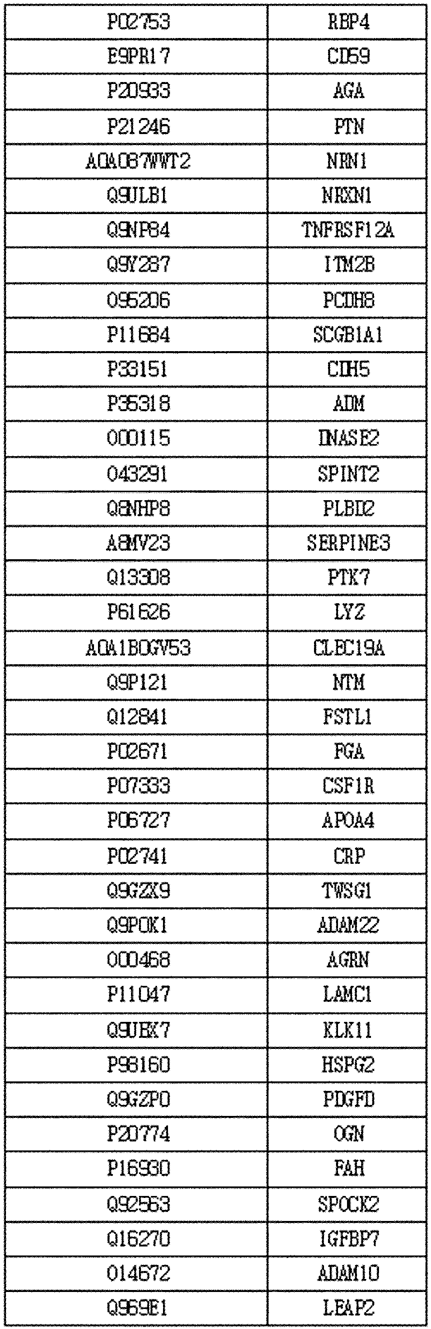 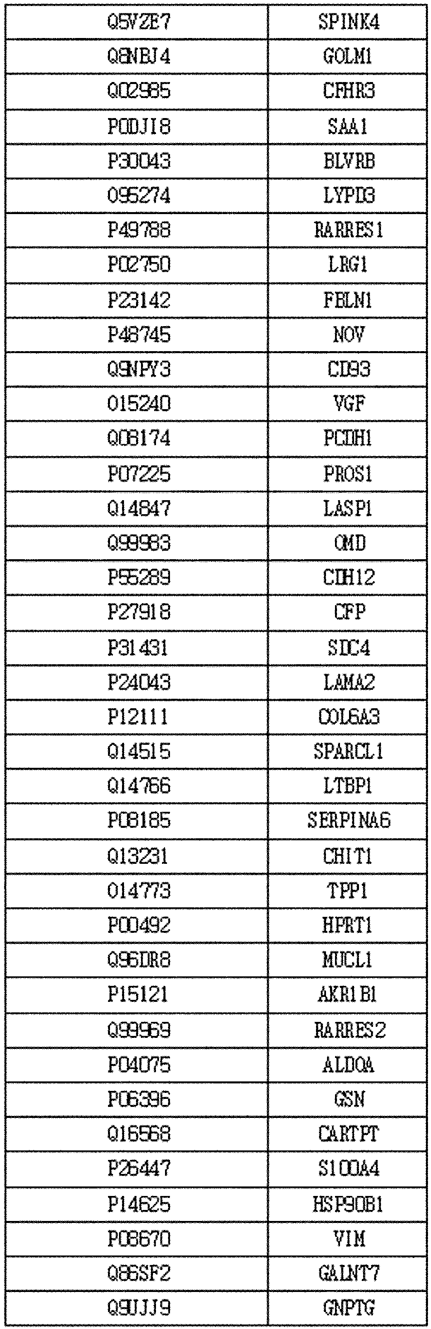 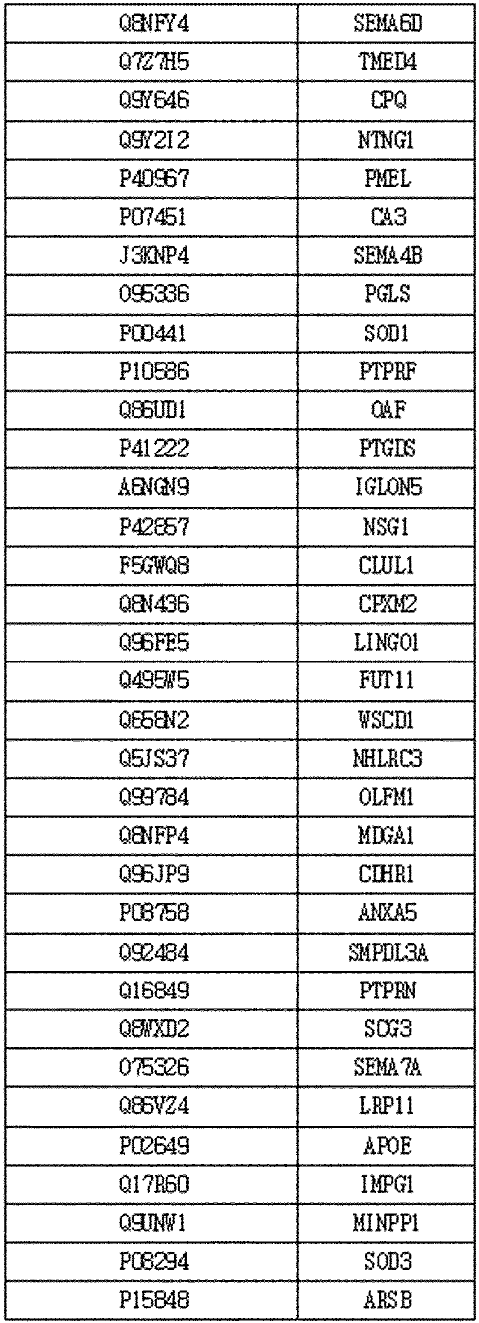
35. The method of item 34, wherein the biological sample is aqueous humor of an eye.
36. The method of item 34, wherein:
   a) the brain and nervous system disease is Alzheimer's disease, and the step of measuring the expression level comprises measuring the expression level of either at least one gene selected from the group consisting of ACHE, SPP1, EEF2, PRDX1, CES1, YWHAB, CNTN4 and BCHE, or a protein encoded thereby; or
   b) the brain and nervous system disease is Parkinson's disease, and the step of measuring the expression level comprises measuring the expression level of either at least one gene selected from the group consisting of CACHD1, PCDH9, GAS1, H3F3B, FUT11, ATF6B, SELL, S100A6, LGALS1, GSR, NETO1 and CTGF, or a protein encoded thereby; or
   c) the brain and nervous system disease is cerebrovascular attack, and the step of measuring the expression level comprises measuring the expression level of either at least one gene selected from the group consisting of PPIA, FAM19A4, KRT13, HSPE1, ANGPTL7, VIM, TFF1, MMRN1, NPVF, PRG4, ADGRL1 and MDK, or a protein encoded thereby; or
   d) the brain and nervous system disease is brain tumor, and the step of measuring the expression level comprises measuring the expression level of either at least one gene selected from the group consisting ofNTM, FSTL1, FGA, CSF1R, APOA4, CRP, HPRT1, MUCL1, AKR1B1, RARRES2, ALDOA and GSN, or a protein encoded thereby.

## Claims

1. A method for diagnosing Parkinson's disease comprising measuring the expression level of at least one protein encoded by a gene selected from the group consisting of CACHD1, PCDH9, GAS1, H3F3B, FUT11, ATF6B, SELL, S100A6, LGALS1, GSR, NETO1 and CTGF, in a biological sample isolated from a subject of interest,
wherein the expression level of the protein encoded by CACHD1, PCDH9, GAS1, H3F3B, FUT11 or ATF6B is higher than in a normal control group indicates a higher likelihood of developing Parkinson's disease or wherein the expression level of the protein encoded by SELL, S100A6, LGALS1, GSR, NETO1 or CTGF is lower than in a normal control group indicates a higher likelihood of developing Parkinson's disease,
wherein the isolated biological sample is aqueous humor of an eye.

2. The method of claim 1, wherein measuring the expression level of the at least one protein comprises using an agent that binds specifically to the protein, said agent selected from the group consisting of antibodies, oligopeptides, ligands, peptide nucleic acids (PNAs) and aptamers.

3. The method of claim 1, wherein a method of measuring the expression level of the at least one protein is protein chip analysis, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) assay, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, or enzyme-linked immunosorbent assay (ELISA).

4. The method of claim 1, further comprising measuring the expression level of at least one protein encoded by a gene selected from the group consisting of APCS, OPCML, LRTM1, AMY1A, GALNS, ADA2, SMOC1, ASPH, WFDC1, SFRP4 and NPVF,
wherein the expression level of the at least one protein is different from that in a normal control group indicates a higher likelihood of developing Parkinson's disease.
